# EUROPEAN PATENT SPECIFICATION

(11) **EP 0 601 135 B1**
(45) Date of publication and mention of the grant of the patent: **02.11.2005**
(21) Application number: 93900497.4
(22) Date of filing: 28.08.1992
(51) Int. Cl.: A61K 38/17, A61K 38/18, C07K 14/51

(54) **OSTEOGENIC PROTEINS IN THE TREATMENT OF METABOLIC BONE DISEASES**
OSTEOGENISCHE PROTEINE IN DER BEHANDLUNG VON METABOLISCHEN KNOCHENKRANKHEITEN
PROTEINES OSTEOGENIQUES POUR LE TRAITEMENT DES MALADIES OSSEUSES METABOLIQUES

(30) Priority: 30.08.1991 US 752857; 30.08.1991 US 752764; 30.08.1991 US 752861; 31.07.1992 US 923780
(43) Date of publication of application: 15.06.1994
(73) Proprietor: Curis, Inc., Cambridge, MA 02138 (US)
(72) Inventor: KUBERASAMPATH, Thangavel, Medway, MA 02053 (US); COHEN, Charles, M., Medway, MA 02053 (US); OPPERMANN, Hermann, Medway, MA 02053 (US); OZKAYNAK, Engin, Milford, MA 01757 (US); RUEGER, David, C., Hopkinton, MA 01748 (US); PANG, Roy, H., L., Etna, NH 03750 (US); SMART, John, E., Weston, MA 02193 (US)
(74) Representative: Price, Vincent Andrew
(86) International application number: PCT/US1992/007432
(87) International publication number: WO 1993/005751

(56) References cited:
- EP-A- 0 416 578
- EP-A- 0 512 844
- EP-A- 0 514 720
- WO-A-89/09788
- WO-A-89/10409
- WO-A-91/05802
- WO-A-92/00382
- WO-A-92/15323
- WO-A-92/21365
- PROCEEDINGS OF THE NATIONAL ACADEMY OF SCIENCES OF USA. vol. 87, March 1990, WASHINGTON US pages 2220 - 2224 WANG, E.A. ET AL 'Recombinant human bone morphogenetic protein induces bone formation'
- PROCEEDINGS OF THE NATIONAL ACADEMY OF SCIENCES OF USA. vol. 86, November 1989, WASHINGTON US pages 8793 - 8797 VUKICEVIC, S. ET AL 'Stimulation of the expression of osteogenic and chondrogenic phenotipes in vitro by osteogenin'
- JOURNAL OF BIOLOGICAL CHEMISTRY. (MICROFILMS) vol. 267, no. 28, 5 October 1992, BALTIMORE, MD US pages 20352 - 20362 SAMPATH T.K. ET AL 'Recombinant human osteogenic protein-1 (hOP-1) induces new bone formation in vivo with a specific activity comparable with natural bovine osteogenic protein and stimulates osteoblast proliferation and differenciation in vitro'
- EMBO JOURNAL vol. 9, no. 7, 1990, EYNSHAM, OXFORD GB pages 2085 - 2093 \ZKAYNAK, E. ET AL 'OP-1 cDNA encodes an osteogenic protein in the TGF-beta family'

### SEQUENCE LISTING

(1) GENERAL INFORMATION:
   (i) APPLICANTS: Thangavel Kuberasampath
      Charles Cohen
      Hermann Oppermann
      Engin Ozkayanak
      David C. Rueger
      Roy H.L. Pang
   (ii) TITLE OF INVENTION: TREATMENT TO PREVENT LOSS OF AND/OR INCREASE BONE MASS IN METABOLIC BONE DISEASE
   (iii) NUMBER OF SEQUENCES: 33
   (iv) CORRESPONDENCE ADDRESS:
      (A) ADDRESSEE: Testa, Hurwitz & Thib eault
      (B) STREET: 53 State Street
      (C) CITY: Boston
      (D) STATE: Massachusetts
      (E) COUNTRY: U.S.A.
      (F) ZIP: 02109
   (v) COMPUTER READABLE FORM:
      (A) MEDIUM TYPE: Floppy Disk
      (B) COMPUTER: IBM XT
      (C) OPERATING SYSTEM: DOS 3.30
      (D) SOFTWARE: PatentIn Release 1.0, Version 1.25
   (vi) CURRENT APPLICATION DATA:
      (B) FILING DATE:
   (vii) PRIOR APPLICATION DATA:
      (A) APPLICATION NUMBER: US 752,857
      (B) FILING DATE: 30-AUG-1991
   (viii) PRIOR APPLICATION DATA:
      (A) APPLICATION NUMBER: US 667,274
      (B) FILING DATA: 11-MAR-1991
(2) INFORMATION FOR SEQ ID NO:1:
   (i) SEQUENCE CHARACTERISTICS:
      (A) LENGTH: 97 amino acids
      (B) TYPE: amino acids
      (C) TOPOLOGY: linear
   (ii) MOLECULE TYPE: protein
   (ix) FEATURE:
      (A) NAME: Generic Sequence 1
      (D) OTHER INFORMATION: Each Xaa indicates one of the 20 naturally-occurring L-isomer, α-amino acids or a derivative thereof.
   (xi) SEQUENCE DESCRIPTION: SEQ ID NO:1:
   Xaa Xaa Xaa Xaa Xaa Xaa
     1               5
   Xaa Xaa Xaa Xaa Xaa Xaa Xaa Xaa Xaa Xaa Xaa
               10                  15
   Xaa Xaa Xaa Xaa Xaa Xaa Xaa Cys Xaa Xaa Xaa
           20                  25
   Cys Xaa Xaa Xaa Xaa Xaa Xaa Xaa Xaa Xaa Xaa
       30                  35
   Xaa Xaa Xaa Xaa Xaa Xaa Xaa Xaa Xaa Xaa Xaa
   40                  45                  50
   Xaa Xaa Xaa Xaa Xaa Xaa Xaa Xaa Xaa Xaa Cys
                   55                  60
   Cys Xaa Xaa Xaa Xaa Xaa Xaa Xaa Xaa Xaa Xaa
               65                  70
   Xaa Xaa Xaa Xaa Xaa Xaa Xaa Xaa Xaa Xaa Xaa
           75                  80
   Xaa Xaa Xaa Xaa Xaa Xaa Xaa Xaa Xaa Xaa Cys
       85                  90
   Xaa Cys Xaa
   95
(2) INFORMATION FOR SEQ ID NO:2:
   (i) SEQUENCE CHARACTERISTICS:
      (A) LENGTH: 97 amino acids
      (B) TYPE: amino acids
      (C) TOPOLOGY: linear
   (ii) MOLECULE TYPE: protein
   (ix) FEATURE:
      (A) NAME: Generic Sequence 2
      (D) OTHER INFORMATION: Each Xaa indicates one of the 20 naturally-occurring L-isomer, α-amino acids or a derivative thereof.
   (xi) SEQUENCE DESCRIPTION: SEQ ID NO:2:
   Xaa Xaa Xaa Xaa Xaa Xaa
    1               5
   Xaa Xaa Xaa Xaa Xaa Xaa Xaa Xaa Xaa Xaa Xaa
                10                  15
   Xaa Xaa Xaa Xaa Xaa Xaa Xaa Cys Xaa Xaa Xaa
            20                  25
   Cys Xaa Xaa Xaa Xaa Xaa Xaa Cys Xaa Xaa Xaa
        30                  35
   Xaa Xaa Xaa Xaa Xaa Xaa Xaa Xaa Xaa Xaa Xaa
   40                  45                  50
   Xaa Xaa Xaa Xaa Xaa Xaa Xaa Xaa Xaa Xaa Cys
                   55                  60
   Cys Xaa Xaa Xaa Xaa Xaa Xaa Xaa Xaa Xaa Xaa
               65                  70
   Xaa Xaa Xaa Xaa Xaa Xaa Xaa Xaa Xaa Xaa Xaa
           75                  80
   Xaa Xaa Xaa Xaa Xaa Xaa Xaa Xaa Xaa Xaa Cys
       85                  90
   Xaa Cys Xaa
    95
(2) INFORMATION FOR SEQ ID NO:3:
   (i) SEQUENCE CHARACTERISTICS:
      (A) LENGTH: 97 amino acids
      (B) TYPE: amino acids
      (C) TOPOLOGY: linear
   (ii) MOLECULE TYPE: protein
   (ix) FEATURE:
      (A) NAME: Generic Sequence 3
      (D) OTHER INFORMATION: wherein each Xaa is independently selected from a group of one or more specified amino acids as defined in the specification.
   (xi) SEQUENCE DESCRIPTION: SEQ ID NO:3:
   Leu Tyr Val Xaa Phe
   1               5
   Xaa Xaa Xaa Gly Trp Xaa Xaa Trp Xaa
                   10
   Xaa Ala Pro Gly Xaa Xaa Xaa Ala
   15                  20
   Xaa Tyr Cys Xaa Gly Xaa Cys Xaa
           25                  30
   Xaa Pro Xaa Xaa Xaa Xaa Xaa
                   35
   Xaa Xaa Xaa Asn His Ala Xaa Xaa
           40                  45
   Xaa Xaa Leu Xaa Xaa Xaa Xaa Xaa
                   50
   Xaa Xaa Xaa Xaa Xaa Xaa Xaa Cys
       55                  60
   Cys Xaa Pro Xaa Xaa Xaa Xaa Xaa
               65
   Xaa Xaa Xaa Leu Xaa Xaa Xaa
   70                  75
   Xaa Xaa Xaa Xaa Val Xaa Leu Xaa
               80
   Xaa Xaa Xaa Xaa Met Xaa Val Xaa
   85                  90
   Xaa Cys Gly Cys Xaa
           95
(2) INFORMATION FOR SEQ ID NO:4:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: Generic Sequence 4
      (D) OTHER INFORMATION: wherein each Xaa is independently selected from a group of one or more specified amino acids as defined in the specification.
   (xi) SEQUENCE DESCRIPTION: SEQ ID NO:4:
   Cys Xaa Xaa Xaa Xaa Leu Tyr Val Xaa Phe
    1               5                   10
   Xaa Xaa Xaa Gly Trp Xaa Xaa Trp Xaa
                   15
   Xaa Ala Pro Xaa Gly Xaa Xaa Ala
    20                  25
   Xaa Tyr Cys Xaa Gly Xaa Cys Xaa
           30                  35
   Xaa Pro Xaa Xaa Xaa Xaa Xaa
                   40
   Asn Xaa Xaa Asn His Ala Xaa Xaa
           45                  50
   Xaa Xaa Leu Xaa Xaa Xaa Xaa Xaa
                   55
   Xaa Xaa Xaa Xaa Xaa Xaa Xaa Cys
       60                  65
   Cys Xaa Pro Xaa Xaa Xaa Xaa Xaa
               70
   Xaa Xaa Xaa Leu Xaa Xaa Xaa
    75                  80
   Xaa Xaa Xaa Xaa Val Xaa Leu Xaa
               85
   Xaa Xaa Xaa Xaa Met Xaa Val Xaa
    90                  95
   Xaa Cys Gly Cys Xaa
           100
(2) INFORMATION FOR SEQ ID NO:5:
   (i) SEQUENCE CHARACTERISTICS:
      (A) LENGTH: 139 amino acids
      (B) TYPE: amino acids
      (C) TOPOLOGY: linear
   (ii) MOLECULE TYPE: protein
   (ix) FEATURE:
      (A) NAME: hOP-1 (mature form)
   (xi) SEQUENCE DESCRIPTION: SEQ ID NO:5:
   Ser Thr Gly Ser Lys Gln Arg Ser Gln
     1             5
   Asn Arg Ser Lys Thr Pro Lys Asn Gln
    10                 15
   Glu Ala Leu Arg Met Ala Asn Val Ala
        20                 25
   Glu Asn Ser Ser Ser Asp Gln Arg Gln
            30                 35
   Ala Cys Lys Lys His Glu Leu Tyr Val
                40                 45
   Ser Phe Arg Asp Leu Gly Trp Gln Asp
                    50
   Trp Ile Ile Ala Pro Glu Gly Tyr Ala
    55                 60
   Ala Tyr Tyr Cys Glu Gly Glu Cys Ala
        65                 70
   Phe Pro Leu Asn Ser Tyr Met Asn Ala
            75                 80
   Thr Asn His Ala Ile Val Gln Thr Leu
                85                 90
   Val His Phe Ile Asn Pro Glu Thr Val
                    95
   Pro Lys Pro Cys Cys Ala Pro Thr Gln
   100                 105
   Leu Asn Ala Ile Ser Val Leu Tyr Phe
       110                 115
   Asp Asp Ser Ser Asn Val Ile Leu Lys
           120                 125
   Lys Tyr Arg Asn Met Val Val Arg Ala
               130                 135
   Cys Gly Cys His
(2) INFORMATION FOR SEQ ID NO:6:
   (i) SEQUENCE CHARACTERISTICS:
      (A) LENGTH: 139 amino acids
      (B) TYPE: amino acids
      (C) TOPOLOGY: linear
   (ii) MOLECULE TYPE: protein
   (ix) FEATURE:
      (A) NAME: mOP-1 (mature form)
   (xi) SEQUENCE DESCRIPTION: SEQ ID NO:6:
   Ser Thr Gly Gly Lys Gln Arg Ser Gln
     1                 5
   Asn Arg Ser Lys Thr Pro Lys Asn Gln
    10                     15
   Glu Ala Leu Arg Met Ala Ser Val Ala
        20                     25
   Glu Asn Ser Ser Ser Asp Gln Arg Gln
            30                     35
   Ala Cys Lys Lys His Glu Leu Tyr Val
               40                  45
   Ser Phe Arg Asp Leu Gly Trp Gln Asp
                   50
   Trp Ile Ile Ala Pro Glu Gly Tyr Ala
   55                  60
   Ala Tyr Tyr Cys Glu Gly Glu Cys Ala
       65                  70
   Phe Pro Leu Asn Ser Tyr Met Asn Ala
           75                  80
   Thr Asn His Ala Ile Val Gln Thr Leu
               85                  90
   Val His Phe Ile Asn Pro Asp Thr Val
                   95
   Pro Lys Pro Cys Cys Ala Pro Thr Gln
   100                 105
   Leu Asn Ala Ile Ser Val Leu Tyr Phe
       110                 115
   Asp Asp Ser Ser Asn Val Ile Leu Lys
           120                 125
   Lys Tyr Arg Asn Met Val Val Arg Ala
               130                 135
   Cys Gly Cys His
(2) INFORMATION FOR SEQ ID NO:7:
   (i) SEQUENCE CHARACTERISTICS:
      (A) LENGTH: 139 amino acids
      (B) TYPE: amino acids
      (C) TOPOLOGY: linear
   (ii) MOLECULE TYPE: protein
   (ix) FEATURE:
      (A) NAME: hOP-2 (mature form)
   (xi) SEQUENCE DESCRIPTION: SEQ ID NO:7:
   Ala Val Arg Pro Leu Arg Arg Arg Gln
   1               5
   Pro Lys Lys Ser Asn Glu Leu Pro Gln
   10                  15
   Ala Asn Arg Leu Pro Gly Ile Phe Asp
       20                  25
   Asp Val His Gly Ser His Gly Arg Gln
           30                  35
   Val Cys Arg Arg His Glu Leu Tyr Val
               40                  45
   Ser Phe Gln Asp Leu Gly Trp Leu Asp
                   50
   Trp Val Ile Ala Pro Gln Gly Tyr Ser
   55                  60
   Ala Tyr Tyr Cys Glu Gly Glu Cys Ser
       65                  70
   Phe Pro Leu Asp Ser Cys Met Asn Ala
           75                  80
   Thr Asn His Ala Ile Leu Gln Ser Leu
               85                  90
   Val His Leu Met Lys Pro Asn Ala Val
                   95
   Pro Lys Ala Cys Cys Ala Pro Thr Lys
   100                 105
   Leu Ser Ala Thr Ser Val Leu Tyr Tyr
       110                 115
   Asp Ser Ser Asn Asn Val Ile Leu Arg
           120                 125
   Lys His Arg Asn Met Val Val Lys Ala
               130                 135
   Cys Gly Cys His
(2) INFORMATION FOR SEQ ID NO:8:
   (i) SEQUENCE CHARACTERISTICS:
      (A) LENGTH: 139 amino acids
      (B) TYPE: amino acids
      (C) TOPOLOGY: linear
   (ii) MOLECULE TYPE: protein
   (ix) FEATURE:
      (A) NAME: mOP-2 (mature form)
   (xi) SEQUENCE DESCRIPTION: SEQ ID NO:8:
   Ala Ala Arg Pro Leu Lys Arg Arg Gln
    1              5
   Pro Lys Lys Thr Asn Glu Leu Pro His
   10                  15
   Pro Asn Lys Leu Pro Gly Ile Phe Asp
       20                  25
   Asp Gly His Gly Ser Arg Gly Arg Glu
           30                  35
   Val Cys Arg Arg His Glu Leu Tyr Val
               40                  45
   Ser Phe Arg Asp Leu Gly Trp Leu Asp
                   50
   Trp Val Ile Ala Pro Gln Gly Tyr Ser
   55                  60
   Ala Tyr Tyr Cys Glu Gly Glu Cys Ala
       65                  70
   Phe Pro Leu Asp Ser Cys Met Asn Ala
           75                  80
   Thr Asn His Ala Ile Leu Gln Ser Leu
               85                  90
   Val His Leu Met Lys Pro Asp Val Val
                   95
   Pro Lys Ala Cys Cys Ala Pro Thr Lys
   100                 105
   Leu Ser Ala Thr Ser Val Leu Tyr Tyr
       110                 115
   Asp Ser Ser Asn Asn Val Ile Leu Arg
           120                 125
   Lys His Arg Asn Met Val Val Lys Ala
               130                 135
   Cys Gly Cys His
(2) INFORMATION FOR SEQ ID NO:9:
   (i) SEQUENCE CHARACTERISTICS:
      (A) LENGTH: 96 amino acids
      (B) TYPE: amino acids
      (C) TOPOLOGY: linear
   (ii) MOLECULE TYPE: protein
   (ix) FEATURE:
      (A) NAME: CBMP2A(fx)
   (xi) SEQUENCE DESCRIPTION: SEQ ID NO:9:
(2) INFORMATION FOR SEQ ID NO:10:
   (i) SEQUENCE CHARACTERISTICS:
      (A) LENGTH: 101 amino acids
      (B) TYPE: amino acids
      (C) TOPOLOGY: linear
   (ii) MOLECULE TYPE: protein
   (ix) FEATURE:
      (A) NAME: CBMP2B(fx)
   (xi) SEQUENCE DESCRIPTION: SEQ ID NO:10:
   Cys Arg Arg His Ser
    1               5
   Leu Tyr Val Asp Phe Ser Asp Val Gly Trp Asn
                    10                  15
   Asp Trp Ile Val Ala Pro Pro Gly Tyr Gln Ala
                20                  25
   Phe Tyr Cys His Gly Asp Cys Pro Phe Pro Leu
            30                  35
   Ala Asp His Leu Asn Ser Thr Asn His Ala Ile
        40                  45
   Val Gln Thr Leu Val Asn Ser Val Asn Ser Ser
    50                  55                  60
   Ile Pro Lys Ala Cys Cys Val Pro Thr Glu Leu
                    65                  70
   Ser Ala Ile Ser Met Leu Tyr Leu Asp Glu Tyr
                75                  80
   Asp Lys Val Val Leu Lys Asn Tyr Gln Glu Met
            85                  90
   Val Val Glu Gly Cys Gly Cys Arg
        95                 100
(2) INFORMATION FOR SEQ ID NO:11:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: DPP(fx)
   (xi) SEQUENCE DESCRIPTION: SEQ ID NO:11:
   Cys Arg Arg His Ser Leu Tyr Val Asp Phe Ser
   1 5 10
   Asp Val Gly Trp Asp Asp Trp Ile Val Ala Pro
   15 20
   Leu Gly Tyr Asp Ala Tyr Tyr Cys His Gly Lys
   25 30
   Cys Pro Phe Pro Leu Ala Asp His Phe Asn Ser
   35 40
   Thr Asn His Ala Val Val Gln Thr Leu Val Asn
   45 50 55
   Asn Asn Asn Pro Gly Lys Val Pro Lys Ala Cys
   60 65
   Cys Val Pro Thr Gln Leu Asp Ser Val Ala Met
   70 75
   Leu Tyr Leu Asn Asp Gln Ser Thr Val Val Leu
   80 85
   Lys Asn Tyr Gln Glu Met Thr Val Val Gly Cys
   90 95
   Gly Cys Arg
   100
(2) INFORMATION FOR SEQ ID N0:12:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: Vgl(fx)
   (xi) SEQUENCE DESCRIPTION: SEQ ID NO:12:
(2) INFORMATION FOR SEQ ID NO:13:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: Vgr-1(fx)
   (xi) SEQUENCE DESCRIPTION: SEQ ID NO:13:
   Cys Lys Lys His Glu Leu Tyr Val Ser Phe Gln
   1 5 10
   Asp Val Gly Trp Gln Asp Trp Ile Ile Ala Pro
   15 20
   Xaa Gly Tyr Ala Ala Asn Tyr Cys Asp Gly Glu
   25 30
   Cys Ser Phe Pro Leu Asn Ala His Met Asn Ala
   35 40
   Thr Asn His Ala Ile Val Gln Thr Leu Val His
   45 50 55
   Val Met Asn Pro Glu Tyr Val Pro Lys Pro Cys
   60 65
   Cys Ala Pro Thr Lys Val Asn Ala Ile Ser Val
   70 75
   Leu Tyr Phe Asp Asp Asn Ser Asn Val Ile Leu
   80 85
   Lys Lys Tyr Arg Asn Met Val Val Arg Ala Cys
   90 95
   Gly Cys His
   100
(2) INFORMATION FOR SEQ ID NO:14:
   (i) SEQUENCE CHARACTERISTICS:
      (A) LENGTH: 106 amino acids
      (B) TYPE: protein
      (C) STRANDEDNESS: single
      (D) TOPOLOGY: linear
   (ii) MOLECULE TYPE: protein
   (vi) ORIGINAL SOURCE:
      (A) ORGANISM: human
      (F) TISSUE TYPE: BRAIN
   (ix) FEATURE:
      (D) OTHER INFORMATION: /product= "GDF-1 (fx)"
   (xi) SEQUENCE DESCRIPTION: SEQ ID NO:14:
   Cys Arg Ala Arg Arg Leu Tyr Val Ser Phe Arg Glu Val Gly
    1               5                   10
   Trp His Arg Trp Val Ile Ala Pro Arg Gly Phe Leu Ala Asn Tyr
    15                  20                  25
   Cys Gln Gly Gln Cys Ala Leu Pro Val Ala Leu Ser Gly Ser Gly
    30                  35                  40
   Gly Pro Pro Ala Leu Asn His Ala Val Leu Arg Ala Leu Met His
    45                  50                  55
   Ala Ala Ala Pro Gly Ala Ala Asp Leu Pro Cys Cys Val Pro Ala
    60                  65                  70
   Arg Leu Ser Pro Ile Ser Val Leu Phe Phe Asp Asn Ser Asp Asn
    75                  80                  85
   Val Val Leu Arg Gln Tyr Glu Asp Met Val Val Asp Glu Cys Gly
    90                  95                  100
   Cys Arg
   105
(2) INFORMATION FOR SEQ ID NO:15:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:15:
   Cys Xaa Xaa Xaa Xaa
    1               5
(2) INFORMATION FOR SEQ ID NO:16:
   (i) SEQUENCE CHARACTERISTICS:
      (A) LENGTH: 1822 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: HOMO SAPIENS
      (F) TISSUE TYPE: HIPPOCAMPUS
   (ix) FEATURE:
      (A) NAME/KEY: CDS
      (B) LOCATION: 49..1341
      (D) OTHER INFORMATION:/standard_name= "hOP1"
   (xi) SEQUENCE DESCRIPTION: SEQ ID NO:16:
(2) INFORMATION FOR SEQ ID NO:17:
   (i) SEQUENCE CHARACTERISTICS:
      (A) LENGTH: 431 amino acids
      (B) TYPE: amino acid
      (D) TOPOLOGY: linear
   (ii) MOLECULE TYPE: protein
   (ix) FEATURE:
      (D) OTHER INFORMATION: /Product="OP1-PP"
   (xi) SEQUENCE DESCRIPTION: SEQ ID NO:17:
(2) INFORMATION FOR SEQ ID NO:18:
   (i) SEQUENCE CHARACTERISTICS:
      (A) LENGTH: 1873 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: HURIDAE
      (F) TISSUE TYPE: EMBRYO
   (ix) FEATURE:
      (A) NAME/KEY: CDS
      (B) LOCATION: 104..1393
      (D) OTHER INFORMATION: /note= "MOP1 (CDNA)"
   (xi) SEQUENCE DESCRIPTION: SEQ ID NO:18:
(2) INFORMATION FOR SEQ ID NO:19:
   (i) SEQUENCE CHARACTERISTICS:
      (A) LENGTH: 430 amino acids
      (B) TYPE: amino acid
      (D) TOPOLOGY: linear
   (ii) MOLECULE TYPE: protein
   (ix) FEATURE:
      (D) OTHER INFORMATION: /product= "mOP1-PP"
   (xi) SEQUENCE DESCRIPTION: SEQ ID NO:19:
   Met His Val Arg Ser Leu Arg Ala Ala Ala Pro His Ser Phe Val Ala
    1               5                  10                  15
   Leu Trp Ala Pro Leu Phe Leu Leu Arg Ser Ala Leu Ala Asp Phe Ser
               20                  25                  30
   Leu Asp Asn Glu Val His Ser Ser Phe Ile His Arg Arg Leu Arg Ser
           35                  40                  45
   Gln Glu Arg Arg Glu Met Gln Arg Glu Ile Leu Ser Ile Leu Gly Leu
       50                  55                  60
   Pro His Arg Pro Arg Pro His Leu Gln Gly Lys His Asn Ser Ala Pro
   65                  70                  75                  80
   Met Phe Met Leu Asp Leu Tyr Asn Ala Met Ala Val Glu Glu Ser Gly
                   85                  90                  95
   Pro Asp Gly Gln Gly Phe Ser Tyr Pro Tyr Lys Ala Val Phe Ser Thr
               100                 105                 110
   Gln Gly Pro Pro Leu Ala Ser Leu Gln Asp Ser His Phe Leu Thr Asp
           115                 120                 125
   Ala Asp Met Val Met Ser Phe Val Asn Leu Val Glu His Asp Lys Glu
       130                 135                 140
   Phe Phe His Pro Arg Tyr His His Arg Glu Phe Arg Phe Asp Leu Ser
   145                 150                 155                 160
   Lys Ile Pro Glu Gly Glu Arg Val Thr Ala Ala Glu Phe Arg Ile Tyr
   165 170 175
   Lys Asp Tyr Ile Arg Glu Arg Phe Asp Asn Glu Thr Phe Gln Ile Thr
   180 185 190
   Val Tyr Gln Val Leu Gln Glu His Ser Gly Arg Glu Ser Asp Leu Phe
   195 200 205
   Leu Leu Asp Ser Arg Thr Ile Trp Ala Ser Glu Glu Gly Trp Leu Val
   210 215 220
   Phe Asp Ile Thr Ala Thr Ser Asn His Trp Val Val Asn Pro Arg His
   225 230 235 240
   Asn Leu Gly Leu Gln Leu Ser Val Glu Thr Leu Asp Gly Gln Ser Ile
   245 250 255
   Asn Pro Lys Leu Ala Gly Leu Ile Gly Arg His Gly Pro Gln Asn Lys
   260 265 270
   Gln Pro Phe Met Val Ala Phe Phe Lys Ala Thr Glu Val His Leu Arg
   275 280 285
   Ser Ile Arg Ser Thr Gly Gly Lys Gln Arg Ser Gln Asn Arg Ser Lys
   290 295 300
   Thr Pro Lys Asn Gln Glu Ala Leu Arg Met Ala Ser Val Ala Glu Asn
   305 310 315 320
   Ser Ser Ser Asp Gln Arg Gln Ala Cys Lys Lys His Glu Leu Tyr Val
   325 330 335
   Ser Phe Arg Asp Leu Gly Trp Gln Asp Trp Ile Ile Ala Pro Glu Gly
   340 345 350
   Tyr Ala Ala Tyr Tyr Cys Glu Gly Glu Cys Ala Phe Pro Leu Asn Ser
   355 360 365
   Tyr Met Asn Ala Thr Asn His Ala Ile Val Gln Thr Leu Val His Phe
   370 375 380
   Ile Asn Pro Asp Thr Val Pro Lys Pro Cys Cys Ala Pro Thr Gln Leu
   385 390 395 400
   Asn Ala Ile Ser Val Leu Tyr Phe Asp Asp Ser Ser Asn Val Ile Leu
   405 410 415
   Lys Lys Tyr Arg Asn Met Val Val Arg Ala Cys Gly Cys His
   420 425 430
(2) INFORMATION FOR SEQ ID NO:20:
   (i)SEQUENCE CHARACTERISTICS:
      (A) LENGTH: 1723 base pairs
      (B) TYPE: nucleic acid
      (C) STRANDEDNESS: single
      (D) TOPOLOGY: linear
   (ii)MOLECULE TYPE: cDNA
   (vi)ORIGINAL SOURCE:
      (A) ORGANISM: Homo sapiens
      (F) TISSUE TYPE: HIPPOCAMPUS
   (ix)FEATURE:
      (A) NAME/KEY: CDS
      (B) LOCATION: 490..1696
      (D) OTHER INFORMATION: /note= "hOP2 (cDNA)"
   (xi)SEQUENCE DESCRIPTION: SEQ ID NO:20:
   GGCGCCGGCA GAGCAGGAGT GGCTGGAGGA GCTGTGGTTG GAGCAGGAGG TGGCACGGCA      60
   GGGCTGGAGG GCTCCCTATG AGTGGCGGAG ACGGCCCAGG AGGCGCTGGA GCAACAGCTC     120
   CCACACCGCA CCAAGCGGTG GCTGCAGGAG CTCGCCCATC GCCCCTGCGC TGCTCGGACC     180
   GCGGCCACAG CCGGACTGGC GGGTACGGCG GCGACAGAGG CATTGGCCGA GAGTCCCAGT     240
   CCGCAGAGTA GCCCCGGCCT CGAGGCGGTG GCGTCCCGGT CCTCTCCGTC CAGGAGCCAG     300
   GACAGGTGTC GCGCGGCGGG GCTCCAGGGA CCGCGCCTGA GGCCGGCTGC CCGCCCGTCC     360
   CGCCCCGCCC CGCCGCCCGC CGCCCGCCGA GCCCAGCCTC CTTGCCGTCG GGGCGTCCCC     420
   AGGCCCTGGG TCGGCCGCGG AGCCGATGCG CGCCCGCTGA GCGCCCCAGC TGAGCGCCCC     480
   CGGCCTGCC ATG ACC GCG CTC CCC GGC CCG CTC TGG CTC CTG GGC CTG         528
   GCG CTA TGC GCG CTG GGC GGG GGC GGC CCC GGC CTG CGA CCC CCG CCC       576
   GGC TGT CCC CAG CGA CGT CTG GGC GCG CGC GAG CGC CGG GAC GTG CAG       624
   CGC GAG ATC CTG GCG GTG CTC GGG CTG CCT GGG CGG CCC CGG CCC CGC       672
   GCG CCA CCC GCC GCC TCC CGG CTG CCC GCG TCC GCG CCG CTC TTC ATG     720
   CTG GAC CTG TAC CAC GCC ATG GCC GGC GAC GAC GAC GAG GAC GGC GCG     768
   CCC GCG GAG CGG CGC CTG GGC CGC GCC GAC CTG GTC ATG AGC TTC GTT     816
   AAC ATG GTG GAG CGA GAC CGT GCC CTG GGC CAC CAG GAG CCC CAT TGG     864
   AAG GAG TTC CGC TTT GAC CTG ACC CAG ATC CCG GCT GGG GAG GCG GTC     912
   ACA GCT GCG GAG TTC CGG ATT TAC AAG GTG CCC AGC ATC CAC CTG CTC     960
   AAC AGG ACC CTC CAC GTC AGC ATG TTC CAG GTG GTC CAG GAG CAG TCC    1008
   AAC AGG GAG TCT GAC TTG TTC TTT TTG GAT CTT CAG ACG CTC CGA GCT    1056
   GGA GAC GAG GGC TGG CTG GTG CTG GAT GTC ACA GCA GCC AGT GAC TGC    1104
   TGG TTG CTG AAG CGT CAC AAG GAC CTG GGA CTC CGC CTC TAT GTG GAG    1152
   ACT GAG GAC GGG CAC AGC GTG GAT CCT GGC CTG GCC GGC CTG CTG GGT    1200
   CAA CGG GCC CCA CGC TCC CAA CAG CCT TTC GTG GTC ACT TTC TTC AGG    1248
   GCC AGT CCG AGT CCC ATC CGC ACC CCT CGG GCA GTG AGG CCA CTG AGG    1296
   AGG AGG CAG CCG AAG AAA AGC AAC GAG CTG CCG CAG GCC AAC CGA CTC     1344
   CCA GGG ATC TTT GAT GAC GTC CAC GGC TCC CAC GGC CGG CAG GTC TGC     1392
   CGT CGG CAC GAG CTC TAC GTC AGC TTC CAG GAC CTC GGC TGG CTG GAC     1440
   TGG GTC ATC GCT CCC CAA GGC TAC TCG GCC TAT TAC TGT GAG GGG GAG     1488
   TGC TCC TTC CCA CTG GAC TCC TGC ATG AAT GCC ACC AAC CAC GCC ATC     1536
   CTG CAG TCC CTG GTG CAC CTG ATG AAG CCA AAC GCA GTC CCC AAG GCG     1584
   TGC TGT GCA CCC ACC AAG CTG AGC GCC ACC TCT GTG CTC TAC TAT GAC     1632
   AGC AGC AAC AAC GTC ATC CTG CGC AAA CAC CGC AAC ATG GTG GTC AAG     1680
   GCC TGC GGC TGC CAC T GAGTCAGCCC GCCCAGCCCT ACTGCAG                 1723
(2) INFORMATION FOR SEQ ID NO:21:
   (i)SEQUENCE CHARACTERISTICS:
      (A) LENGTH: 402 amino acids
      (B) TYPE: amino acid
      (D) TOPOLOGY: linear
   (ii)MOLECULE TYPE: protein
   (ix)FEATURE:
      (A)OTHER INFORMATION: /product= "hOP2-PP"
   (xi)SEQUENCE DESCRIPTION: SEQ ID NO:21:
(2) INFORMATION FOR SEQ ID NO:22:
   (i) SEQUENCE CHARACTERISTICS:
      (A) LENGTH: 1926 base pairs
      (B) TYPE: nucleic acid
      (C) STRANDEDNESS: single
      (D TOPOLOGY: linear
   (ii) HOLECULE TYPE: cDNA
   (vi) ORIGINAL SOURCE:
      (A) ORGANISM: HURIDAE
      (F) TISSUE TYPE: EMBRYO
   (ix) FEATURE:
      (A) NAME/KEY: CDS
      (B) LOCATION: 93..1289
      (D) OTHER INFORMATION: /note= "mOP2 cDNA"
   (xi) SEQUENCE DESCRIPTION: SEQ ID NO:22:
   GCCAGGCACA GGTGCGCCGT CTGGTCCTCC CCGTCTGGCG TCAGCCGAGC         50
   CCGACCAGCT ACCAGTGGAT GCGCGCCGGC TGAAAGTCCG AG ATG GCT ATG CGT    104
                                                  Met Ala Met Arg
                                                    1
   CCC GGG CCA CTC TGG CTA TTG GGC CTT GCT CTG TGC GCG CTG GGA GGC   152
   Pro Gly Pro Leu Trp Leu Leu Gly Leu Ala Leu Cys Ala Leu Gly Gly
     5                  10                  15                  20
   GGC CAC GGT CCG CGT CCC CCG CAC ACC TGT CCC CAG CGT CGC CTG GGA    200
   Gly His Gly Pro Arg Pro Pro His Thr Cys Pro Gln Arg Arg Leu Gly
                   25                  30                  35
   GCG CGC GAG CGC CGC GAC ATG CAG CGT GAA ATC CTG GCG GTG CTC GGG    248
   Ala Arg Glu Arg Arg Asp Met Gln Arg Glu Ile Leu Ala Val Leu Gly
               40                  45                  50
   CTA CCG GGA CGG CCC CGA CCC CGT GCA CAA CCC GCG GCT GCC CGG CAG    296
   Leu Pro Gly Arg Pro Arg Pro Arg Ala Gln Pro Ala Ala Ala Arg Gln
           55                  60                  65
   CCA GCG TCC GCG CCC CTC TTC ATG TTG GAC CTA TAC CAC GCC ATG ACC    344
   Pro Ala Ser Ala Pro Leu Phe Met Leu Asp Leu Tyr His Ala Met Thr
       70                  75                  80
   GAT GAC GAC GAC GGC GGG CCA CCA CAG GCT CAC TTA GGC CGT GCC GAC    392
   Asp Asp Asp Asp Gly Gly Pro Pro Gln Ala His Leu Gly Arg Ala Asp
   85                  90                  95                  100
   CTG GTC ATG AGC TTC GTC AAC ATG GTG GAA CGC GAC CGT ACC CTG GGC    440
   Leu Val Met Ser Phe Val Asn Met Val Glu Arg Asp Arg Thr Leu Gly
                   105                 110                 115
   TAC CAG GAG CCA CAC TGG AAG GAA TTC CAC TTT GAC CTA ACC CAG ATC    488
   Tyr Gln Glu Pro His Trp Lys Glu Phe His Phe Asp Leu Thr Gln Ile
               120                 125                 130
   CCT GCT GGG GAG GCT GTC ACA GCT GCT GAG TTC CGG ATC TAC AAA GAA    536
   Pro Ala Gly Glu Ala Val Thr Ala Ala Glu Phe Arg Ile Tyr Lys Glu
           135                 140                 145
   CCC AGC ACC CAC CCG CTC AAC ACA ACC CTC CAC ATC AGC ATG TTC GAA    584
   Pro Ser Thr His Pro Leu Asn Thr Thr Leu His Ile Ser Met Phe Glu
       150                 155                 160
   GTG GTC CAA GAG CAC TCC AAC AGG GAG TCT GAC TTG TTC TTT TTG GAT    632
   Val Val Gln Glu His Ser Asn Arg Glu Ser Asp Leu Phe Phe Leu Asp
   165                 170                 175                 180
   CTT CAG ACG CTC CGA TCT GGG GAC GAG GGC TGG CTG GTG CTG GAC ATC    680
   Leu Gln Thr Leu Arg Ser Gly Asp Glu Gly Trp Leu Val Leu Asp Ile
                   185                 190                 195
   ACA GCA GCC AGT GAC CGA TGG CTG CTG AAC CAT CAC AAG GAC CTG GGA    728
   Thr Ala Ala Ser Asp Arg Trp Leu Leu Asn His His Lys Asp Leu Gly
               200                 205                 210
   CTC CGC CTC TAT GTG GAA ACC GCG GAT GGG CAC AGC ATG GAT CCT GGC    776
   Leu Arg Leu Tyr Val Glu Thr Ala Asp Gly His Ser Met Asp Pro Gly
           215                 220                 225
   CTG GCT GGT CTG CTT GGA CGA CAA GCA CCA CGC TCC AGA CAG CCT TTC      824
   Leu Ala Gly Leu Leu Gly Arg Gln Ala Pro Arg Ser Arg Gln Pro Phe
       230                 235                 240
   ATG GTA ACC TTC TTC AGG GCC AGC CAG AGT CCT GTG CGG GCC CCT CGG      872
   Met Val Thr Phe Phe Arg Ala Ser Gln Ser Pro Val Arg Ala Pro Arg
   245                 250                 255                 260
   GCA GCG AGA CCA CTG AAG AGG AGG CAG CCA AAG AAA ACG AAC GAG CTT      920
   Ala Ala Arg Pro Leu Lys Arg Arg Gln Pro Lys Lys Thr Asn Glu Leu
                   265                 270                 275
   CCG CAC CCC AAC AAA CTC CCA GGG ATC TTT GAT GAT GGC CAC GGT TCC      968
   Pro His Pro Asn Lys Leu Pro Gly Ile Phe Asp Asp Gly His Gly Ser
               280                 285                 290
   CGC GGC AGA GAG GTT TGC CGC AGG CAT GAG CTC TAC GTC AGC TTC CGT     1016
   Arg Gly Arg Glu Val Cys Arg Arg His Glu Leu Tyr Val Ser Phe Arg
           295                 300                 305
   GAC CTT GGC TGG CTG GAC TGG GTC ATC GCC CCC CAG GGC TAC TCT GCC     1064
   Asp Leu Gly Trp Leu Asp Trp Val Ile Ala Pro Gln Gly Tyr Ser Ala
       310                 315                 320
   TAT TAC TGT GAG GGG GAG TGT GCT TTC CCA CTG GAC TCC TGT ATG AAC     1112
   Tyr Tyr Cys Glu Gly Glu Cys Ala Phe Pro Leu Asp Ser Cys Met Asn
   325                 330                 335                 340
   GCC ACC AAC CAT GCC ATC TTG CAG TCT CTG GTG CAC CTG ATG AAG CCA     1160
   Ala Thr Asn His Ala Ile Leu Gln Ser Leu Val His Leu Met Lys Pro
                   345                 350                 355
   GAT GTT GTC CCC AAG GCA TGC TGT GCA CCC ACC AAA CTG AGT GCC ACC     1208
   Asp Val Val Pro Lys Ala Cys Cys Ala Pro Thr Lys Leu Ser Ala Thr
               360                 365                 370
   TCT GTG CTG TAC TAT GAC AGC AGC AAC AAT GTC ATC CTG CGT AAA CAC     1256
   Ser Val Leu Tyr Tyr Asp Ser Ser Asn Asn Val Ile Leu Arg Lys His
           375                 380                 385
   CGT AAC ATG GTG GTC AAG GCC TGT GGC TGC CAC TGAGGCCCCG CCCAGCATCC   1309
   Arg Asn Met Val Val Lys Ala Cys Gly Cys His
       390                 395
   TGCTTCTACT ACCTTACCAT CTGGCCGGGC CCCTCTCCAG AGGCAGAAAC CCTTCTATGT   1369
   TATCATAGCT CAGACAGGGG CAATGGGAGG CCCTTCACTT CCCCTGGCCA CTTCCTGCTA   1429
   AAATTCTGGT CTTTCCCAGT TCCTCTGTCC TTCATGGGGT TTCGGGGCTA TCACCCCGCC   1489
   CTCTCCATCC TCCTACCCCA AGCATAGACT GAATGCACAC AGCATCCCAG AGCTATGCTA   1549
   ACTGAGAGGT CTGGGGTCAG CACTGAAGGC CCACATGAGG AAGACTGATC CTTGGCCATC   1609
   CTCAGCCCAC AATGGCAAAT TCTGGATGGT CTAAGAAGGC CGTGGAATTC TAAACTAGAT   1669
   GATCTGGGCT CTCTGCACCA TTCATTGTGG CAGTTGGGAC ATTTTTAGGT ATAACAGACA   1729
   CATACACTTA GATCAATGCA TCGCTGTACT CCTTGAAATC AGAGCTAGCT TGTTAGAAAA   1789
   AGAATCAGAG CCAGGTATAG CGGTGCATGT CATTAATCCC AGCGCTAAAG AGACAGAGAC   1849
   AGGAGAATCT CTGTGAGTTC AAGGCCACAT AGAAAGAGCC TGTCTCGGGA GCAGGAAAAA   1909
   AAAAAAAAAC GGAATTC                                                  1926
(2) INFORMATION FOR SEQ ID NO:23:
   (i) SEQUENCE CHARACTERISTICS:
      (A) LENGTH: 399 amino acids
      (B) TYPE: amino acid
      (D) TOPOLOGY: linear
   (ii) MOLECULE TYPE: protein
   (ix) FEATURE:
      (D) OTHER INFORMATION: /product= "mOP2-PP"
   (xi) SEQUENCE DESCRIPTION: SEQ ID NO:23:
   Met Ala Met Arg Pro Gly Pro Leu Trp Leu Leu Gly Leu Ala Leu Cys
    1               5                  10                  15
   Ala Leu Gly Gly Gly His Gly Pro Arg Pro Pro His Thr Cys Pro Gln
               20                  25                  30
   Arg Arg Leu Gly Ala Arg Glu Arg Arg Asp Met Gln Arg Glu Ile Leu Ala
           35                  40                  45
   Val Leu Gly Leu Pro Gly Arg Pro Arg Pro Arg Ala Gln Pro Ala Ala
    50                  55                  60                  65
   Ala Arg Gln Pro Ala Ser Ala Pro Leu Phe Met Leu Asp Leu Tyr His Ala
                   70                  75                  80
   Met Thr Asp Asp Asp Asp Gly Gly Pro Pro Gln Ala His Leu Gly Arg
           85                  90                  95
   Ala Asp Leu Val Met Ser Phe Val Asn Met Val Glu Arg Asp Arg Thr
       100                 105                 110
   Leu Gly Tyr Gln Glu Pro His Trp Lys Glu Phe His Phe Asp Leu Thr
   115                 120                 125                 130
   Gln Ile Pro Ala Gly Glu Ala Val Thr Ala Ala Glu Phe Arg Ile Tyr
                   135                 140                 145
   Lys Glu Pro Ser Thr His Pro Leu Asn Thr Thr Leu His Ile Ser Met
               150                 155                 160
   Phe Glu Val Val Gln Glu His Ser Asn Arg Glu Ser Asp Leu Phe Phe
           165                 170                 175
   Leu Asp Leu Gln Thr Leu Arg Ser Gly Asp Glu Gly Trp Leu Val Leu
       180                 185                 190
   Asp Ile Thr Ala Ala Ser Asp Arg Trp Leu Leu Asn His His Lys Asp
   195                 200                 205                 210
   Leu Gly Leu Arg Leu Tyr Val Glu Thr Ala Asp Gly His Ser Met Asp
                   215                 220                 225
   Pro Gly Leu Ala Gly Leu Leu Gly Arg Gln Ala Pro Arg Ser Arg Gln
               230                 235                 240
   Pro Phe Met Val Thr Phe Phe Arg Ala Ser Gln Ser Pro Val Arg Ala
           245                 250                 255
   Pro Arg Ala Ala Arg Pro Leu Lys Arg Arg Gln Pro Lys Lys Thr Asn
       260                 265                 270
   Glu Leu Pro His Pro Asn Lys Leu Pro Gly Ile Phe Asp Asp Gly His
   275                 280                 285                 290
   Gly Ser Arg Gly Arg Glu Val Cys Arg Arg His Glu Leu Tyr Val Ser
                   295                 300                 305
   Phe Arg Asp Leu Gly Trp Leu Asp Trp Val Ile Ala Pro Gln Gly Tyr
               310                 315                 320
   Ser Ala Tyr Tyr Cys Glu Gly Glu Cys Ala Phe Pro Leu Asp Ser Cys
           325                 330                 335
   Met Asn Ala Thr Asn His Ala Ile Leu Gln Ser Leu Val His Leu Met
       340                 345                 350
   Lys Pro Asp Val Val Pro Lys Ala Cys Cys Ala Pro Thr Lys Leu Ser
   355                 360                 365                 370
   Ala Thr Ser Val Leu Tyr Tyr Asp Ser Ser Asn Asn Val Ile Leu Arg
                   375                 380                 385
   Lys His Arg Asn Met Val Val Lys Ala Cys Gly Cys His
               390                 395
(2) INFORMATION FOR SEQ ID NO:24:
   (i) SEQUENCE CHARACTERISTICS:
      (A) LENGTH: 1368 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1368
      (D) OTHER INFORHATION:/STANDARD NAME="60A"
   (x) PUBLICATION INFORMATION:
      (A) AUTHORS: WHARTON, KRISTI A.; THOMSEN, GERALD H.; GELBERT, WILLIAM M.
      (B) TITLE: DROSOPHILA 60A GENE...
      (C) JOURNAL: PROC. NAT'L ACAD. SCI. USA
      (D) VOLUME: 88
      (E) RELEVANT RESIDUES IN SEQ ID NO:3: FROM 1 TO 1368
      (F) PAGES: 9214-9218
      (G) DATE: OCT - 1991
   (xi) SEQUENCE DESCRIPTION: SEQ ID NO:24:
   ATG TCG GGA CTG CGA AAC ACC TCG GAG GCC GTT GCA GTG CTC GCC TCC        48
   Met Ser Gly Leu Arg Asn Thr Ser Glu Ala Val Ala Val Leu Ala Ser
     1               5                  10                  15
   CTG GGA CTC GGA ATG GTT CTG CTC ATG TTC GTG GCG ACC ACG CCG CCG        96
   Leu Gly Leu Gly Met Val Leu Leu Met Phe Val Ala Thr Thr Pro Pro
                20                  25                  30
   GCC GTT GAG GCC ACC CAG TCG GGG ATT TAC ATA GAC AAC GGC AAG GAC       144
   Ala Val Glu Ala Thr Gln Ser Gly Ile Tyr Ile Asp Asn Gly Lys Asp
            35                  40                  45
   CAG ACG ATC ATG CAC AGA GTG CTG AGC GAG GAC GAC AAG CTG GAC GTC       192
   Gln Thr Ile Met His Arg Val Leu Ser Glu Asp Asp Lys Leu Asp Val
        50                  55                  60
   TCG TAC GAG ATC CTC GAG TTC CTG GGC ATC GCC GAA CGG CCG ACG CAC       240
   Ser Tyr Glu Ile Leu Glu Phe Leu Gly Ile Ala Glu Arg Pro Thr His
    65                  70                  75                  80
   CTG AGC AGC CAC CAG TTG TCG CTG AGG AAG TCG GCT CCC AAG TTC CTG       288
   Leu Ser Ser His Gln Leu Ser Leu Arg Lys Ser Ala Pro Lys Phe Leu
                    85                  90                  95
   CTG GAC GTC TAC CAC CGC ATC ACG GCG GAG GAG GGT CTC AGC GAT CAG       336
   Leu Asp Val Tyr His Arg Ile Thr Ala Glu Glu Gly Leu Ser Asp Gln
               100                 105                 110
   GAT GAG GAC GAC GAC TAC GAA CGC GGC CAT CGG TCC AGG AGG AGC GCC     384
   Asp Glu Asp Asp Asp Tyr Glu Arg Gly His Arg Ser Arg Arg Ser Ala
           115                 120                 125
   GAC CTC GAG GAG GAT GAG GGC GAG CAG CAG AAG AAC TTC ATC ACC GAC     432
   Asp Leu Glu Glu Asp Glu Gly Glu Gln Gln Lys Asn Phe Ile Thr Asp
       130                 135                 140
   CTG GAC AAG CGG GCC ATC GAC GAG AGC GAC ATC ATC ATG ACC TTC CTG     480
   Leu Asp Lys Arg Ala Ile Asp Glu Ser Asp Ile Ile Met Thr Phe Leu
   145                 150                 155                 160
   AAC AAG CGC CAC CAC AAT GTG GAC GAA CTG CGT CAC GAG CAC GGC CGT     528
   Asn Lys Arg His His Asn Val Asp Glu Leu Arg His Glu His Gly Arg
                   165                 170                 175
   CGC CTG TGG TTC GAC GTC TCC AAC GTG CCC AAC GAC AAC TAC CTG GTG     576
   Arg Leu Trp Phe Asp Val Ser Asn Val Pro Asn Asp Asn Tyr Leu Val
               180                 185                 190
   ATG GCC GAG CTG CGC ATC TAT CAG AAC GCC AAC GAG GGC AAG TGG CTG     624
   Met Ala Glu Leu Arg Ile Tyr Gln Asn Ala Asn Glu Gly Lys Trp Leu
           195                 200                 205
   ACC GCC AAC AGG GAG TTC ACC ATC ACG GTA TAC GCC ATT GGC ACC GGC     672
   Thr Ala Asn Arg Glu Phe Thr Ile Thr Val Tyr Ala Ile Gly Thr Gly
       210                 215                 220
   ACG CTG GGC CAG CAC ACC ATG GAG CCG CTG TCC TCG GTG AAC ACC ACC     720
   Thr Leu Gly Gln His Thr Met Glu Pro Leu Ser Ser Val Asn Thr Thr
   225                 230                 235                 240
   GGG GAC TAC GTG GGC TGG TTG GAG CTC AAC GTG ACC GAG GGC CTG CAC     768
   Gly Asp Tyr Val Gly Trp Leu Glu Leu Asn Val Thr Glu Gly Leu His
                   245                 250                 255
   GAG TGG CTG GTC AAG TCG AAG GAC AAT CAT GGC ATC TAC ATT GGA GCA     816
   Glu Trp Leu Val Lys Ser Lys Asp Asn His Gly Ile Tyr Ile Gly Ala
               260                 265                 270
   CAC GCT GTC AAC CGA CCC GAC CGC GAG GTG AAG CTG GAC GAC ATT GGA     864
   His Ala Val Asn Arg Pro Asp Arg Glu Val Lys Leu Asp Asp Ile Gly
           275                 280                 285
   CTG ATC CAC CGC AAG GTG GAC GAC GAG TTC CAG CCC TTC ATG ATC GGC     912
   Leu Ile His Arg Lys Val Asp Asp Glu Phe Gln Pro Phe Met Ile Gly
       290                 295                 300
   TTC TTC CGC GGA CCG GAG CTG ATC AAG GCG ACG GCC CAC AGC AGC CAC     960
   Phe Phe Arg Gly Pro Glu Leu Ile Lys Ala Thr Ala His Ser Ser His
   305                 310                 315                 320
   CAC AGG AGC AAG CGA AGC GCC AGC CAT CCA CGC AAG CGC AAG AAG TCG        1008
   His Arg Ser Lys Arg Ser Ala Ser His Pro Arg Lys Arg Lys Lys Ser
                   325                 330                 335
   GTG TCG CCC AAC AAC GTG CCG CTG CTG GAA CCG ATG GAG AGC ACG CGC        1056
   Val Ser Pro Asn Asn Val Pro Leu Leu Glu Pro Met Glu Ser Thr Arg
               340                 345                 350
   AGC TGC CAG ATG CAG ACC CTG TAC ATA GAC TTC AAG GAT CTG GGC TGG        1104
   Ser Cys Gln Met Gln Thr Leu Tyr Ile Asp Phe Lys Asp Leu Gly Trp
           355                 360                 365
   CAT GAC TGG ATC ATC GCA CCA GAG GGC TAT GGC GCC TTC TAC TGC AGC        1152
   His Asp Trp Ile Ile Ala Pro Glu Gly Tyr Gly Ala Phe Tyr Cys Ser
       370                 375                 380
   GGC GAG TGC AAT TTC CCG CTC AAT GCG CAC ATG AAC GCC ACG AAC CAT        1200
   Gly Glu Cys Asn Phe Pro Leu Asn Ala His Met Asn Ala Thr Asn His
   385                 390                 395                 400
   GCG ATC GTC CAG ACC CTG GTC CAC CTG CTG GAG CCC AAG AAG GTG CCC        1248
   Ala Ile Val Gln Thr Leu Val His Leu Leu Glu Pro Lys Lys Val Pro
                   405                 410                 415
   AAG CCC TGC TGC GCT CCG ACC AGG CTG GGA GCA CTA CCC GTT CTG TAC        1296
   Lys Pro Cys Cys Ala Pro Thr Arg Leu Gly Ala Leu Pro Val Leu Tyr
               420                 425                 430
   CAC CTG AAC GAC GAG AAT GTG AAC CTG AAA AAG TAT AGA AAC ATG ATT        1344
   His Leu Asn Asp Glu Asn Val Asn Leu Lys Lys Tyr Arg Asn Met Ile
           435                 440                 445
   GTG AAA TCC TGC GGG TGC CAT TGA                                        1368
   Val Lys Ser Cys Gly Cys His
       450                 455
(2) INFORMATION FOR SEQ ID NO:25:
   (i) SEQUENCE CHARACTERISTICS:
      (A) LENGTH: 455 amino acids
      (B) TYPE: amino acid
      (D) TOPOLOGY: linear
   (ii) MOLECULE TYPE: protein
   (xi) SEQUENCE DESCRIPTION: SEQ ID NO:25:
   Met Ser Gly Leu Arg Asn Thr Ser Glu Ala Val Ala Val Leu Ala Ser
    1               5                  10                  15
   Leu Gly Leu Gly Met Val Leu Leu Met Phe Val Ala Thr Thr Pro Pro
               20                  25                  30
   Ala Val Glu Ala Thr Gln Ser Gly Ile Tyr Ile Asp Asn Gly Lys Asp
   35 40 45
   Gln Thr Ile Met His Arg Val Leu Ser Glu Asp Asp Lys Leu Asp Val
   50 55 60
   Ser Tyr Glu Ile Leu Glu Phe Leu Gly Ile Ala Glu Arg Pro Thr His
   65 70 75 80
   Leu Ser Ser His Gln Leu Ser Leu Arg Lys Ser Ala Pro Lys Phe Leu
   85 90 95
   Leu Asp Val Tyr His Arg Ile Thr Ala Glu Glu Gly Leu Ser Asp Gln
   100 105 110
   Asp Glu Asp Asp Asp Tyr Glu Arg Gly His Arg Ser Arg Arg Ser Ala
   115 120 125
   Asp Leu Glu Glu Asp Glu Gly Glu Gln Gln Lys Asn Phe Ile Thr Asp
   130 135 140
   Leu Asp Lys Arg Ala Ile Asp Glu Ser Asp Ile Ile Met Thr Phe Leu
   145 150 155 160
   Asn Lys Arg His His Asn Val Asp Glu Leu Arg His Glu His Gly Arg
   165 170 175
   Arg Leu Trp Phe Asp Val Ser Asn Val Pro Asn Asp Asn Tyr Leu Val
   180 185 190
   Met Ala Glu Leu Arg Ile Tyr Gln Asn Ala Asn Glu Gly Lys Trp Leu
   195 200 205
   Thr Ala Asn Arg Glu Phe Thr Ile Thr Val Tyr Ala Ile Gly Thr Gly
   210 215 220
   Thr Leu Gly Gln His Thr Met Glu Pro Leu Ser Ser Val Asn Thr Thr
   225 230 235 240
   Gly Asp Tyr Val Gly Trp Leu Glu Leu Asn Val Thr Glu Gly Leu His
   245 250 255
   Glu Trp Leu Val Lys Ser Lys Asp Asn His Gly Ile Tyr Ile Gly Ala
   260 265 270
   His Ala Val Asn Arg Pro Asp Arg Glu Val Lys Leu Asp Asp Ile Gly
   275 280 285
   Leu Ile His Arg Lys Val Asp Asp Glu Phe Gln Pro Phe Met Ile Gly
   290 295 300
   Phe Phe Arg Gly Pro Glu Leu Ile Lys Ala Thr Ala His Ser Ser His
   305 310 315 320
   His Arg Ser Lys Arg Ser Ala Ser His Pro Arg Lys Arg Lys Lys Ser
   325 330 335
   Val Ser Pro Asn Asn Val Pro Leu Leu Glu Pro Met Glu Ser Thr Arg
   340 345 350
   Ser Cys Gln Met Gln Thr Leu Tyr Ile Asp Phe Lys Asp Leu Gly Trp
   355 360 365
   His Asp Trp Ile Ile Ala Pro Glu Gly Tyr Gly Ala Phe Tyr Cys Ser
   370 375 380
   Gly Glu Cys Asn Phe Pro Leu Asn Ala His Met Asn Ala Thr Asn His
   385 390 395 400
   Ala Ile Val Gln Thr Leu Val His Leu Leu Glu Pro Lys Lys Val Pro
   405 410 415
   Lys Pro Cys Cys Ala Pro Thr Arg Leu Gly Ala Leu Pro Val Leu Tyr
   420 425 430
   His Leu Asn Asp Glu Asn Val Asn Leu Lys Lys Tyr Arg Asn Met Ile
   435 440 445
   Val Lys Ser Cys Gly Cys His
   450 455
(2) INFORMATION FOR SEQ ID NO:26:
   (i) SEQUENCE CHARACTERISTICS:
      (A) LENGTH: amino acids
      (B) TYPE: amino acid
      (D) TOPOLOGY: linear
   (ii) MOLECULE TYPE: protein
   (iii) ORIGINAL SOURCE:
      (A) ORGANISM: Homo Sapiens
   (ix) FEATURE:
      (A) NAME/KEY: Protein
      (B) LOCATION: 1..102
      (D) OTHER INFORMATION: /note="BHP3"
(2) INFORMATION FOR SEQ ID NO:26:
   (i)SEQUENCE CHARACTERISTICS:
      (A) LENGTH: 104 amino acids
      (B) TYPE: amino acid
      (C) STRANDEDNESS: single
      (D) TOPOLOGY: linear
   (ii)HOLECULE TYPE: protein
   (ix)FEATURE:
      (A) NAME/KEY: Protein
      (B) LOCATION: 1..104
      (D) OTHER INFORMATION: /note="BMP3"
   (xi)SEQUENCE DESCRIPTION: SEQ ID NO:26:
(2) INFORMATION FOR SEQ ID NO:27:
   (i) SEQUENCE CHARACTERISTICS:
      (A) LENGTH: 102 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: HOMO SAPIENS
   (ix) FEATURE:
      (A) NAME/KEY: Protein
      (B) LOCATION: 1..102
      (D) OTHER INFORMATION: /note= "BHP5"
   (xi) SEQUENCE DESCRIPTION: SEQ ID NO:27:
   Cys Lys Lys His Glu Leu Tyr Val Ser Phe Arg Asp Leu Gly Trp Gln
   1               5                   10                  15
   Asp Trp Ile Ile Ala Pro Glu Gly Tyr Ala Ala Phe Tyr Cys Asp Gly
               20                  25                  30
   Glu Cys Ser Phe Pro Leu Asn Ala His Met Asn Ala Thr Asn His Ala
           35                  40                  45
   Ile Val Gln Thr Leu Val His Leu Met Phe Pro Asp His Val Pro Lys
       50                  55                  60
   Pro Cys Cys Ala Pro Thr Lys Leu Asn Ala Ile Ser Val Leu Tyr Phe
   65                  70                  75                  80
   Asp Asp Ser Ser Asn Val Ile Leu Lys Lys Tyr Arg Asn Met Val Val
                   85                  90                  95
   Arg Ser Cys Gly Cys His
               100
(2) INFORMATION FOR SEQ ID NO:28:
   (i) SEQUENCE CHARACTERISTICS:
      (A) LENGTH: 102 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (A) ORGANISM: HOMO SAPIENS
   (ix) FEATURE:
      (A) NAME/KEY: Protein
      (B) LOCATION: 1..102
      (D) OTHER INFORMATION: /note= "BMP6"
   (xi) SEQUENCE DESCRIPTION: SEQ ID NO:28:
   Cys Arg Lys His Glu Leu Tyr Val Ser Phe Gln Asp Leu Gly Trp Gln
   1               5                   10                  15
   Asp Trp Ile Ile Ala Pro Lys Gly Tyr Ala Ala Asn Tyr Cys Asp Gly
               20                  25                  30
   Glu Cys Ser Phe Pro Leu Asn Ala His Met Asn Ala Thr Asn His Ala
           35                  40                  45
   Ile Val Gln Thr Leu Val His Leu Met Asn Pro Glu Tyr Val Pro Lys
       50                  55                  60
   Pro Cys Cys Ala Pro Thr Lys Leu Asn Ala Ile Ser Val Leu Tyr Phe
   65                  70                  75                  80
   Asp Asp Asn Ser Asn Val Ile Leu Lys Lys Tyr Arg Trp Met Val Val
   85 90 95
   Arg Ala Cys Gly Cys His
   100
(2) INFORMATION FOR SEQ ID NO:29:
   (i) SEQUENCE CHARACTERISTICS:
      (A) LENGTH: 102 amino acids
      (B) TYPE: amino acid
      (D) TOPOLOGY: linear
   (ii) MOLECULE TYPE: protein
   (ix) FEATURE:
      (A) NAME/KEY: Protein
      (B) LOCATION: 1..102
      (D) OTHER INFORMATION: /label= OPX /note= "WHEREIN XAA AT EACH POS'N IS INDEPENDENTLY SELECTED FROM THE RESIDUES OCCURRING AT THE CORRESPONDING POS'N IN THE C-TERHINAL SEQUENCE OF MOUSE OR HUMAN OP1 OR OP2 (SEE SEQ. ID NOS. 5,6,7 and 8 or 16,18,20 and 22.)"
   (xi) SEQUENCE DESCRIPTION: SEQ ID NO:29:
   Cys Xaa Xaa His Glu Leu Tyr Val Xaa Phe Xaa Asp Leu Gly Trp Xaa
   1 5 10 15
   Asp Trp Xaa Ile Ala Pro Xaa Gly Tyr Xaa Ala Tyr Tyr Cys Glu Gly
   20 25 30
   Glu Cys Xaa Phe Pro Leu Xaa Ser Xaa Met Asn Ala Thr Asn His Ala
   35 40 45
   Ile Xaa Gln Xaa Leu Val His Xaa Xaa Xaa Pro Xaa Xaa Val Pro Lys
   50 55 60
   Xaa Cys Cys Ala Pro Thr Xaa Leu Xaa Ala Xaa Ser Val Leu Tyr Xaa
   65 70 75 80
   Asp Xaa Ser Xaa Asn Val Xaa Leu Xaa Lys Xaa Arg Asn Met Val Val
   85 90 95
   Xaa Ala Cys Gly Cys His
   100
(2) INFORMATION FOR SEQ ID NO:30:
   (i) SEQUENCE CHARACTERISTICS:
      (A) LENGTH: 97 amino acids
      (B) TYPE: amino acids
      (C) TOPOLOGY: linear
   (ii) HOLECULE TYPE: protein
   (ix) FEATURE:
      (A) NAME: Generic Sequence 5
      (D) OTHER INFORMATION: wherein each Xaa is independently selected from a group of one or more specified amino acids as defined in the specification.
   (xi) SEQUENCE DESCRIPTION: SEQ ID NO:30:
   Leu Xaa Xaa Xaa Phe
   1               5
   Xaa Xaa Xaa Gly Trp Xaa Xaa Trp Xaa
                   10
   Xaa Xaa Pro Xaa Xaa Xaa Xaa Ala
    15             20
   Xaa Tyr Cys Xaa Gly Xaa Cys Xaa
           25              30
   Xaa Pro Xaa Xaa Xaa Xaa Xaa
                   35
   Xaa Xaa Xaa Asn His Ala Xaa Xaa
           40                      45
   Xaa Xaa Xaa Xaa Xaa Xaa Xaa Xaa
                   50
   Xaa Xaa Xaa Xaa Xaa Xaa Xaa Cys
       55                      60
   Cys Xaa Pro Xaa Xaa Xaa Xaa Xaa
               65
   Xaa Xaa Xaa Leu Xaa Xaa Xaa
    70                 75
   Xaa Xaa Xaa Xaa Val Xaa Leu Xaa
                   80
   Xaa Xaa Xaa Xaa Met Xaa Val Xaa
    85                 90
   Xaa Cys Xaa Cys Xaa
           95
(2) INFORMATION FOR SEQ ID NO:31:
   (i) SEQUENCE CHARACTERISTICS:
      (A) LENGTH: 102 amino acids
      (B) TYPE: amino acids
      (C) TOPOLOGY: linear
   (ii) MOLECULE TYPE: protein
   (ix) FEATURE:
      (A) NAME: Generic Sequence 6
      (D) OTHER INFORMATION: wherein each Xaa is independently selected from a group of one or more specified amino acids as defined in the specification.
   (xi) SEQUENCE DESCRIPTION: SEQ ID NO:31:
   Cys Xaa Xaa Xaa Xaa Leu Xaa Xaa Xaa Phe
    1               5                   10
   Xaa Xaa Xaa Gly Trp Xaa Xaa Trp Xaa
                   15
   Xaa Xaa Pro Xaa Xaa Xaa Xaa Ala
    20                  25
   Xaa Tyr Cys Xaa Gly Xaa Cys Xaa
           30                  35
   Xaa Pro Xaa Xaa Xaa Xaa Xaa
                   40
   Xaa Xaa Xaa Asn His Ala Xaa Xaa
           45                  50
   Xaa Xaa Xaa Xaa Xaa Xaa Xaa Xaa
                   55
   Xaa Xaa Xaa Xaa Xaa Xaa Xaa Cys
       60                  65
   Cys Xaa Pro Xaa Xaa Xaa Xaa Xaa
               70
   Xaa Xaa Xaa Leu Xaa Xaa Xaa
    75                  80
   Xaa Xaa Xaa Xaa Val Xaa Leu Xaa
               85
   Xaa Xaa Xaa Xaa Met Xaa Val Xaa
    90                  95
   Xaa Cys Xaa Cys Xaa
           100
(2) INFORMATION FOR SEQ ID NO:32:
   (i) SEQUENCE CHARACTERISTICS:
      (A) LENGTH: 1238 base pairs, 372 amino acids
      (B) TYPE: nucleic acid, amino acid
      (C) STRANDEDNESS: single
      (D) TOPOLOGY: linear
   (ii) MOLECULE TYPE: cDNA
   (iii) ORIGINAL SOURCE:
      (A) ORGANISM: human
      (F) TISSUE TYPE: BRAIN
   (iv) FEATURE:
      (A) NAME/KEY: CDS
      (B) LOCATION:
      (D) OTHER INFORMATION:
         /product= "GDF-1"
         /note= "GDF-1 CDNA"
   (x) PUBLICATION INFORMATION:
      (A) AUTHORS: Lee, Se-Jin
      (B) TITTLE: Expression of Growth/Differentiation Factor 1
      (C) JOURNAL: Proc. Nat'l Acad. Sci.
      (D) VOLUME: 88
      (E) RELEVANT RESIDUES: 1-1238
      (F) PAGES: 4250-4254
      (G) DATE: May-1991
   (xi) SEQUENCE DESCRIPTION: SEQ ID NO:32:
(34) INFORMATION FOR SEQ ID NO:33:
   (i) SEQUENCE CHARACTERISTICS:
      (A) LENGTH: 372 amino acids
      (B) TYPE: amino acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: human
      (F) TISSUE TYPE: BRAIN
   (ix) FEATURE:
      (A) NAME/KEY: CDS
      (B) LOCATION:
      (D) OTHER INFORMATION: /function= /product= "GDF-1"
   (xi) SEQUENCE DESCRIPTION: SEQ ID NO:33:

## Description

This invention relates to means for increasing the bone mass and/or preventing the loss of bone mass in a mammal. In particular it relates to use of a morphogen in the manufacture of a medicament for treatment of metabolic bone disease.

### Background of the Invention

WO 92/21365 discloses a method for generating new bone growth in a mammal comprising administering to the mammal a safe and effective amount of Vitamin D compound in combination with a safe and effective amount of osteoinductive extract or at least one BMP.

EP 0 514 720 discloses bone growth factors used to stimulate bone formation when administered with agents that inhibit bone resorption.

Throughout adult life, bone is continually undergoing remodeling through the interactive cycles of bone formation and resorption (bone turnover). Bone resorption typically is rapid, and is mediated by osteoclasts (bone resorbing cells), formed by mononuclear phagocytic precursor cells at bone remodeling sites. This process then is followed by the appearance of osteoblasts (bone forming cells) which form bone slowly to replace the lost bone. The activities of the various cell types that participate in the remodeling process are controlled by interacting systemic (e.g., hormones, lymphokines, growth factors, vitamins) and local factors (e.g., cytokines, adhesion molecules, lymphokines and growth factors). The fact that completion of this process normally leads to balanced replacement and renewal of bone indicates that the molecular signals and events that influence bone remodeling are tightly controlled.

A number of bone growth disorders are known which cause an imbalance in the bone remodeling cycle. Chief among these are metabolic bone diseases, such as osteoporosis, osteoplasia (osteomalacia), chronic renal failure and hyperparathyroidism, which result in abnormal or excessive loss of bone mass (osteopenia). Other bone diseases, such as Paget's disease, also cause excessive loss of bone mass at localized sites.

Osteoporosis is a structural deterioration of the skeleton caused by loss of bone mass resulting from an imbalance in bone formation, bone resorption, or both, such that the resorption dominates the bone formation phase, thereby reducing the weight-bearing capacity of the affected bone. In a healthy adult, the rate at which bone is formed and resorbed is tightly coordinated so as to maintain the renewal of skeletal bone. However, in osteoporotic individuals an imbalance in these bone remodeling cycles develops which results in both loss of bone mass and in formation of microarchitectural defects in the continuity of the skeleton. These skeletal defects, created by perturbation in the remodeling sequence, accumulate and finally reach a point at which the structural integrity of the skeleton is severely compromised and bone fracture is likely. Although this imbalance occurs gradually in most individuals as they age ("senile osteoporosis"), it is much more severe and occurs at a rapid rate in postmenopausal women. In addition, osteoporosis also may result from nutritional and endocrine imbalances, hereditary disorders and a number of malignant transformations.

Patients suffering from chronic renal (kidney) failure almost universally suffer loss of skeletal bone mass (renal osteodystrophy). While it is known that kidney malfunction causes a calcium and phosphate imbalance in the blood, to date replenishment of calcium and phosphate by dialysis does not significantly inhibit osteodystrophy in patients suffering from chronic renal failure. In adults, osteodystrophic symptoms often are a significant cause of morbidity. In children, renal failure often results in a failure to grow, due to the failure to maintain and/or to increase bone mass.

Osteoplasia, also known as osteomalacia ("soft bones"), is a defect in bone mineralization (e.g., incomplete mineralization), and classically is related to vitamin D deficiency (1,25-dihydroxy vitamin D₃). The defect can cause compression fractures in bone, and a decrease in bone mass, as well as extended zones of hypertrophy and proliferative cartilage in place of bone tissue. The deficiency may result from a nutritional deficiency (e.g., rickets in children), malabsorption of vitamin D or calcium, and/or impaired metabolism of the vitamin.

Hyperparathyroidism (overproduction of the parathyroid hormone) is known to cause malabsorption of calcium, leading to abnormal bone loss. In children, hyperparathyroidism can inhibit growth, in adults the skeleton integrity is compromised and fracture of the ribs and vertebrae are characteristic. The parathyroid hormone imbalance typically may result from thyroid adenomas or gland hyperplasia, or may result from prolonged pharmacological use of a steroid. Secondary hyperparathyroidism also may result from renal osteodystrophy. In the early stages of the disease osteoclasts are stimulated to resorb bone in response to the excess hormone present. As the disease progresses, the trabecular bone ultimately is resorbed and marrow is replaced with fibrosis, macrophages and areas of hemorrhage as a consequence of microfractures. This condition is referred to clinically as osteitis fibrosa.

Paget's disease (osteitis deformans) is a disorder currently thought to have a viral etiology and is characterized by excessive bone resorption at localized sites which flare and heal but which ultimately are chronic and progressive, and may lead to malignant transformation. The disease typically affects adults over the age of 25.

To date, osteopenia treatments are based on inhibiting further bone resorption, e.g., by 1) inhibiting the differentiation of hemopoietic mononuclear cells into mature osteoclasts, 2) by directly preventing osteoclast-mediated bone resorption, or 3) by affecting the hormonal control of bone resorption. Drug regimens used for the treatment of osteoporosis include calcium supplements, estrogen, calcitonin and diphosphonates. Vitamin D₃ and its metabolites, known to enhance calcium and phosphate absorption, also are being tried. None of the current therapies stimulate regeneration of new bone tissue. In addition, all of these agents have only a transient efffect on bone remodeling. Thus, while in some cases the progression of the disease may be halted or slowed, patients with significant bone deterioration remain actively at risk. This is particularly prevalent in disorders such as osteoporosis where early diagnosis is difficult and/or rare and significant structural deterioration of the bone already may have occurred.

It is an object of the present invention to develop medical use of a morphogen for treatment of metabolic bone disease in an individual who, for example, is afflicted with a disease which decreases skeletal bone mass, particularly where the disease causes an imbalance in bone remodeling. Another object is use to enhance bone growth in children suffering from bone disorders, including metabolic bone diseases. Still another object is to prevent or inhibit bone deterioration in individuals at risk for loss of bone mass, including postmenopausal women, aged individuals, and patients undergoing dialysis. Yet another object is to provide methods and compositions for repairing defects in the microstructure of structurally compromised bone, including repairing bone fractures. Thus, the invention is aimed at stimulating bone formation and increasing bone mass, optionally over prolonged periods of time, and particularly to decrease the occurrence of new fractures resulting from structural deterioration of the skeleton. These and other objects and features of the invention will be apparent from the description, drawings, and claims which follow.

### Summary of the Invention

The present invention provides use of a morphogen as defined in the appended claims.

In one aspect, the invention features use of a morphogen in the manufacture of a medicament for treatment of metabolic bone disease in a mammal. The treatment includes administering to the individual a therapeutically effective morphogen in an amount and for a time sufficient to inhibit the loss of bone mass, and/or to increase bone mass in the individual.

A therapeutic treatment method and composition for preventing loss of bone mass and/or for increasing bone mass in a mammal could include administering to the mammal a compound that stimulates in vivo a therapeutically effective concentration of an endogenous morphogen in the body of the mammal sufficient to prevent loss of and/or to increase bone mass in the individual. These compounds are referred to herein as morphogen-stimulating agents, and are understood to include substances which, when administered to a mammal, act on tissue(s) or organ(s) that normally are responsible for, or capable of, producing a morphogen and/or secreting a morphogen, and which cause the endogenous level of the morphogen to be altered. The agent may act, for example, by stimulating expression and/or secretion of an endogenous morphogen.

The morphogens described herein are believed to play a significant role in maintaining appropriate bone mass in an individual. Thus, a morphogen may be administered to any individual who requires assistance in maintaining appropriate bone mass and/or who suffers from a bone remodeling imbalance. For example, the morphogen or morphogen-stimulating agent may be administered to an adult suffering from renal failure to prevent bone deterioration which is associated with that disease, e.g., to correct bone loss due to late stage kidney failure. Similarly, the administration of a morphogen to a child suffering from renal failure is expected not only to alleviate loss of bone mass in the child, but also to stimulate bone formation and thus growth. In addition, administration of a morphogen or morphogen-stimulating agent to an individual suffering from defects in skeletal microstructure is expected to result in repair of that defect, and to enhance the weight-bearing capacity of the treated bone.

Accordingly, treatment methods and compositions may be used to treat a bone fracture or any disease which causes or results in bone fractures or other defects in skeletal microstructure, including loss of bone mass, and which compromise the weight-bearing capacity of bone. Such diseases include, for example, chronic renal failure and other kidney diseases, particularly those requiring dialysis; osteomalacia; vitamin D deficiency-induced osteopenia or osteoporosis; postmenopausal or senile osteoporosis; hyperparathyroidism and Paget's disease.

A morphogen or morphogen-stimulating agent could be administered systemically to the individual, e.g., orally or parenterally. In another embodiment the morphogen may be provided directly to the bone, e.g., by injection to the bone periosteum or endosteum. Direct injection is particularly useful for repairing defects in the microstructure of the bone, including bone fractures.

In any treatment method "administration of morphogen" refers to the administration of the morphogen, either alone or in combination with other molecules. For example, the mature form of the morphogen may be provided in association with its precursor "pro" domain, which is known to enhance the solubility of the protein. Other useful molecules known to enhance protein solubility include casein and other milk components, as well as various serum proteins. Additional useful molecules which may be associated with the morphogen or morphogen-stimulating agent include tissue targeting molecules capable of directing the morphogen or morphogen-stimulating agent to bone. Tissue targeting molecules envisioned to be useful in the treatment protocols include tetracycline, diphosphonates, and antibodies or other binding proteins which interact specifically with surface molecules on bone tissue cells.

Still another useful tissue targeting molecule is the morphogen precursor "pro" domain, particularly that of OP-1, BMP2 or BMP4. These proteins are found naturally associated with bone tissue but likely are synthesized in other tissues and targeted to bone tissue after secretion from the synthesizing tissue. For example, the primary source of OP-1 synthesis appears to be the tissue of the urinary tract (e.g., renal tissue), while the protein has been shown to be active in bone tissue (see below.) Moreover, the protein has been identified in serum, saliva and various milk forms. In addition, the secreted form of the protein comprises the mature dimer in association with the pro domain of the intact morphogen sequence. Accordingly, the associated morphogen pro domains may act to target specific morphogens to different tissues in vivo.

Associated tissue targeting or solubility-enhancing molecules also may be covalently linked to the morphogen using standard chemical means, including acid-labile linkages, which likely will be preferentially cleaved in the acidic environment of bone remodeling sites.

The morphogens or morphogen-stimulating agents also may be administered together with other "co-factors" known to have a beneficial effect on bone remodeling, including parathyroid hormone, vitamin D₃, prostaglandins, dexamethasone, IGF (I, II) and their binding proteins, and other agents known to enhance osteoblast activity. Other useful confactors include calcitonin and estrogen and other agents which inhibit bone resorption.

Among the morphogens useful in this invention are proteins originally identified as osteogenic proteins, such as the OP-1, OP-2 and CHMP2 proteins, as well as amino acid sequence-related proteins such as DPP (from Drosophila), Vgl (from Xenopus), Vgr-1 (from mouse, see U.S. 5,011,691 to Oppermann et al.), GDF-1 (from mouse, see Lee (1991) PNAS 88:4250-4254), all of which are presented in Table II and Seq. ID Nos.5-14), and the recently identified 60A protein (from Drosophila, Seq. ID No. 24, see Wharton et al. (1991) PNAS 88:9214-9218.) The members of this family, which include members of the TGF-β super-family of proteins, share substantial amino acid sequence homology in their C-terminal regions. The proteins are translated as a precursor, having an N-terminal signal peptide sequence, typically less than about 30 residues, followed by a "pro" domain that is cleaved to yield the mature sequence. The signal peptide is cleaved rapidly upon translation, at a cleavage site that can be predicted in a given sequence using the method of Von Heijne ((1986) Nucleic Acids Research 14:4683-4691.) Table I, below, describes the various morphogens identified to date, including their nomenclature as used herein, their Seq. ID references, and publication sources for the amino acid sequences of the full length proteins not included in the Seq. Listing.

**TABLE I**

| | |
|---|---|
| "OP-1" | Refers generically to the group of morphogenically active proteins expressed from part or all of a DNA sequence encoding OP-1 protein, including allelic and species variants thereof, e.g., human OP-1 ("hOP-1", Seq. ID No. 5, mature protein amino acid sequence), or mouse OP-1 ("mOP-1", Seq. ID No. 6, mature protein amino acid sequence.) The conserved seven cysteine skeleton is defined by residues 38 to 139 of Seq. ID Nos. 5 and 6. The cDNA sequences and the amino acids encoding the full length proteins are provided in Seq. Id Nos. 16 and 17 (hOP1) and Seq. ID Nos. 18 and 19 (mOP1.) The mature proteins are defined by residues 293-431 (hOP1) and 292-430 (mOP1). The "pro" regions of the proteins, cleaved to yield the mature, morphogenically active proteins are defined essentially by residues 30-292 (hOP1) and residues 30-291 (mOP1). |
| "OP-2" | refers generically to the group of active proteins expressed from part or all of a DNA sequence encoding OP-2 protein, including allelic and species variants thereof, e.g., human OP-2 ("hOP-2", Seq. ID No. 7, mature protein amino acid sequence) or mouse OP-2 ("mOP-2", Seq. ID No. 8, mature protein amino acid sequence). The conserved seven cysteine skeleton is defined by residues 38 to 139 |
| | of Seq. ID Nos. 7 and 8. The cDNA sequences and the amino acids encoding the full length proteins are provided in Seq. ID Nos. 20 and 21 (hOP2) and Seq. ID Nos. 22 and 23 (mOP2.) The mature proteins are defined essentially by residues 264-402 (hOP2) and 261-399 (mOP2). The "pro" regions of the proteins, cleaved to yield the mature, morphogenically active proteins are defined essentially by residues 18-263 (hOP2) and residues 18-260 (mOP2). (Another cleavage site also occurs 21 residues further upstream for both OP-2 proteins.) |
| "CBMP2" | refers generically to the morphogenically active proteins expressed from a DNA sequence encoding the CBMP2 proteins, including allelic and species variants thereof, e.g., human CBMP2A ("CBMP2A(fx)", Seq ID No. 9) or human CBMP2B DNA ("CBMP2B(fx)", Seq. ID No. 10). The amino acid sequence for the full length proteins, referred to in the literature collectively as BMP2A and BMP2B, or BMP2 and BMP4, appear in Wozney, et al. (1988) Science 242:1528-1534. The pro domain for BMP2 (BMP2A) likely includes residues 25-248 or 25-282; the mature protein, residues 249-396 or 283-396. The pro domain for BMP4 (BMP2B) likely includes residues 25-256 or 25-292; the mature protein, residues 257-408 or 293-408. |
| "DPP(fx)" | refers to protein sequences encoded by the Drosophila DPP gene and defining the conserved seven cysteine skeleton (Seq. ID No. 11). The amino acid sequence for the full length protein appears in Padgett, et al (1987) Nature 325: 81-84. The pro domain likely extends from the signal peptide cleavage site to residue 456; the mature protein likely is defined by residues 457-588. |
| "Vgl(fx)" | refers to protein sequences encoded by the Xenopus Vgl gene and defining the conserved seven cysteine skeleton (Seq. ID No. 12). The amino acid sequence for the full length protein appears in Weeks (1987) Cell 51: 861-867. The pro domain likely extends from the signal peptide cleavage site to residue 246; the mature protein likely is defined by residues 247-360. |
| "Vgr-1(fx)" | refers to protein sequences encoded by the murine Vgr-1 gene and defining the conserved seven cysteine skeleton (Seq. ID No. 13). The amino acid sequence for the full length protein appears in Lyons, et al, (1989) PNAS 86: 4554-4558. The pro domain likely extends from the signal peptide cleavage site to residue 299; the mature protein likely is defined by residues 300-438. |
| "GDF-1(fx)" | refers to protein sequences encoded by the human GDF-1 gene and defining the conserved seven cysteine skeleton (Seq. ID No. 14). The cDNA and encoded amino sequence for the full length protein is provided in Seq. ID. No. 32. The pro domain likely extends from the signal peptide clavage site to residue 214; the mature protein likely is defined by residues 215-372. |
| "60A" | refers generically to the morphogenically active proteins expressed from part or all of a DNA sequence (from the Drosophila 60A gene) encoding the 60A proteins (see Seq. ID No. 24 wherein the cDNA and encoded amino acid sequence for the full length protein is provided). "60A(fx)" refers to the protein sequences defining the conserved seven cysteine skeleton (residues 354 to 455 of Seq. ID No. 24.) The pro domain likely extends from the signal peptide cleavage site to residue 324; the mature protein likely is defined by residues 325-455. |
| "BMP3(fx)" | refers to protein sequences encoded by the human BMP3 gene and defining the conserved seven cysteine skeleton (Seq. ID No. 26). The amino acid sequence for the full length protein appears in wozney et al. (1988) Science 242: 1528-1534. The pro domain likely extends from the signal peptide cleavage site to residue 290; the mature protein likely is defined by residues 291-472. |
| "BMP5(fx)" | refers to protein sequences encoded by the human BMP5 gene and defining the conserved seven cysteine skeleton (Seq. ID No. 27). The amino acid sequence for the full length protein appears in Celeste, et al. (1991) PNAS 87: 9843-9847. The pro domain likely extends from the signal peptide cleavage site to residue 316; the mature protein likely is defined by residues 317-454. |
| "BMP6(fx)" | refers to protein sequences encoded by the human BMP6 gene and defining the conserved seven cysteine skeleton (Seq. ID No. 28). The amino acid sequence for the full length protein appear sin Celeste, et al. (1990) PNAS 87: 9843-5847. The pro domain likely includes extends from the signal peptide cleavage site to residue 374; the mature sequence likely includes residues 375-513. |

The OP-2 proteins have an additional cysteine residue in this region (e.g., see residue 41 of Seq. ID Nos. 7 and 8), in addition to the conserved cysteine skeleton in common with the other proteins in this family. The GDF-1 protein has a four amino acid insert within the conserved skeleton (residues 44-47 of Seq. ID No. 14) but this insert likely does not interfere with the relationship of the cysteines in the folded structure. In addition, the CBMP2 proteins are missing one amino acid residue within the cysteine skeleton.

The morphogens are inactive when reduced, but are active as oxidized homodimers and when oxidized in combination with other morphogens of this invention. Thus, as defined herein, a morphogen is a dimeric protein comprising a pair of polypeptide chains, wherein each polypeptide chain comprises at least the C-terminal six cysteine skeleton defined by residues 43-139 of Seq. ID No. 5, including functionally equivalent arrangements of these cysteines (e.g., amino acid insertions or deletions which alter the linear arrangement of the cysteines in the sequence but not their relationship in the folded structure), such that, when the polypeptide chains are folded, the dimeric protein species comprising the pair of polypeptide chains has the appropriate three-dimensional structure, including the appropriate intra- or inter-chain disulfide bonds such that the protein is capable of acting as a morphogen as defined herein. Specifically, the morphogens generally are capable of all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells, including the "redifferentiation" of transformed cells. In addition, it also is anticipated that these morphogens are capable of inducing redifferentiation of committed cells under appropriate environmental conditions.

The morphogens used in this invention comprise one of two species of generic amino acid sequences: Generic Sequence 1 (Seq. ID No. 1) or Generic Sequence 2 (Seq. ID No. 2); where each Xaa indicates one of the 20 naturally-occurring L-isomer, α-amino acids or a derivative thereof. Generic Sequence 1 comprises the conserved six cysteine skeleton and Generic Sequence 2 comprises the conserved six cysteine skeleton plus the additional cysteine identified in OP-2 (see residue 36, Seq. ID No. 2). In another preferred aspect, these sequences further comprise the following additional sequence at their N-terminus:

Preferred amino acid sequences within the foregoing generic sequences include: Generic Sequence 3 (Seq. ID No. 3), Generic Sequence 4 (Seq. ID No. 4), Generic Sequence 5 (Seq. ID No. 30) and Generic Sequence 6 (Seq. ID No. 31), listed below. These Generic Sequences accommodate the homologies shared among the various preferred members of this morphogen family identified in Table II, as well as the amino acid sequence variation among them. Specifically, Generic Sequences 3 and 4 are composite amino acid sequences of the following proteins presented in Table II and identified in Seq. ID Nos. 5-14: human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-22), CBMP2A (Seq. ID No. 9), CBMP2B (Seq. ID No. 10), DPP (from Drosophila, Seq. ID No. 11), Vgl, (from Xenopus, Seq. ID No. 12), Vgr-1 (from mouse, Seq. ID No. 13), and GDF-1 (from mouse, Seq. ID No. 14.) The generic sequences include both the amino acid identity shared by the sequences in Table II, as well as alternative residues for the variable positions within the sequence. Note that these generic sequences allow for an additional cysteine at position 41 or 46 in Generic Sequences 3 or 4, respectively, providing an appropriate cysteine skeleton where inter- or intramolecular disulfide bonds can form, and contain certain critical amino acids which influence the tertiary structure of the proteins.

### Generic Sequence 3

wherein each Xaa is independently selected from a group of one or more specified amino acids defined as follows: "Res." means "residue" and Xaa at res.4 = (Ser, Asp or Glu); Xaa at res.6 = (Arg, Gln, Ser or Lys); Xaa at res.7 = (Asp or Glu); Xaa at res.8 = (Leu or Val); Xaa at res.11 = (Gln, Leu, Asp, His or Asn); Xaa at res.12 = (Asp, Arg or Asn); Xaa at res.14 = (Ile or Val); Xaa at res.15 = (Ile or Val); Xaa at res.18 = (Glu, Gln, Leu, Lys, Pro or Arg); Xaa at res.20 = (Tyr or Phe); Xaa at res.21 = (Ala, Ser, Asp, Met, His, Leu or Gln); Xaa at res.23 = (Tyr, Asn or Phe); Xaa at res.26 = (Glu, His, Tyr, Asp or Gln); Xaa at res.28 = (Glu, Lys, Asp or Gln); Xaa at res.30 = (Ala, Ser, Pro or Gln); Xaa at res.31 = (Phe, Leu or Tyr); Xaa at res.33 = (Leu or Val); Xaa at res.34 = (Asn, Asp, Ala or Thr); Xaa at res.35 = (Ser, Asp, Glu, Leu or Ala); Xaa at res.36 = (Tyr, Cys, His, Ser or Ile); Xaa at res.37 = (Met, Phe, Gly or Leu); Xaa at res.38 = (Asn or Ser); Xaa at res.39 = (Ala, Ser or Gly); Xaa at res.40 = (Thr, Leu or Ser); Xaa at res.44 = (Ile or Val); Xaa at res.45 = (Val or Leu); Xaa at res.46 = (Gln or Arg); Xaa at res.47 = (Thr, Ala or Ser); Xaa at res.49 = (Val or Met); Xaa at res.50 = (His or Asn); Xaa at res.51 = (Phe, Leu, Asn, Ser, Ala or Val); Xaa at res.52 = (Ile, Met, Asn, Ala or Val); Xaa at res.53 = (Asn, Lys, Ala or Glu); Xaa at res.54 = (Pro or Ser); Xaa at res.55 = (Glu, Asp, Asn, or Gly); Xaa at res.56 = (Thr, Ala, Val, Lys, Asp, Tyr, Ser or Ala); Xaa at res.57 = (Val, Ala or Ile); Xaa at res.58 = (Pro or Asp); Xaa at res.59 = (Lys or Leu); Xaa at res.60 = (Pro or Ala); Xaa at res.63 = (Ala or Val); Xaa at res.65 = (Thr or Ala); Xaa at res.66 = (Gln, Lys, Arg or Glu); Xaa at res.67 = (Leu, Met or Val); Xaa at res.68 = (Asn, Ser or Asp); Xaa at res.69 = (Ala, Pro or Ser); Xaa at res.70 = (Ile, Thr or Val); Xaa at res.71 = (Ser or Ala); Xaa at res.72 = (Val or Met); Xaa at res.74 = (Tyr or Phe); Xaa at res.75 = (Phe, Tyr or Leu); Xaa at res.76 = (Asp or Asn); Xaa at res.77 = (Asp, Glu, Asn or Ser); Xaa at res.78 = (Ser, Gln, Asn or Tyr); Xaa at res.79 = (Ser, Asn, Asp or Glu); Xaa at res.80 = (Asn, Thr or Lys); Xaa at res.82 = (Ile or Val); Xaa at res.84 = (Lys or Arg); Xaa at res.85 = (Lys, Asn, Gln or His); Xaa at res.86 = (Tyr or His); Xaa at res.87 = (Arg, Gln or Glu); Xaa at res.88 = (Asn, Glu or Asp); Xaa at res.90 = (Val, Thr or Ala); Xaa at res.92 = (Arg, Lys, Val, Asp or Glu); Xaa at res.93 = (Ala, Gly or Glu); and Xaa at res.97 = (His or Arg);

### Generic Sequence 4

wherein each Xaa is independently selected from a group of one or more specified amino acids as defined by the following: "Res." means "residue" and Xaa at res.2 = (Lys or Arg); Xaa at res.3 = (Lys or Arg); Xaa at res.4 = (His or Arg); Xaa at res.5 = (Glu, Ser, His, Gly, Arg or Pro); Xaa at res.9 = (Ser, Asp or Glu); Xaa at res.11 = (Arg, Gln, Ser or Lys); Xaa at res.12 = (Asp or Glu); Xaa at res.13 = (Leu or Val); Xaa at res.16 = (Gln, Leu, Asp, His or Asn); Xaa at res.17 = (Asp, Arg, or Asn); Xaa at res.19 = (Ile or Val); Xaa at res.20 = (Ile or Val); Xaa at res.23 = (Glu, Gln, Leu, Lys, Pro or Arg); Xaa at res.25 = (Tyr or Phe); Xaa at res.26 = (Ala, Ser, Asp, Met, His, Leu, or Gln); Xaa at res.28 = (Tyr, Asn or Phe); Xaa at res.31 = (Glu, His, Tyr, Asp or Gln); Xaa at res.33 = Glu, Lys, Asp or Gln); Xaa at res.35 = (Ala, Ser or Pro); Xaa at res.36 = (Phe, Leu or Tyr); Xaa at res.38 = (Leu or Val); Xaa at res.39 = (Asn, Asp, Ala or Thr); Xaa at res.40 = (Ser, Asp, Glu, Leu or Ala); Xaa at res.41 = (Tyr, Cys, His, Ser or Ile); Xaa at res.42 = (Met, Phe, Gly or Leu); Xaa at res.44 = (Ala, Ser or Gly); Xaa at res.45 = (Thr, Leu or Ser); Xaa at res.49 = (Ile or Val); Xaa at res.50 = (Val or Leu); Xaa at res.51 = (Gln or Arg); Xaa at res.52 = (Thr, Ala or Ser); Xaa at res.54 = (Val or Met); Xaa at res.55 = (His or Asn); Xaa at res.56 = (Phe, Leu, Asn, Ser, Ala or Val); Xaa at res.57 = (Ile, Met, Asn, Ala or Val); Xaa at res.58 = (Asn, Lys, Ala or Glu); Xaa at res.59 = (Pro or Ser); Xaa at res.60 = (Glu, Asp, or Gly); Xaa at res.61 = (Thr, Ala, Val, Lys, Asp, Tyr, Ser or Ala); Xaa at res.62 = (Val, Ala or Ile); Xaa at res.63 = (Pro or Asp); Xaa at res.64 = (Lys or Leu); Xaa at res.65 = (Pro or Ala); Xaa at res.68 = (Ala or Val); Xaa at res.70 = (Thr or Ala); Xaa at res.71 = (Gln, Lys, Arg or Glu); Xaa at res.72 = (Leu, Met or Val); Xaa at res.73 = (Asn, Ser or Asp); Xaa at res.74 = (Ala, Pro or Ser); Xaa at res.75 = (Ile, Thr or Val); Xaa at res.76 = (Ser or Ala); Xaa at res.77 = (Val or Met); Xaa at res.79 = (Tyr or Phe); Xaa at res.80 = (Phe, Tyr or Leu); Xaa at res.81 = (Asp or Asn); Xaa at res.82 = (Asp, Glu, Asn or Ser); Xaa at res.83 = (Ser, Gln, Asn or Tyr); Xaa at res.84 = (Ser, Asn, Asp or Glu); Xaa at res.85 = (Asn, Thr or Lys); Xaa at res.87 = (Ile or Val); Xaa at res.89 = (Lys or Arg); Xaa at res.90 = (Lys, Asn, Gln or His); Xaa at res.91 = (Tyr or His); Xaa at res.92 = (Arg, Gln or Glu); Xaa at res.93 = (Asn, Glu or Asp); Xaa at res.95 = (Val, Thr or Ala); Xaa at res.97 = (Arg, Lys, Val, Asp or Glu); Xaa at res.98 = (Ala, Gly or Glu); and Xaa at res.102 = (His or Arg).

Similarly, Generic Sequence 5 (Seq. ID No. 30) and Generic Sequence 6 (Seq. ID No. 31) accommodate the homologies shared among all the morphogen protein family members identified in Table II. Specifically, Generic Sequences 5 and 6 are composite amino acid sequences of human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-22), CBMP2A (Seq. ID No. 9), CBMP2B (Seq. ID No. 10), DPP (from Drosophila, Seq. ID No. 11), Vgl, (from Xenopus, Seq. ID No. 12), Vgr-1 (from mouse, Seq. ID No. 13), and GDF-1 (from mouse, Seq. ID No. 14 and 32), human BMP3 (Seq. ID No. 26), human BMP5 (Seq. ID No. 27), human BMP6 (Seq. ID No. 28) and 60A (from Drosophila, Seq. ID No. 24). The generic sequences include both the amino acid identity shared by these sequences in the C-terminal domain, defined by the six and seven cysteine skeletons (Generic Sequences 5 and 6, respectively), as well as alternative residues for the variable positions within the sequence. As for Generic Sequences 3 and 4, Generic Sequences 5 and 6 allow for an additional cysteine at position 41 (Generic Sequence 5) or position 46 (Generic Sequence 6), providing an appropriate cysteine skeleton where inter- or intramolecular disulfide bonds can form, and containing certain critical amino acids which influence the tertiary structure of the proteins.

### Generic Sequence 5

wherein each Xaa is independently selected from a group of one or more specified amino acids defined as follows: "Res." means "residue" and Xaa at res.2 = (Tyr or Lys); Xaa at res.3 = Val or Ile); Xaa at res.4 = (Ser, Asp or Glu); Xaa at res.6 = (Arg, Gln, Ser, Lys or Ala); Xaa at res.7 = (Asp, Glu or Lys); Xaa at res.8 = (Leu, Val or Ile); Xaa at res.11 = (Gln, Leu, Asp, His, Asn or Ser); Xaa at res.12 = (Asp, Arg, Asn or Glu); Xaa at res.14 = (Ile or Val); Xaa at res.15 = (Ile or Val); Xaa at res.16 (Ala or Ser); Xaa at res.18 = (Glu, Gln, Leu, Lys, Pro or Arg); Xaa at res.19 = (Gly or Ser); Xaa at res.20 = (Tyr or Phe); Xaa at res.21 = (Ala, Ser, Asp, Met, His, Gln, Leu or Gly); Xaa at res.23 = (Tyr, Asn or Phe); Xaa at res.26 = (Glu, His, Tyr, Asp, Gln or Ser); Xaa at res.28 = (Glu, Lys, Asp, Gln or Ala); Xaa at res.30 = (Ala, Ser, Pro, Gln or Asn); Xaa at res.31 = (Phe, Leu or Tyr); Xaa at res.33 = (Leu, Val or Met); Xaa at res.34 = (Asn, Asp, Ala, Thr or Pro); Xaa at res.35 = (Ser, Asp, Glu, Leu, Ala or Lys); Xaa at res.36 = (Tyr, Cys, His, Ser or Ile); Xaa at res.37 = (Met, Phe, Gly or Leu); Xaa at res.38 = (Asn, Ser or Lys); Xaa at res.39 = (Ala, Ser, Gly or Pro); Xaa at res.40 = (Thr, Leu or Ser); Xaa at res.44 = (Ile, Val or Thr); Xaa at res.45 = (Val, Leu or Ile); Xaa at res.46 = (Gln or Arg); Xaa at res.47 = (Thr, Ala or Ser); Xaa at res.48 = (Leu or Ile); Xaa at res.49 = (Val or Met); Xaa at res.50 = (His, Asn or Arg); Xaa at res.51 = (Phe, Leu, Asn, Ser, Ala or Val); Xaa at res.52 = (Ile, Met, Asn, Ala, Val or Leu); Xaa at res.53 = (Asn, Lys, Ala, Glu, Gly or Phe); Xaa at res.54 = (Pro, Ser or Val); Xaa at res.55 = (Glu, Asp, Asn, Gly, Val or Lys); Xaa at res.56 = (Thr, Ala, Val, Lys, Asp, Tyr, Ser, Ala, Pro or His); Xaa at res.57 = (Val, Ala or Ile); Xaa at res.58 = (Pro or Asp); Xaa at res.59 = (Lys, Leu or Glu); Xaa at res.60 = (Pro or Ala); Xaa at res.63 = (Ala or Val); Xaa at res.65 = (Thr, Ala or Glu); Xaa at res.66 = (Gln, Lys, Arg or Glu); Xaa at res.67 = (Leu, Met or Val); Xaa at res.68 = (Asn, Ser, Asp or Gly); Xaa at res.69 = (Ala, Pro or Ser); Xaa at res.70 = (Ile, Thr, Val or Leu); Xaa at res.71 = (Ser, Ala or Pro); Xaa at res.72 = (Val, Met or Ile); Xaa at res.74 = (Tyr or Phe); Xaa at res.75 = (Phe, Tyr, Leu or His); Xaa at res.76 = (Asp, Asn or Leu); Xaa at res.77 = (Asp, Glu, Asn or Ser); Xaa at res.78 = (Ser, Gln, Asn, Tyr or Asp); Xaa at res.79 = (Ser, Asn, Asp, Glu or Lys); Xaa at res.80 = (Asn, Thr or Lys); Xaa at res.82 = (Ile, Val or Asn); Xaa at res.84 = (Lys or Arg); Xaa at res.85 = (Lys, Asn, Gln, His or Val); Xaa at res.86 = (Tyr or His); Xaa at res.87 = (Arg, Gln, Glu or Pro); Xaa at res.88 = (Asn, Glu or Asp); Xaa at res.90 = (Val, Thr, Ala or Ile); Xaa at res.92 = (Arg, Lys, Val, Asp or Glu); Xaa at res.93 = (Ala, Gly, Glu or Ser); Xaa at res.95 = (Gly or Ala) and Xaa at res.97 = (His or Arg).

### Generic Sequence 6

wherein each Xaa is independently selected from a group of one or more specified amino acids as defined by the following: "Res." means "residue" and Xaa at res.2 = (Lys, Arg, Ala or Gln); Xaa at res.3 = (Lys, Arg or Met); Xaa at res.4 = (His, Arg or Gln); Xaa at res.5 = (Glu, Ser, His, Gly, Arg, Pro, Thr, or Tyr); Xaa at res.7 = (Tyr or Lys); Xaa at res.8 = (Val or Ile); Xaa at res.9 = (Ser, Asp or Glu); Xaa at res.11 = (Arg, Gln, Ser, Lys or Ala); Xaa at res.12 = (Asp, Glu, or Lys); Xaa at res.13 = (Leu, Val or Ile); Xaa at res.16 = (Gln, Leu, Asp, His, Asn or Ser); Xaa at res.17 = (Asp, Arg, Asn or Glu); Xaa at res.19 = (Ile or Val); Xaa at res.20 = (Ile or Val); Xaa at res.21 = (Ala or Ser); Xaa at res.23 = (Glu, Gln, Leu, Lys, Pro or Arg); Xaa at res.24 = (Gly or Ser); Xaa at res.25 = (Tyr or Phe); Xaa at res.26 = (Ala, Ser, Asp, Met, His, Gln, Leu, or Gly); Xaa at res.28 = (Tyr, Asn or Phe); Xaa at res.31 = (Glu, His, Tyr, Asp, Gln or Ser); Xaa at res.33 = Glu, Lys, Asp, Gln or Ala); Xaa at res.35 = (Ala, Ser, Pro, Gln or Asn); Xaa at res.36 = (Phe, Leu or Tyr); Xaa at res.38 = (Leu, Val or Met); Xaa at res.39 = (Asn, Asp, Ala, Thr or Pro); Xaa at res.40 = (Ser, Asp, Glu, Leu, Ala or Lys); Xaa at res.41 = (Tyr, Cys, His, Ser or Ile); Xaa at res.42 = (Met, Phe, Gly or Leu); Xaa at res.43 = (Asn, Ser or Lys); Xaa at res.44 = (Ala, Ser, Gly or Pro); Xaa at res.45 = (Thr, Leu or Ser); Xaa at res.49 = (Ile, Val or Thr); Xaa at res.50 = (Val, Leu or Ile); Xaa at res.51 = (Gln or Arg); Xaa at res.52 = (Thr, Ala or Ser); Xaa at res.53 = (Leu or Ile); Xaa at res.54 = (Val or Met); Xaa at res.55 = (His, Asn or Arg); Xaa at res.56 = (Phe, Leu, Asn, Ser, Ala or Val); Xaa at res.57 = (Ile, Met, Asn, Ala, Val or Leu); Xaa at res.58 = (Asn, Lys, Ala, Glu, Gly or Phe); Xaa at res.59 = (Pro, Ser or Val); Xaa at res.60 = (Glu, Asp, Gly, Val or Lys); Xaa at res.61 = (Thr, Ala, Val, Lys, Asp, Tyr, Ser, Ala, Pro or His); Xaa at res.62 = (Val, Ala or Ile); Xaa at res.63 = (Pro or Asp); Xaa at res.64 = (Lys, Leu or Glu); Xaa at res.65 = (Pro or Ala); Xaa at res.68 = (Ala or Val); Xaa at res.70 = (Thr, Ala or Glu); Xaa at res.71 = (Gln, Lys, Arg or Glu); Xaa at res.72 = (Leu, Met or Val); Xaa at res.73 = (Asn, Ser, Asp or Gly); Xaa at res.74 = (Ala, Pro or Ser); Xaa at res.75 = (Ile, Thr, Val or Leu); Xaa at res.76 = (Ser, Ala or Pro); Xaa at res.77 = (Val, Met or Ile); Xaa at res.79 = (Tyr or Phe); Xaa at res.80 = (Phe, Tyr, Leu or His); Xaa at res.81 = (Asp, Asn or Leu); Xaa at res.82 = (Asp, Glu, Asn or Ser); Xaa at res.83 = (Ser, Gln, Asn, Tyr or Asp); Xaa at res.84 = (Ser, Asn, Asp, Glu or Lys); Xaa at res.85 = (Asn, Thr or Lys); Xaa at res.87 = (Ile, Val or Asn); Xaa at res.89 = (Lys or Arg); Xaa at res.90 = (Lys, Asn, Gln, His or Val); Xaa at res.91 = (Tyr or His); Xaa at res.92 = (Arg, Gln, Glu or Pro); Xaa at res.93 = (Asn, Glu or Asp); Xaa at res.95 = (Val, Thr, Ala or Ile); Xaa at res.97 = (Arg, Lys, Val, Asp or Glu); Xaa at res.98 = (Ala, Gly, Glu or Ser); Xaa at res.100 = (Gly or Ala); and Xaa at res.102 = (His or Arg).

Particularly useful sequences for use as morphogens include the C-terminal domains, e.g., the C-terminal 96-102 amino acid residues of Vgl, . Vgr-1, DPP, OP-1, OP-2, CBMP-2A, CBMP-2B, GDF-1 (see Table II, below, and Seq. ID Nos. 5-14), as well as proteins comprising the C-terminal domains of 60A, BMP3, BMP5 and BMP6 (see Table II, below, and Seq. ID Nos. 24-28), all of which include at least the conserved six or seven cysteine skeleton. In addition, biosynthetic constructs designed from the generic sequences, such as COP-1, 3-5, 7, 16, disclosed in U.S. Pat. No. 5,011,691, also are useful. Other sequences include the inhibins/activin proteins (see, for example, U.S. Pat. Nos. 4,968,590 and 5,011,691). Accordingly, other useful sequences are those sharing at least 70% amino acid sequence homology or "similarity", and preferably 80% homology or similarity with any of the sequences above. These are anticipated to include allelic and species variants and mutants, and biosynthetic muteins, as well as novel members of this morphogenic family of proteins. Particularly envisioned in the family of related proteins are those proteins exhibiting morphogenic activity and wherein the amino acid changes from the preferred sequences include conservative changes, e.g., those as defined by Dayoff et al., Atlas of Protein Sequence and Structure; vol. 5, Suppl. 3, pp. 345-362, (M.O. Dayoff, ed., Nat'l BioMed. Research Fdn., Washington, D.C. 1979). As used herein, potentially useful sequences are aligned with a known morphogen sequence using the method of Needleman et al. ((1970) J.Mol.Biol. 48:443-453) and identities calculated by the Align program (DNAstar, Inc.). "Homology" or "similarity" as used herein includes allowed conservative changes as defined by Dayoff et al.

The currently most preferred protein sequences useful as morphogens include those having greater than 60% identity, preferably greater than 65% identity, with the amino acid sequence defining the conserved six cysteine skeleton of hOP1 (e.g., residues 43-139 of Seq. ID No.5). These most preferred sequences include both allelic and species variants of the OP-1 and OP-2 proteins, including the Drosophila 60A protein. Accordingly, useful morphogens include active proteins comprising species of polypeptide chains having the generic amino acid sequence herein referred to as "OPX", which accommodates the homologies between the various identified species of OP1 and OP2 (Seq. ID No. 29).

The morphogens useful in the use of this invention include proteins whether isolated from naturally-occurring sources, or produced by recombinant DNA or other synthetic techniques, and includes allelic and species variants of these proteins, naturally-occurring or biosynthetic mutants thereof, as well as various truncated and fusion constructs. Deletion or addition mutants also are envisioned to be active, including those which may alter the conserved C-terminal cysteine skeleton, provided that the alteration does not functionally disrupt the relationship of these cysteines in the folded structure. Accordingly, such active forms are considered the equivalent of the specifically described constructs disclosed herein. The proteins may include forms having varying glycosylation patterns, varying N-termini, a family of related proteins having regions of amino acid sequence homology, and active truncated or mutated forms of native or biosynthetic proteins, produced by expression of recombinant DNA in host cells.

The morphogenic proteins can be expressed from intact or truncated cDNA or from synthetic DNAs in procaryotic or eucaryotic host cells, and purified, cleaved, refolded, and dimerized to form morphogenically active compositions. Currently preferred host cells include E. coli or mammalian cells, such as CHO, COS or BSC cells.

Thus, in view of this disclosure, skilled genetic engineers can isolate genes from cDNA or genomic libraries of various different species which encode appropriate amino acid sequences, or construct DNAs from oligonucleotides, and then can express them in various types of host cells, including both procaryotes and eucaryotes, to produce large quantities of active proteins capable of enhancing bone formation and/or inhibiting abnormal bone deterioration in a variety of mammals, including humans, for use in maintaining appropriate bone mass and bone remodeling in developing and adult bone tissue.

### Brief Description of the Drawings

The foregoing and other objects and features of this invention, as well as the invention itself, may be more fully understood from the following description, when read together with the accompanying drawings, in which:
**FIG. 1** compares the mitogenic effect of hOP-1 and TGF-β on rat osteoblasts;
**FIG. 2** illustrates the effect of human osteogenic protein-1 (hOP-1) on the collagen synthesis of osteoblasts;
**FIG. 3** compares the alkaline phosphatase induction effect of hOP-1 and TGF-β on rat osteoblasts;
**FIG. 4** shows the long-term effect of hOP-1 on the production of alkaline posphatase by rat osteoblasts;
**FIG. 5** shows the effect of hOP-1 on parathyroid hormone-mediated cAMP production using rat osteoblasts in culture;
**FIG. 6A and B** graphs the effect of morphogen on osteoclacin synthesis (A), and the effect of morphogen on the rate of mineralization (B);
**FIG. 7** shows Western Blot analysis of bovine colostrum using OP-1 and BMP2-specific antibodies;
**FIG. 8A and B** show results of in vivo and in vitro activity assays, respectively, for mammary extract purified OP-1;
**FIG. 9** is a photomicrograph of an immunoblot showing the presence of hOP-1 in serum; and
**FIG. 10 (A and B)** are photomicrographs showing new endosteum bone formation following morphogen injection onto the endosteal surface (A), and new periosteum bone formation following morphogen injection onto the periosteal surface (B);
**FIG. 11** is a graphic representation of the dose-dependent effect of morphogen on bone resorption; and
**FIG. 12 (A and B)** are schematic representations of morphogen inhibition of early mononuclear phagocytic cell multinuclearization in vivo;

### Detailed Description of the Invention

It now has been discovered that the proteins described herein are effective agents for preventing loss of bone mass and/or for stimulating bone formation when provided systemically or injected directed into bone tissue in a mammal. As described herein, these proteins ("morphogens") may be used in the treatment of metabolic bone diseases and other disorders that cause an imbalance of the bone remodeling cycle, and/or which cause deterioration of the skeletal microstructure.

The invention is based on the discovery of a family of morphogenic proteins capable of inducing tissue morphogenesis in a mammal. More particularly, the invention is based on the discovery that these proteins play an important role, not only in embryogenesis, but also in the growth, maintenance and repair of bone tissue in juvenile and adult mammals.

It has been shown that implantation of a morphogen (including OP-1, CBMP2, DPP and 60A protein, and various biosynthetic constructs, such as COPS and COP7) together with a suitable matrix in subcutaneous sites in mammals induces a sequence of cellular events which leads to the formation of fully functional new bone, as determined by the specific activity of alkaline phosphatase, calcium content and histology of day 12 implants (see, for example, U.S. Pat. Nos. 4,968,590 and 5,011,691).

The morphogen-containing implants recruit nearby mesenchymal stem cells and trigger their differentiation into chondrocytes within 5-7 days. Upon capillary invasion, the chondrocytes hypertrophy, become calcified and subsequently are replaced by newly formed bone within 9-12 days. The mineralized bone then is remodeled extensively and becomes occupied by ossicles filled with functional bone marrow elements by 14-21 days.

As described herein, the morphogens provided herein stimulate the proliferation, growth and differentiation of osteoblasts in vitro (see Examples 2-7, below), and can induce bone formation in osteoporotic bone tissue in vivo when provided systemically to a mammal, or directly to bone tissue, without an associated matrix carrier (see Examples 8, 9, below.) In addition, the morphogens inhibit multinucleation of activated early mononuclear phagocytic cells (see Example 12, below). Moreover, inhibition of endogenous morphogen activity can inhibit normal skeleton development in a mammal (see Example 13, below.)

As described in Example 1 the naturally-occurring morphogens are widely distributed in the different tissues of the body. For example, as determined by northern blot hybridization, OP-1 is expressed primarily in the tissue of the urogental tract (e.g., renal and bladder tissues). By contrast, Vgr-1, BMP3, BMP4 and BMP5 appear to be expressed primarily in the heart and lung. BMP5 also appears to be expressed significantly in liver tissue. GDF-1 appears to be expressed primarily in brain tissue. (See, for example, Ozkaynak et al. (1992) JBC, in publication.) Moreover, the tissue of synthesis may differ from the natural site of action of specific morphogens. For example, although OP-1 appears to be primarily synthesized in renal tissue, the protein is active in bone tissue. In addition, at least one morphogen, OP-1, is present in a number of body fluids, including saliva, milk (including mammary gland extract, colostrum and 57-day milk) and serum (see Example 11, below.) Accordingly, without being limited to a given theory, the morphogens described herein may behave as endocrine factors, e.g., proteins secreted from a factor-producing tissue in response to particular stimuli, and capable of being transported to, and acting on, a distant tissue. These findings further distinguish morphogens from other members of the TGF-β superfamily of proteins, including TGF-β, which act as local or autocrine factors produced by the tissue on which they act.

The pro domain may function to enhance protein solubility and/or to assist in tissue targeting of morphogens to particular tissues. For example, the mature, active form of OP-1 appears to be secreted from cells in association with the pro domain of the intact sequence. Accordingly, while, as explained herein, the morphogens useful in this invention have significant amino acid sequence homologies within the active domains and are similar in their ability to induce tissue morphogenesis, without being limited to any theory, it is hypothesized that the sequence variation within the morphogenic protein family members may reflect the different specific roles each morphogen plays in specific tissues under natural occurring conditions. For example, the significant sequence variation within the pro domains may mean that these regions of the protein sequence are important for targeting specific morphogens to different tissues for morphogenic activity therein.

Accordingly, the present disclosure comprises two fundamental aspects. In one aspect, the methods and compositions comprise a morphogen which, when administered to an individual, is capable of inhibiting loss of bone mass and/or stimulating bone formation in the individual. In another aspect, the methods and compositions comprise a morphogen-stimulating agent which, when administered to an individual, is capable of inducing the expression and/or secretion of sufficient endogenous morphogen within the individual to provide therapeutically effective concentrations capable of inhibiting loss of bone mass and/or stimulating bone formation in the individual.

Example 14 describes an assay for screening compounds to identify candidate morphogen-stimulating agents. Candidate agents then may be tested for their efficacy in vivo using, for example, the osteoporosis model described in Examples 8 and 9 below.

Provided below are detailed descriptions of suitable morphogens useful in the methods and compositions of this invention, as well as methods for the administration and application of these morphogens and/or of morphogen-stimulating agents. Also provided are numerous, nonlimiting examples which 1) illustrate the suitability of the morphogens and morphogen-stimulating agents described herein as therapeutic agents for inhibiting abnormal bone loss and/or for enhancing bone formation in a human, and 2) provide assays with which to test candidate morphogens and morphogen-stimulating agents for their efficacy.

### I. Useful Morphogens

As defined herein a protein is morphogenic if it is capable of inducing the developmental cascade of cellular and molecular events that culminate in the formation of new, organ-specific tissue and comprises at least the conserved C-terminal six cysteine skeleton or its functional equivalent (see supra). Specifically, the morphogens generally are capable of all of the following biological functions in a morphogenically permissive environment: stimulating proliferation of progenitor cells; stimulating the differentiation of progenitor cells; stimulating the proliferation of differentiated cells; and supporting the growth and maintenance of differentiated cells, including the "redifferentiation" of transformed cells.

As disclosed therein, the morphogens may be purified from naturally-sourced material or recombinantly produced from procaryotic or eucaryotic host cells, using the genetic sequences disclosed therein. Alternatively, novel morphogenic sequences may be identified following the procedures disclosed therein.

Particularly useful proteins include those which comprise the naturally derived sequences disclosed in Table II. Other useful sequences include biosynthetic constructs such as those disclosed in U.S. Pat. 5,011,691 (e.g. COP-1, COP-3, COP-4, COP-5, COP-7, and COP-16).

The morphogens useful in this invention also can be described by any of the 6 generic sequences described herein (Generic Sequences 1, 2, 3, 4, 5 and 6). Generic sequences 1 and 2 also may include, at their N-terminus, the sequence

Table II, set forth below, compares the amino acid sequences of the active regions of native proteins that have been identified as morphogens, including human OP-1 (hOP-1, Seq. ID Nos. 5 and 16-17), mouse OP-1 (mOP-1, Seq. ID Nos. 6 and 18-19), human and mouse OP-2 (Seq. ID Nos. 7, 8, and 20-23), CBMP2A (Seq. ID No. 9), CBMP2B (Seq. ID No. 10), BMP3 (Seq. ID No. 26), DPP (from Drosophila, Seq. ID No. 11), Vgl, (from Xenopus, Seq. ID No. 12), Vgr-1 (from mouse, Seq. ID No. 13), GDF-1 (from mouse, Seq. ID No. 14, 32 and 33), 60A protein (from Drosophila, Seq. ID Nos. 24 and 25), BMP5 (Seq. ID No. 27) and BMP6 (Seq. ID No. 28). The sequences are aligned essentially following the method of Needleman et al. (1970) J. Mol. Biol., 48:443-453, calculated using the Align Program (DNAstar, Inc.) In the table, three dots indicates that the amino acid in that position is the same as the amino acid in hOP-1. Three dashes indicates that no amino acid is present in that position, and are included for purposes of illustrating homologies. For example, amino acid residue 60 of CBMP-2A and CBMP-2B is "missing". Of course, both these amino acid sequences in this region comprise Asn-Ser (residues 58, 59), with CBMP-2A then comprising Lys and Ile, whereas CBMP-2B comprises Ser and Ile.

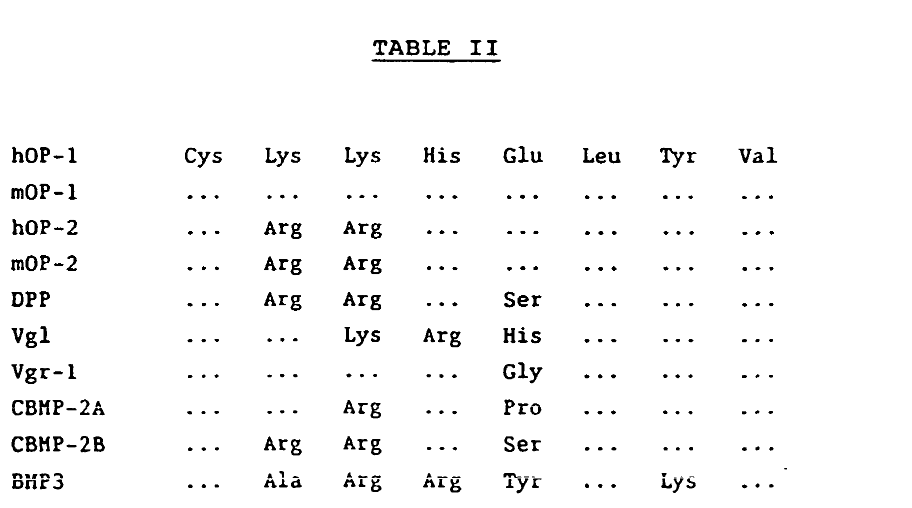

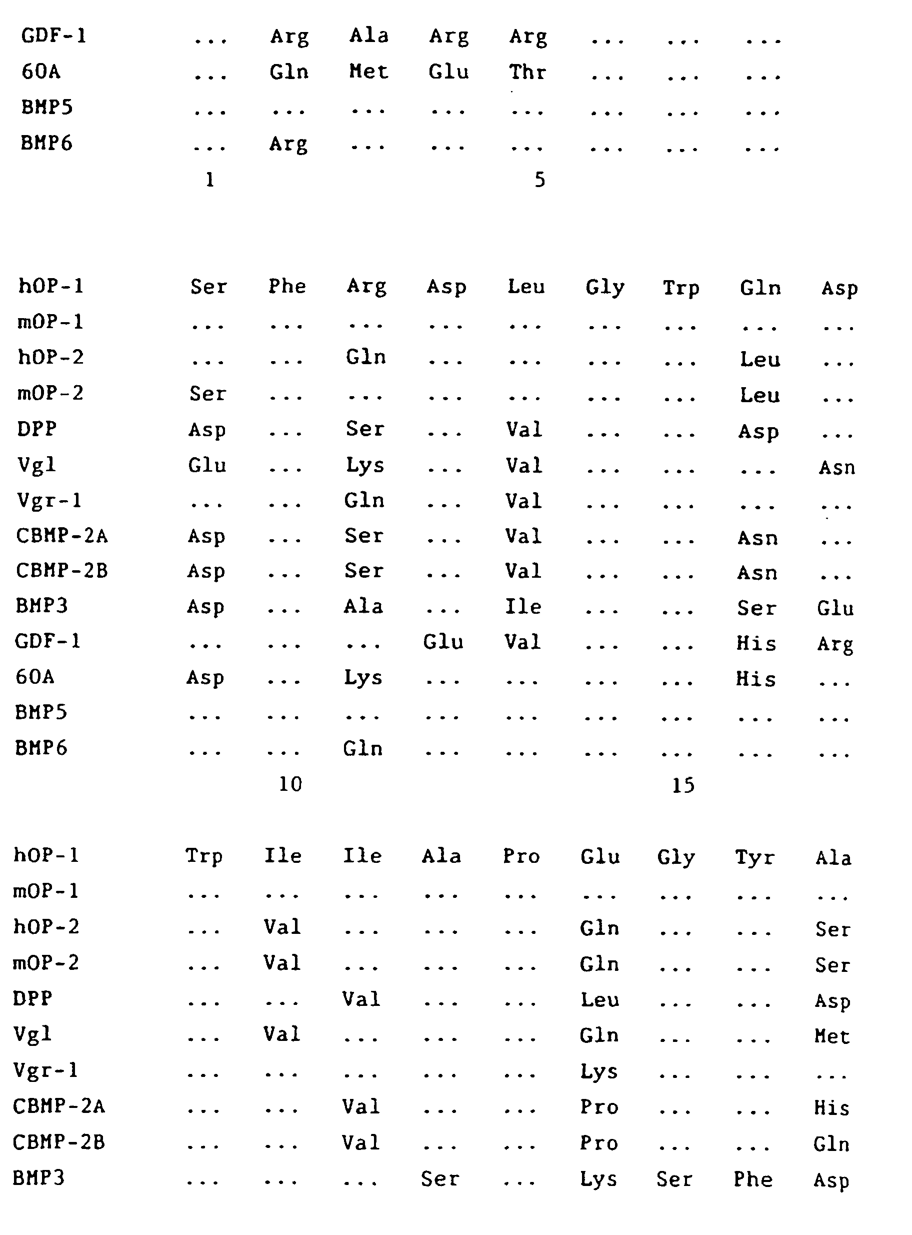

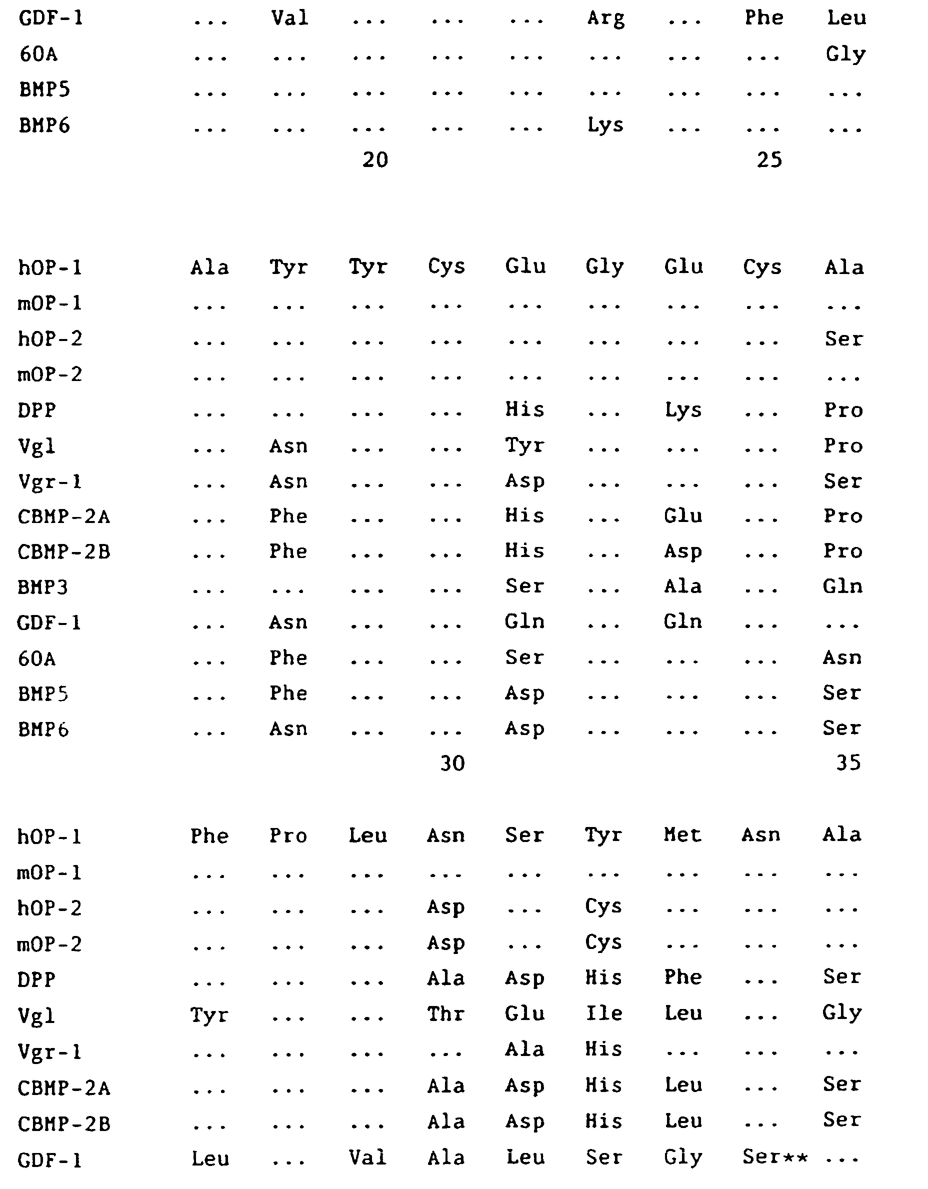

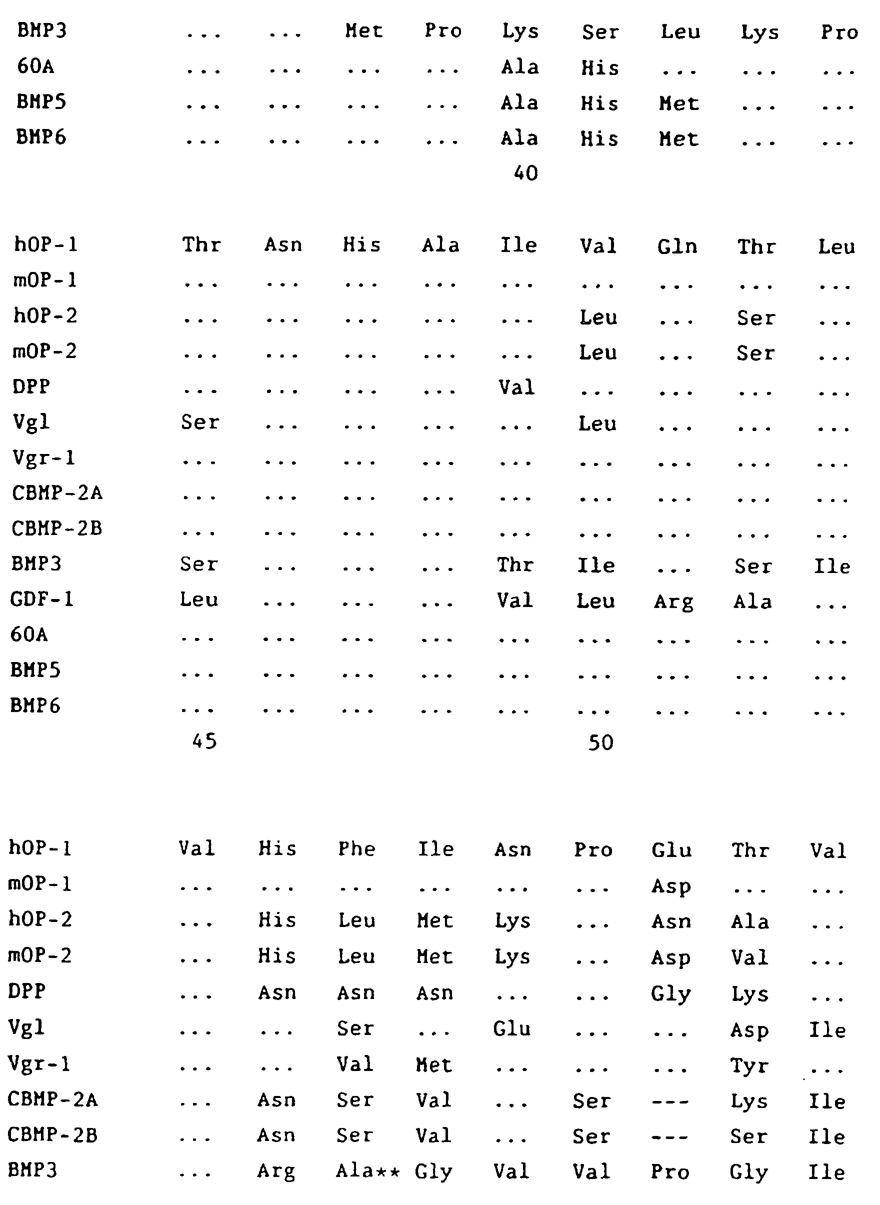

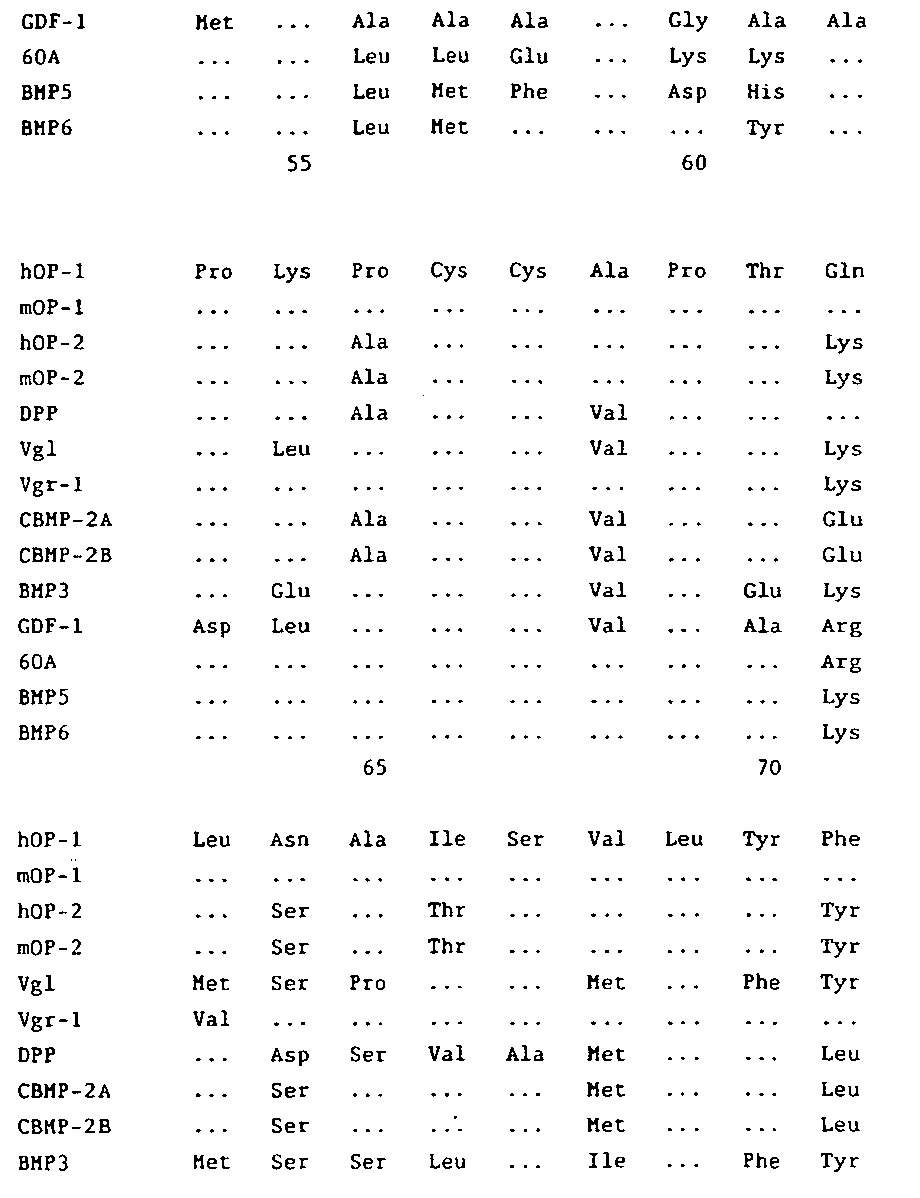

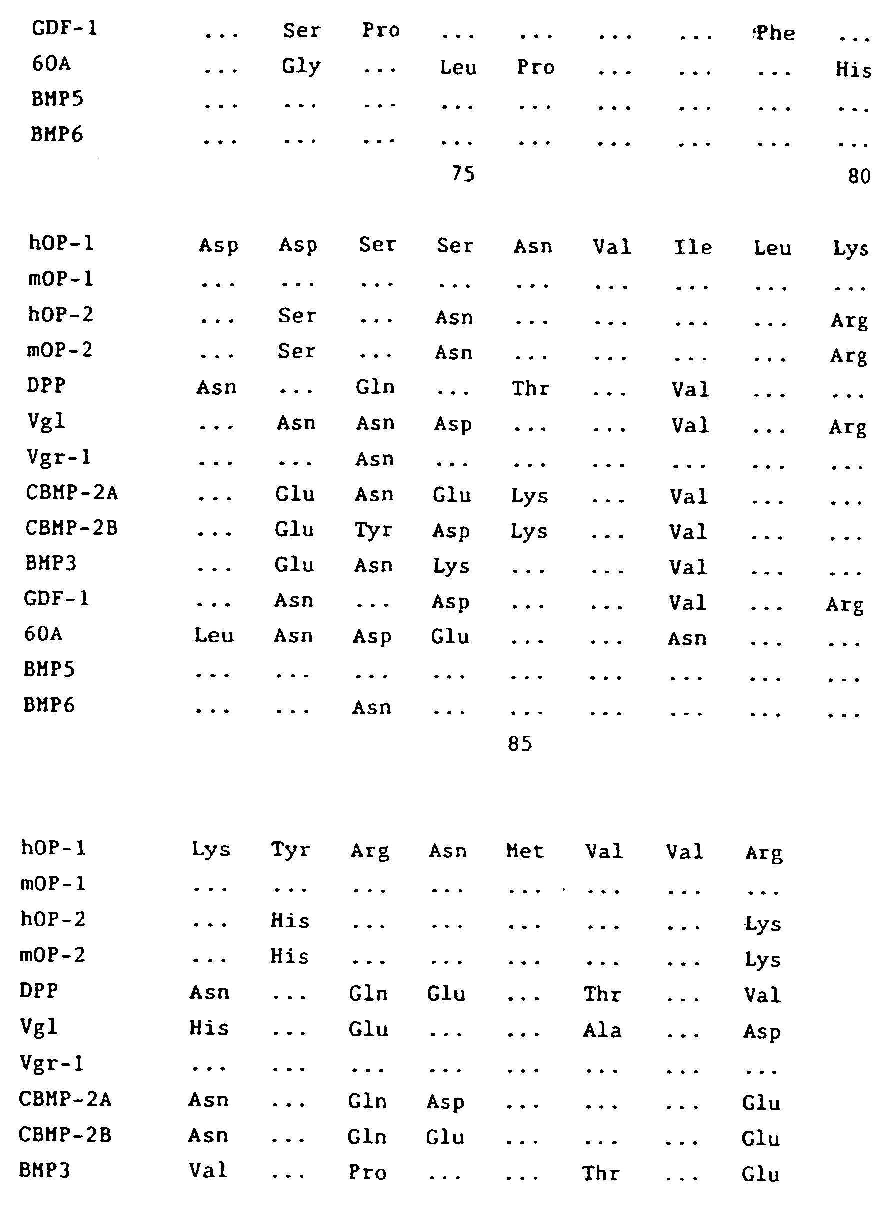

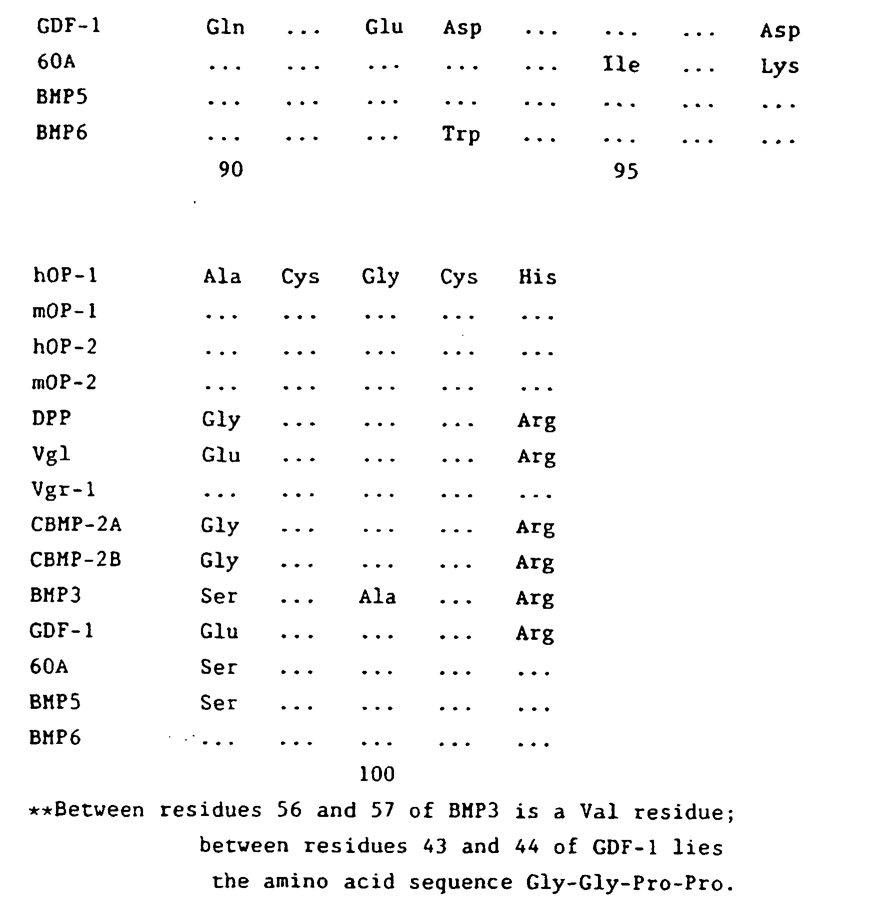

As is apparent from the foregoing amino acid sequence comparisons, significant amino acid changes can be made within the generic sequences while retaining the morphogenic activity. For example, while the GDF-1 protein sequence depicted in Table II shares only about 50% amino acid identity with the hOP-1 sequence described therein, the GDF-1 sequence shares greater than 70% amino acid sequence homology (or "similarity") with the hOP-1 sequence, where "homology" or "similarity" includes allowed conservative amino acid changes within the sequence as defined by Dayoff, et al., Atlas of Protein Sequence and Structure vol.5, supp.3, pp.345-362, (M.O. Dayhoff, ed., Nat'l BioMed. Res. Fd'n, Washington D.C. 1979.)

The currently most preferred protein sequences useful as morphogens in this disclosure include those having greater than 60% identity, preferably greater than 65% identity, with the amino acid sequence defining the conserved six cysteine skeleton of hOP-1 (e.g., residues 43-139 of Seq. ID No. 5). These most preferred sequences include both allelic and species variants of the OP-1 and OP-2 proteins, including the Drosophila 60A protein. Accordingly, the invention includes use of a morphogen in the manufacture of a medicament wherein the morphogen comprises a species of polypeptide chains having the generic amino acid sequence referred to herein as "OPX", which defines the seven cysteine skeleton and accommodates the identities between the various identified mouse and human OP1 and OP2 proteins. OPX is presented in Seq. ID No. 29. As described therein, each Xaa at a given position independently is selected from the residues occurring at the corresponding position in the C-terminal sequence of mouse or human OP1 or OP2 (see Seq. ID Nos. 5-8 and/or Seq, ID Nos. 16-23).

### II. Formulations and Methods for Administering Therapeutic Agents

The morphogens may be provided to an individual by any suitable means, preferably directly, parenterally or orally. Where the morphogen is to be provided directly (e.g., locally, as by injection, to a bone tissue site), or parenterally, such as by intravenous, subcutaneous, intramuscular, intraorbital, ophthalmic, intraventricular, intracranial, intracapsular, intraspinal, intracisternal, intraperitoneal, buccal, rectal, vaginal, intranasal or by aerosol administration, the morphogen preferably comprises part of an aqueous solution. The solution is physiologically acceptable so that in addition to delivery of the desired morphogen to the patient, the solution does not otherwise adversely affect the patient's electrolyte and volume balance. The aqueous medium for the morphogen thus may comprise normal physiologic saline (9.85% NaCl, 0.15M), pH 7-7.4. The aqueous solution containing the morphogen can be made, for example, by dissolving the protein in 50% ethanol containing acetonitrile in 0.1% trifluoroacetic acid (TFA) or 0.1% HCl, or equivalent solvents. One volume of the resultant solution then is added, for example, to ten volumes of phosphate buffered saline (PBS), which further may include 0.1-0.2% human serum albumin (HSA). The resultant solution preferably is vortexed extensively. If desired, a given morphogen may be made more soluble by association with a suitable molecule. For example, association of the mature dimer with the pro domain of the morphogen increases solubility of the protein significantly. In fact, the endogenous protein is thought to be transported in this form. Another molecule capable of enhancing solubility and particularly useful for oral administrations, is casein. For example, addition of 0.2% casein increases solubility of the mature active form of OP-1 by 80%. Other components found in milk and/or various serum proteins also may be useful.

Useful solutions for oral or parenteral administration may be prepared by any of the methods well known in the pharmaceutical art, described, for example, in Remington's Pharmaceutical Sciences, (Gennaro, A., ed.), Mack Pub., 1990. Formulations may include, for example, polyalkylene glycols such as polyethylene glycol, oils of vegetable origin, hydrogenated naphthalenes, and the like. Formulations for direct administration, in particular, may include glycerol and other compositions of high viscocity. Biocompatible, preferablly bioresorbable polymers, including, for example, hyaluronic acid, collagen, tricalcium phosphate, polybutyrate, lactide and lactide/glycolide copolymers, may be useful excipients to control the release of the morphogen in vivo. Other potentially useful parenteral delivery systems for these morphogens include ethylene-vinyl acetate copolymer particles, osmotic pumps, implantable infusion systems, and liposomes. Formulations for inhalation administration contain as excipients, for example, lactose, or may be aqueous solutions containing, for example, polyoxyethylene-9-lauryl ether, glycocholate and deoxycholate, or oily solutions for administration in the form of nasal drops, or as a gel to be applied intranasally. Formulations for parenteral administration may also include glycocholate for buccal administration, methoxysalicylate for rectal administration, or cutric acid for vaginal administration.

Alternatively, the morphogens described herein may be administered orally. Oral administration of proteins as therapeutics generally is not practiced as most proteins readily are degraded by digestive enzymes and acids in the mammalian digestive system before they can be absorbed into the bloodstream. However, the morphogens described herein typically are acid-stable and protease-resistant (see, for example, U.S. Pat. No. 4,968,590.) In addition, at least one morphogen, OP-1, has been identified in bovine mammary gland extract, colostrum and milk (see Example 10, below) as well as saliva. Moreover, the OP-1 purified from mammary gland extract has been shown to be morphogenically active. Specifically, this protein has been shown to induce endochondral bone formation in mammals when implanted subcutaneously in association with a suitable matrix material, using a standard in vivo bone assay, such as is disclosed in U.S. Pat.No. 4,968,590. In addition, endogenous morphogen also has been detected in the bloodstream (see Example 11). These findings indicate that oral and parenteral administration are viable means for administering morphogens to an individual. In addition, while the mature forms of certain morphogens described herein typically are sparingly soluble, the morphogen form found in milk (and mammary gland extract and colostrum) is readily soluble, probably by association of the mature, morphogenically active form with the pro domain of the intact sequence and/or by association with one or more milk components. Accordingly, the compounds provided herein also may be associated with molecules capable of enhancing their solubility in vitro or in vivo, including, for example, part or all of a morphogen pro domain, and casein, as described above.

The compounds provided herein also may be associated with molecules capable of targeting the morphogen or morphogen-stimulating agent to bone tissue. For example, tetracycline and diphosphonates are known to bind to bone mineral, particularly at zones of bone remodeling, when they are provided systemically in a mammal. Alternatively, an antibody or other binding protein that interacts specifically with a surface molecule on bone tissue cells also may be used. Such targeting molecules further may be covalently associated to the morphogen or morphogen-stimulating agent with, for example, an acid labile bond such as an Asp-Pro linkage, using standard chemical means well known in the art. Because the local environment at bone remodeling sites is acidic, acid-labile linkages are expected to be preferentially cleaved at these sites, yielding active morphogen or morphogen-stimulating agent at the desired site. Useful targeting molecules may be designed, for example, using the single chain binding site technology disclosed, for example, in U.S. Pat. No. 5,091,513.

As described above, the morphogens provided herein share significant sequence homology in the C-terminal active domains. By contrast, the sequences diverge significantly in the sequences which define the pro domain. Accordingly, the pro domain may be morphogen-specific. As described above, it is also known that the various morphogens identified to date are differentially expressed in the different tissues. Accordingly, without being limited to any given theory, it is likely that, under natural conditions in the body, selected morphogens typically act on a given tissue. Accordingly, part or all of pro domains, which have been identified associated with the active form of the morphogen in solution, may serve as targeting molecules for the morphogens described herein. For example, the pro domains may interact specifically with one or more molecules at the target tissue to direct the morphogen associated with the pro domain to that tissue. Accordingly, another useful targeting molecule for targeting morphogen to bone tissue is part or all of a morphogen pro domain, particularly part or all of the pro domains of OP-1, BMP2 or BMP4, all of which proteins are found naturally associated with bone tissue.

Finally, the morphogens or morphogen-stimulating agents provided herein may be administered alone or in combination with other molecules known to have a beneficial effect on maintaining appropriate bone remodeling cycles in an individual at risk for excessive bone loss. Examples of useful cofactors include vitamin D₃, calcitonin, prostaglandins, parathyroid hormone, dexamethasone, estrogen and IGF.

The compounds provided herein can be formulated into pharmaceutical compositions by admixture with pharmaceutically acceptable nontoxic excipients and carriers. As noted above, such compositions may be prepared for parenteral administration, particularly in the form of liquid solutions or suspensions; for oral administration, particularly in the form of tablets or capsules; or intranasally, particularly in the form of powders, nasal drops, or aerosols.

The compositions can be formulated for parenteral or oral administration to humans or other mammals in therapeutically effective amounts, e.g., amounts which provide appropriate concentrations of a morphogen to bone tissue for a time sufficient to inhibit loss of bone mass and/or to stimulate bone formation in individuals suffering from metabolic bone diseases and other bone remodeling disorders as described above. Therapeutic concentrations also are sufficient to repair fractures and other defects in skeletal microstructure, and to enhance maintenance of appropriate bone mass in developing juveniles and adults, including protecting individuals at risk for bone mass deterioration.

As will be appreciated by those skilled in the art, the concentration of the compounds described in a therapeutic composition will vary depending upon a number of factors, including the dosage of the drug to be administered, the chemical characteristics (e.g., hydrophobicity) of the compounds employed, and the route of administration. The preferred dosage of drug to be administered also is likely to depend on such variables as the type and extent of bone loss or defect, the overall health status of the particular patient, the relative biological efficacy of the compound selected, the formulation of the compound excipients, and its route of administration. In general terms, the compounds of this invention may be provided in an aqueous physiological buffer solution containing about 0.1 to 10% w/v compound for parenteral administration. Typical dose ranges are from about 10 ng/kg to about 1 g/kg of body weight per day; a preferred dose range is from about 0.1 µg/kg to 100 mg/kg of body weight per day. Optimally, the morphogen dosage given in all cases is between 2-20 µg of protein per kilogram weight of the patient per day. Currently preferred dose ranges for local injection of soluble morphogen to bone tissue are 0.1-50 µg morphogen/injection. No obvious morphogen-induced pathological lesions are induced when mature morphogen (e.g., OP-1, 20 µg) is administered daily to normal growing rats for 21 consecutive days. Moreover, 10 µg systemic injections of morphogen (e.g., OP-1) injected daily for 10 days into normal newborn mice does not produce any gross abnormalties.

### III. Examples

### Example 1. Identification of Morphogen-Expressing Tissue

Determining the tissue distribution of morphogens may be used to identify different morphogens expressed in a given tissue, as well as to identify new, related morphogens. Tissue distribution also may be used to identify useful morphogen-producing tissue for use in screening and identifying candidate morphogen-stimulating agents. The morphogens (or their mRNA transcripts) readily are identified in different tissues using standard methodologies and minor modifications thereof in tissues where expression may be low. For example, protein distribution may be determined using standard Western blot analysis or immunofluorescent techniques, and antibodies specific to the morphogen or morphogens of interest. Similarly, the distribution of morphogen transcripts may be determined using standard Northern hybridization protocols and transcript-specific probes.

Any probe capable of hybridizing specifically to a transcript, and distinguishing the transcript of interest from other, related transcripts may be used. Because the morphogens described herein share such high sequence homology in their active, C-terminal domains, the tissue distribution of a specific morphogen transcript may best be determined using a probe specific for the pro region of the immature protein and/or the N-terminal region of the mature protein. Another useful sequence is the 3' non-coding region flanking and immediately following the stop codon. These portions of the sequence vary substantially among the morphogens of this invention, and accordingly, are specific for each protein. For example, a particularly useful Vgr-1-specific probe sequence is the PvuII-SacI fragment, a 265 bp fragment encoding both a portion of the untranslated pro region and the N-terminus of the mature sequence (see Lyons et al. (1989) PNAS 86:4554-4558 for a description of the cDNA sequence). Similarly, particularly useful mOP-1-specific probe sequences are the BstX1-BglI fragment, a 0.68 Kb sequence that covers approximately two-thirds of the mOP-1 pro region; a StuI-StuI fragment, a 0.2 Kb sequence immediately upstream of the 7-cysteine domain; and the Ear1-Pst1 fragment, an 0.3 Kb fragment containing a portion of the 3'untranslated sequence (See Seq. ID No. 18, where the pro region is defined essentially by residues 30-291.) Similar approaches may be used, for example, with hOP-1 (Seq. ID No. 16) or human or mouse OP-2 (Seq. ID Nos. 20 and 22.)

Using these morphogen-specific probes, which may be synthetically engineered or obtained from cloned sequences, morphogen transcripts can be identified in mammalian tissue, using standard methodologies well known to those having ordinary skill in the art. Briefly, total RNA is prepared from various adult murine tissues (e.g., liver, kidney, testis, heart, brain, thymus and stomach) by a standard methodology such as by the method of Chomczyaski et al. ((1987) Anal. Biochem 162:156-159) and described below. Poly (A)+ RNA is prepared by using oligo (dT)-cellulose chromatography (e.g., Type 7, from Pharmacia LKB Biotechnology, Inc.). Poly (A)+ RNA (generally 15 µg) from each tissue is fractionated on a 1% agarose/formaldehyde gel and transferred onto a Nytran membrane (Schleicher & Schuell). Following the transfer, the membrane is baked at 80°C and the RNA is cross-linked under UV light (generally 30 seconds at 1 mW/cm²). Prior to hybridization, the appropriate probe is denatured by heating. The hybridization is carried out in a lucite cylinder rotating in a roller bottle apparatus at approximately 1 rev/min for approximately 15 hours at 37°C using a hybridization mix of 40% formamide, 5 x Denhardts, 5 x SSPE, and 0.1% SDS. Following hybridization, the non-specific counts are washed off the filters in 0.1 x SSPE, 0.1% SDS at 50°C.

Examples demonstrating the tissue distribution of various morphogens, including Vgr-1, OP-1, BMP2, BMP3, BMP4, BMP5, GDF-1, and OP-2 in developing and adult tissue are disclosed in co-pending USSN 752,764, and in Ozkaynak, et al., (1991) Biochem. Biophys. Res. Commn. 179:116-123, and Ozkaynak, et al. (1992) (JBC, in press), the disclosures of which are incorporated herein by reference. Using the general probing methodology described herein, northern blot hybridizations using probes specific for these morphogens to probe brain, spleen, lung, heart, liver and kidney tissue indicate that kidney-related tissue appears to be the primary expression source for OP-1, with brain, heart and lung tissues being secondary sources. Lung tissue appears to be the primary tissue expression source for Vgr-1, BMP5, BMP4 and BMP3. Lower levels of Vgr-1 also are seen in kidney and heart tissue, while the liver appears to be a secondary expression source for BMP5, and the spleen appears to be a secondary expression source for BMP4. GDF-1 appears to be expressed primarily in brain tissue. To date, OP-2 appears to be expressed primarily in early embryonic tissue. Specifically, northern blots of murine embryos and 6-day post-natal animals shows abundant OP2 expression in 8-day embryos. Expression is reduced significantly in 17-day embryos and is not detected in post-natal animals.

### Example 2. Mitogenic Effect of Morphogen on Rat and Human Osteoblasts

The ability of a morphogen to induce proliferation of osteoblasts may be determined in vitro using the following assay. In this and all examples involving osteoblast cultures, rat osteoblast-enriched primary cultures preferably are used. Although these cultures are heterogeneous in that the individual cells are at different stages of differentiation, the culture is believed to more accurately reflect the metabolism and function of osteoblasts in vivo than osteoblast culture obtained from established cell lines. Unless otherwise indicated, all chemicals referenced are standard, commercially available reagents, readily available from a number of sources, including Sigma Chemical, Co., St. Louis; Calbiochem, Corp., San Diego, and Aldrich Chemical Co., Milwaukee.

Rat osteoblast-enriched primary cultures were prepared by sequential collagenase digestion of newborn suture-free rat calvaria (e.g., from 1-2 day-old animals, Long-Evans strain, Charles River Laboratories, Wilmington, MA), following standard procedures, such as are described, for example, in Wong et al., (1975) PNAS 72:3167-3171. Rat osteoblast single cell suspensions then were plated onto a multi-well plate (e.g., a 48 well plate) at a concentration of 50,000 osteoblasts per well in alpha MEM (modified Eagle's medium, Gibco, Inc., Long Island) containing 10% FBS (fetal bovine serum), L-glutamine and penicillin/streptomycin. The cells were incubated for 24 hours at 37°C, at which time the growth medium was replaced with alpha MEM containing 1% FBS and the cells incubated for an additional 24 hours so that cells were in serum-deprived growth medium at the time of the experiment.

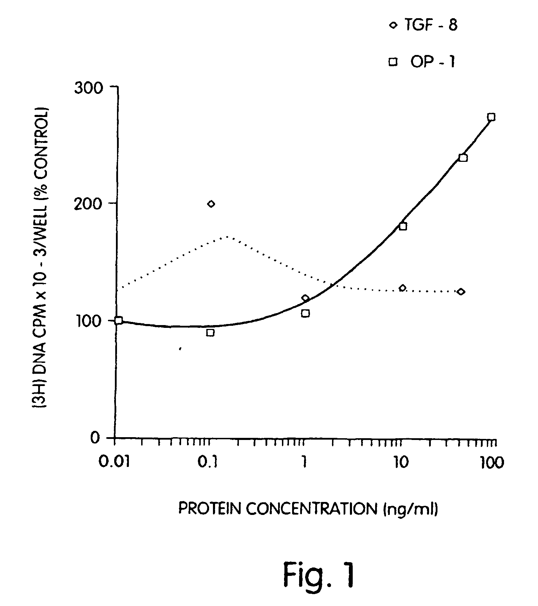

The cell culture then was divided into three groups: (1) wells which received 0.1, 1.0, 10.0, 40 and 80.0 ng of morphogen; (2) wells which received 0.1, 1.0, 10.0 and 40 ng of a local-acting growth factor; and (3) the control group, which received no growth factors. In this example, OP-1 was the morphogen tested, and TGF-β was the local-acting growth factor. The cells then were incubated for an additional 18 hours after which the wells were pulsed with 2µCi/well of ³H-thymidine and incubated for six more hours. The excess label then was washed off with a cold solution of 0.15 M NaCl, 250 µl of 10% tricholoracetic acid then was added to each well and the wells incubated at room temperature for 30 minutes. The cells then were washed three times with cold distilled water, and lysed by the addition of 250 µl of 1% sodium dodecyl sulfate (SDS) for a period of 30 minutes at 37°C. The cell lysates then were harvested using standard means well known in the art, and the incorporation of ³H-thymidine into cellular DNA was determined by liquid scintillation as an indication of mitogenic activity of the cells. The results, shown in FIG. 1, demonstrate that OP-1 (identified in the figure by squares) stimulates ³H-thymidine incorporation into DNA, and thus promotes osteoblast cell proliferation. The mitogenesis stimulated by 40 ng of OP-1 in serum-free medium was equivalent to the mitogenic effect of 10% fresh serum alone. By contrast, the effect of TGF-β (indicated by diamonds in Fig. 1) is transient and biphasic. At high concentrations, TGF-β has no significant effect on osteoblast cell proliferation. This system may be used to test other morphogens for their effect on cell proliferation.

The in vitro effect of a morphogen on osteoblast proliferation also was tested on human primary osteoblasts (obtained from bone tissue of a normal adult patient and prepared as described above) and on osteosarcoma-derived cells, and in all cases induced cell proliferation. In addition, similar experiments, performed using BMP4 (BMP2B) and BMP3 shows these morphogens also can stimulate osteoblast proliferation and growth. (See Chen et al., (1991) J. Bone and Min. Res. 6: 1387-1393, and Vukicevic, (1989) PNAS 86: 8793-8797.)

The effect of a given morphogen on bone cell growth and/or development also may be tested using a variety of bone cell markers: e.g., collagen synthesis, alkaline phosphatase activity, parathyroid hormone-mediated cyclic AMP (cAMP) production, osteocalcin synthesis, and by assessing the rate of mineralization in osteoblasts. Of these, alkaline phophatase activity, parathyroid hormone-mediated cAMP production, osteocalcin synthesis and mineralization promotion are specific markers for the differentiated osteoblast phenotype. Experimental systems for testing these parameters as well as collagen synthesis are described below in Examples 3-7. In all cases morphogen alone stimulated expression of these phenotype-specific markers. In Examples 3-7 OP-1 was the morphogen tested. Similar experiments, performed using BMP4 (BMP2B) shows that this morphogen also induces osteoblast differentiation. (See Chen, et al. (1991) T. Bone and Min. Res. 6: 1387-1392, and Vukicevic, (1989) PNAS 86: 8793-8797.)

### Example 3. Effect of Morphogen on Collagen Synthesis in Rat Osteoblasts

The effect of a morphogen on collagen production in rat osteoblasts in vitro may be determined as follows.

Rat osteoblasts were prepared and cultured in a multi-well plate as described for Example 2. In this example a 24-well plate was used. The cultured cells then were divided into three groups: (1) wells which received 1, 10 or 40 ng of morphogen per ml of medium; (2) wells which received 1, 10 or 40 ng of a local-acting growth factor per ml of medium; and (3) a control group which received no growth factors. In this example, OP-1 was the morphogen tested, and TGF-β was the local-acting growth factor.

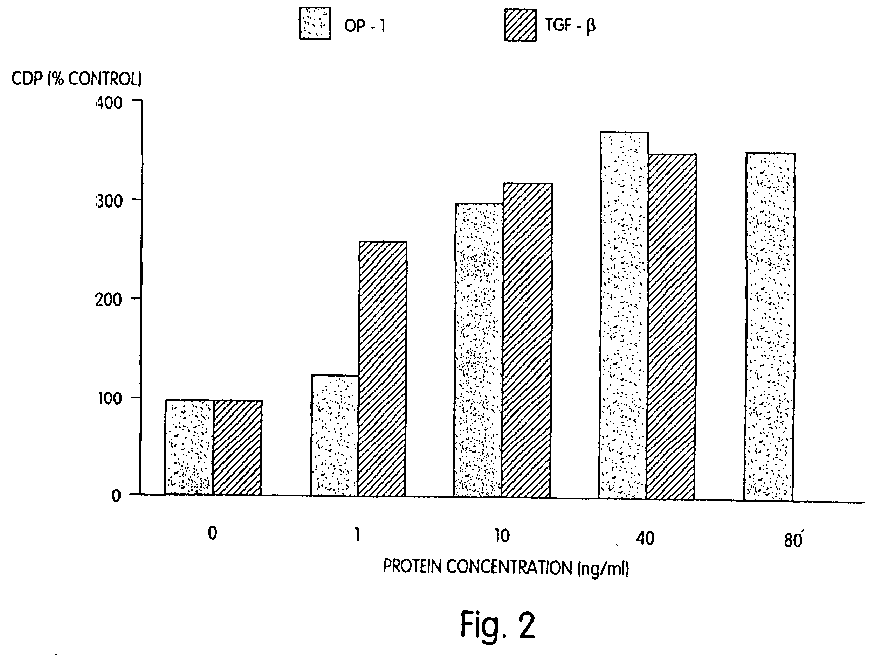

The samples were incubated for 68 hours at 37°C with 5% CO₂ in a humidified incubator. Twenty-five (25) µCi of ³H proline were added into each well and incubated for six additional hours. The cells then were frozen at -20°C until the collagen assay was performed. The cells then were assayed for collagen production by detecting incorporation of ³H-proline into total collagenase-digestible protein (CDP). The results, shown in FIG. 2, demonstrate that OP-1 stimulates type-I collagen synthesis, as measured by ³H-proline incorporation into total CDP. Thus, OP-1 promotes collagen synthesis in vitro by preosteoblasts and mature osteoblasts.

### Example 4. Alkaline Phosphatase Induction of Osteoblasts by Morphogen

### 4.1 Morphogen-specific Alkaline Phosphatase Induction

Since alkaline phosphatase production is an indicator of bone formation by differentiated, functional osteoblasts, a morphogen may be assessed for its potential osteogenic effects using this osteoblast marker in the following in vitro test system.

Rat osteoblasts were prepared and cultured in a multi-well plate as described for Example 2. In this example a 24-well plate was used. The cultured cells then were divided into three groups: (1) wells which received varying concentrations of morphogen; (2) wells which received varying concentrations of a local-acting growth factor; and (3) a control group which received no growth factors. In this example OP-1 was the morphogen tested at the following concentrations: 0.1, 1.0, 10.0, 40.0 or 80.0 ng/ml medium; and TGF-β was the local-acting growth factor, tested at 0.1, 1.0, 10.0, 40.0 or 80.0 ng/ml medium. The cells then were incubated for 72 hours. After the incubation period the cell layer was extracted with 0.5 ml of 1% Triton X-100. The resultant cell extract was centrifuged, 100 µl of the extract was added to 90 µl of paranitrosophenylphospate (PNPP)/glycerine mixture and incubated for 30 minutes in a 37°C water bath and the reaction stopped with 100 µl NaOH. The samples then were run through a plate reader (e.g., Dynatech MR700 plate reader, and absorbance measured at 400 nm, using p-nitrophenol as a standard) to determine the presence and amount of alkaline phosphate activity. Protein concentrations were determined by the Biorad method. Alkaline phosphatase activity was calculated in units/µg protein, where 1 unit=1 nmol p-nitrophenol liberated/30 minutes at 37°C.

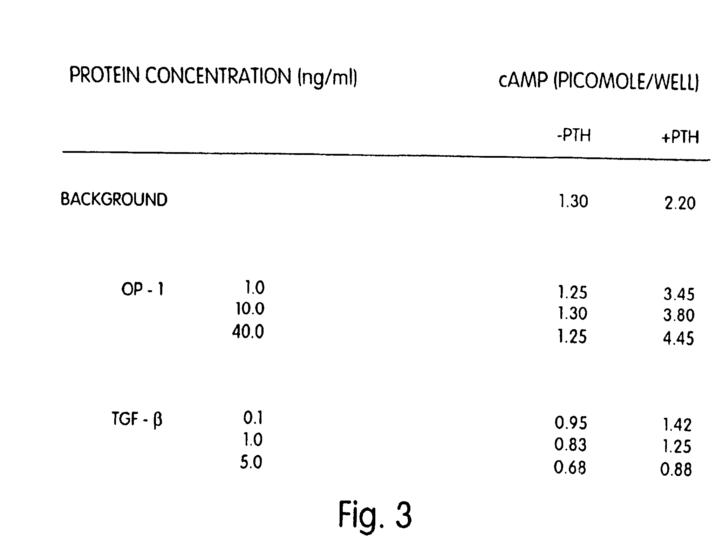

The results, shown in FIG. 3, illustrate that morphogen alone stimulates the production of alkaline phosphatase in osteoblasts, and thus promotes the growth and expression of the osteoblast differentiated phenotype. In the figure, squares represent OP-1 concentrations, and diamonds represent TGF-β concentrations.

### 4.2. Long Term Effect of Morphogen on the Production of Alkaline Phosphatase by Rat Osteoblasts

In order to determine the long term effect of a morphogen on the production of alkaline phosphatase by rat osteoblasts, the following assay may be performed.

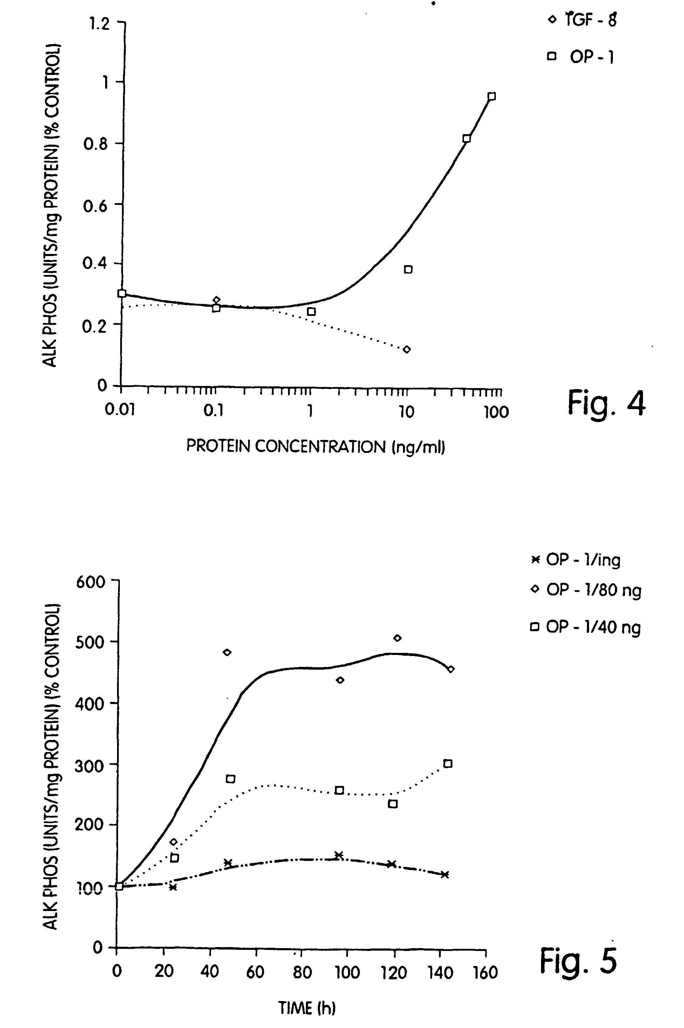

Rat osteoblasts were prepared and cultured in multi-well plates as described in Example 2. In this example six sets of 24 well plates are plated with 50,000 rat osteoblasts per well. The wells in each plate, prepared as described above, then were divided into three groups: (1) those which received 1 ng of morphogen per ml of medium; (2) those which received 40 ng of morphogen/ml of medium; and (3) those which received 80 ng of morphogen/ml of medium. Each plate then was incubated for different lengths of time: 0 hours (control time), 24 hours, 48 hours, 96 hours, 120 hours and 144 hours. After each incubation period, the cell layer was extracted with 0.5 ml of 1% Triton X-100. The resultant cell extract was centrifuged, and alkaline phosphatase activity determined as for Example 4, using paranitrosophenylphosphate (PNPP). The results, shown in FIG. 4, illustrate that morphogen alone stimulates the production of alkaline phosphatase in osteoblasts, that increasing doses of OP-1 further increase the level of alkaline phosphatase production, and that the morphogen-stimulated elevated levels of alkaline phosphatase in the treated osteoblasts lasts for an extended period of time. In the figure, circles represent 1 ng OP-1; squares, 40 ng OP-1; and diamonds, 80 ng OP-1.

### Example 5. Morphogen-Induced Parathyroid Hormone Mediated cAMP Production in Rat Osteoblasts

The effect of a morphogen on parathyroid hormone-mediated cAMP production in rat osteoblasts in vitro may be determined as follows.

Rat osteoblasts were prepared and cultured in a multiwell plate as described for Example 2 above. In this example a 24-well plate was used. The cultured cells then were divided into three groups: (1) wells which received varying concentrations of morphogen (in this example, OP-1, at 1.0, 10.0 and 40.0 ng/ml medium); (2) wells which received varying concentrations of a local-acting growth factor (in this example, TGF-β, at 0.1, 1.0, and 5.0 ng/ml medium); and (3) a control group which received no growth factors. The plate was then incubated for another 72 hours. At the end of the 72 hours the cells were treated with medium containing 0.5% bovine serum albumin (BSA) and 1mM 3-isobutyl-1-methyl xanthine for 20 minutes followed by the addition into half of the wells of human recombinant parathyroid hormone (hPTH, Sigma, St. Louis) at a concentration of 200ng/ml for 10 minutes. The cell layer was extracted from each well with 0.5 ml of 1% Triton X-100. The cAMP levels were then determined using a radioimmunoassay kit (Amersham, Arlington Heights, Illinois). The results, shown in FIG. 5, demonstrate that morphogen alone stimulates an increase in the PTH-mediated cAMP response, and thus promotes the growth and expression of the osteoblast differentiated phenotype.

### Example 6. Effect of Morphogen on Osteocalcin Synthesis and the Rate of Mineralization by Osteoblasts in Culture

Osteocalcin is a bone-specific protein synthesized by osteoblasts which plays an integral role in the rate of bone mineralization in vivo. Circulating levels of osteocalcin in serum are used as a marker for osteoblast activity and bone formation in vivo. Induction of osteocalcin synthesis in osteoblast-enriched cultures can be used to assay morphogen efficacy in vitro.

Rat osteoblasts are prepared and cultured in a multi-well plate as for Example 2. In this example cells were cultured in a 24-well plate. In this experiment the medium was supplemented with 10%FBS, and on day 2, cells were fed with fresh medium supplemented with fresh 10 mM β-glycerophosphate (Sigma, Inc.). Beginning on day 5 and twice weekly thereafter, cells were fed with a complete mineralization medium containing all of the above components plus fresh L(+)-ascorbate, at a final concentration of 50µg/ml medium. Morphogen then was added to the wells directly. In this example, OP-1 in 50% acetonitrile (or 50% ethanol) containing 0.1% trifluoroacetic acid (TFA) was added at no more than 5µl morphogen/ml medium. Control wells received solvent vehicle only. The cells then were re-fed and the conditioned medium sample diluted 1:1 in standard radioimmunoassay buffer containing standard protease inhibitors and stored at -20° C until assayed for osteocalcin. Osteocalcin synthesis then was measured by standard radioimmoassay using a commercially available rat osteocalcin-specific antibody.

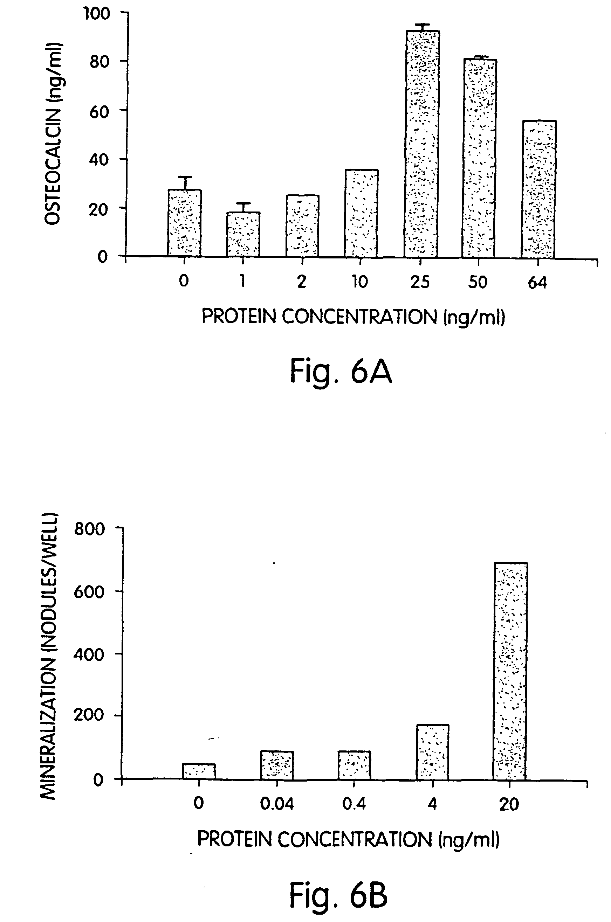

Mineralization was determined on long term cultures (13 day) using a modified von Kossa staining technique on fixed cell layers: cells were fixed in fresh 4% paraformaldehyde at 23° C for 10 mn, following rinsing cold 0.9% NaCl. Fixed cells then were stained for endogenous alkaline phosphatase at pH 9.5 for 10 min, using a commercially available kit (Sigma, Inc.) Purple stained cells then were dehydrated with methanol and air dried after 30 min incubation in 3% AgNO₃ in the dark, H₂O-rinsed samples were exposed for 30 sec to 254 nm UV light to develop the black silver-stained phosphate nodules. Individual mineralized foci (at least 20 µm in size) were counted under a dissecting microscope and expressed as nodules/culture (see Fig. 6B).

As can be seen in Fig. 6A OP-1 stimulates osteocalcin synthesis in oseoblast cultures. The increased osteocalcin synthesis in response to OP-1 is dose dependent and showed a 5-fold increase over the basal level using 25 ng of OP-1/10 ml medium after 13 days of incubation. The enhanced osteocalcin synthesis also can be confirmed by detecting the elevated osteocalcin mRNA message (20-fold increase) using a rat osteocalcin-specific probe. In addition, the increase in osteoclacin synthesis correlates with increased mineralization in long term osteoblast cultures as determined by the appearance of mineral nodules (compare Fig. 6A and 6B.) OP-1 increases the initial mineralization rate about 20-fold compared to untreated cultures. Similar experiments performed using TGF-β indicate that TGF-β does not induce osteocalcin synthesis or promote the mineralization process. Thus, morphogen alone promotes the growth and expression of the osteoblast differentiated phenotype.

### Example 7. Effect of Morphogen on Bone Derived Growth Factor Induction in vitro

IGF-I and IGF-II are bone-derived growth factors involved in coupling bone formation with bone resorption in the bone remodeling cycle. The effect of morphogen on the production of these and other bone-derived growth factors, including TGF-β, may be evaluated using the following procedure.

Rat or human osteoblasts were prepared and cultured in a multiwell plate as for Example 2. The wells of the plate were divided in to groups in which different concentrations of morphogen were added (e.g., 0, 1, 10, and 100 ng). In this example, OP-1 was the morphogen used. The plate then was incubated for a prescribed period of time, e.g., 72 hours, and the level of IGF detected, e.g., by immunolocalization, using a commercially available antibody specific for IGFs. OP-1 induced the level of both IGF-I and IGF-II significantly. Greater than six fold IGF-I and two fold IGF-II were induced following exposure to 100 ng OP-1/ml. In addition, OP-1 stimulated production of the IGF-I stimulating factor, BP3 (IGF-I binding protein 3).

### Example 8. Effect of Morphogen on Trabecular Bone in Ovariectomized (OVX) Rats

As indicated above, serum alkaline phosphatase and osteocalcin levels are indicators of bone formation within an individual. In order to determine the effect of a morphogen on bone production in vivo, these parameters are measured under conditions which promote osteoporosis, e.g., wherein osteoporosis is induced by ovary removal in rats.

Forty Long-Evans rats (Charles River Laboratories, Wilmington) weighing about 200g each are ovariectomized (OVX) using standard surgical procedures, and ten rats are sham-operated. The ovariectomization of the rats produces an osteoporotic condition within the rats as a result of decreased estrogen production. Food and water are provided ad libitum. Eight days after ovariectomy, the rats, prepared as described above, were divided into five groups: (A), 10 sham-operated rats; (B), 10 ovariectomized rats receiving 1 ml of phosphate-buffered saline (PBS) i.v. in the tail vein; (C) 10 ovariectomized rats receiving about 1 mg of 17βE₂ (17-β-estradiol E₂) by intravenous injection through the tail vein; (D) 9 ovariectomized rats receiving daily injections of approximately 2µg of morphogen by tail vein for 22 days; and (E) 9 ovariectomized rats receiving daily injections of approximately 20 µg of morphogen by tail vein for 22 days. In this example, OP-1 was the morphogen tested.

On the 15th and 21st day of the study, each rat was injected with 5 mg of tetracycline, and on day 22, the rats were sacrificed. The body weights, uterine weights, serum alkaline phosphate levels, serum calcium levels and serum osteocalcin levels then were determined for each rat. The results are shown in Tables III and IV.

**Table III**

| **Body Weights, Uterine Weights and Alkaline Phosphatase** | | | |
|---|---|---|---|
| G**roup** | **Body Weights** | **Uterine Weights** | **Alk. Phosphatase** |
| | **(g)** | **(g)** | **(U/L)** |
| A-SHAM | 250.90 ± 17.04 | 0.4192 ± 0.10 | 43.25 ± 6.11 |
| B-OVX+PBS | 273.40 ± 16.81 | 0.1650 ± 0.04 | 56.22 ± 6.21 |
| C-OVX+E2 | 241.66 ± 21.54 | 0.3081 ± 0.03 | 62.66 ± 4.11 |
| D-OVX+OP-1 (2µg) | 266.67 ± 10.43 | 0.1416 ± 0.03 | 58.09 + 12.97 |
| E-OVX+OP-1 (20 µg) | 272.40 ± 20.48 | 0.1481 ± 0.05 | 66.24 ± 15.74 |

**TABLE IV**

| **Serum Calcium and Serum Osteocalcin Levels** | | |
|---|---|---|
| **Group** | **Serum Calcium** | **Serum Osteocalcin** |
| | **(ng/dl)** | **(ng/ml)** |
| A-SHAM | 8.82 ± 1.65 | 64.66 ± 14.77 |
| B-OVX+PBS | 8.95 ± 1.25 | 69.01 ± 10.20 |
| C-OVX+E2 | 9.20 ± 1.39 | 67.13 ± 17.33 |
| D-OVX+OP-1 (2µg) | 8.77 ± 0.95 | 148.50 ± 84.11 |
| E-OVX+OP-1 (20µg) | 8.67 ± 1.94 | 182.42 ± 52.11 |

The results presented in Table III and IV show that intravenous injection of morphogen into ovariectomized rats produces a significant increase in serum alkaline phosphatase and serum osteocalcin levels and demonstrates that systemic administration of the morphogen stimulates bone formation in osteoporotic bone.

### Example 9. Histomorphometric Analysis of Morphogen on the Tibia Diaphysis in Ovariectomized (OVX) Rats

Fifteen female Long-Evans rats weighing about 160 g were ovariectomized (OVX) to produce an osteoporotic condition and five rats were sham operated (Charles River Laboratories, Wilmington, MA.) as described for Example 8. Food and water were provided ad libitum. Twenty-two days after ovariectomy, the rats were divided into four groups: (A) sham-operated (1 ml of PBS by intravenous injection through tail vein (5 rats); (B) OVX, into which nothing was injected (5 rats); (C) OVX, receiving about 1 mg of 17βE₂ by intravenous injection through the tail vein (5 rats), and (D) OVX, receiving about 1 µg of morphogen by intravenous injection through the tail vein (5 rats). In this example, OP-1 was morphogen tested.

The rats were injected daily as described for seven days, except no injections were given on the thirteenth day. The rats then were sacrificed on the nineteenth day. The tibial diaphyseal long bones then were removed and fixed in ethanol and histomorphometric analysis was carried out using standard procedures well known in the art. The results are shown in Table V.

**Table V**

| | (A) | (B) | (C) | (D) |
|---|---|---|---|---|
| MEASUREMENT | CONTROL | OVX | OVX + E₂ | OVX + OP-1 |
| Longitudinal Growth Rate (µm/day) | 20.2 ± 0.3 | 19.4 ± 0.2 | 4.9 ± 0.5 | 17.9 ± 0.9 |
| Cancellous Bone Volume (BV/TV, bone vol/total vol) | 20.2 ± 1.5 | 13.0 ± 1.6 | 13.7 ± 2.1 | 16.6 ± 1.8 |
| Cancellous Bone Perimeter (mm) | 16.2 ± 1.8 | 9.6 ± 0.9 | 11.5 ± 1.1 | 12.2 ± 0.7 |
| Labeled Cancellous Perimeter (%) | 35.5 ± 1.5 | 51.9 ± 5.6 | 58.0 ± 4.2 | 39.2 ± 1.9 |
| Mineral Apposition Rate (µm/day) | 1.76 ± 0.14 | 2.25 ± 0.16 | 1.87 ± 0.08 | 1.86 ± 0.20 |

The results presented in Table V confirm the results of Example 8, that intravenous injection of OP-1 into ovariectomized rats stimulates bone growth for bone which had been lost due to the drop in estrogen within the individual rat. Specifically, the inhibition of cancellous bone volume in OVX rats is repaired by the systemically provided morphogen. In addition, in morphogen-treated rats the labelled cancellous perimeter and mineral apposition rate now return to levels measured in the control, sham-operated rats. Moreover, morphogen treatment does not inhibit longitudinal bone growth, unlike estrogen treatment, which appears to inhibit bone growth significantly. Accordingly, systemic administration of a morphogen in therapeutically effective concentations effectively inhibits loss of bone mass in a mammal without inhibiting natural bone formation.

### Example 10. Determination of the Presence of Morphogen in Body Fluids

OP-1 has been identified in saliva, human blood serum, and various milk forms, including mammary gland extract, colostrum, and 57-day bovine milk. Moreoever, as described below, the body fluid extracted protein is morphogenically active. The discovery that the morphogen naturally is present in milk, together with the known observation that mature, active OP-1 is acid-stable and protease-resistant, indicate that oral administration is a useful route for therapeutic administration of morphogen to a mammal. Oral administration typically is the preferred mode of delivery for extended or prophylactic therapies. In addition, the identification of morphogen in all milk forms, including colostrum, indicates that the protein plays a significant role in tissue development, including skeletal development of juveniles (see Example 13, below).

### 10.1 Morphogen Detection in Milk

OP-1 was partially purified from rat mammary gland extract and bovine colostrum and 57 day milk by passing these fluids over a series of chromatography columns: (e.g., cation-exchange, affinity and reverse phase). At each step the eluant was collected in fractions and these were tested for the presence of OP-1 by standard immunoblot. Immunoreactive fractions then were combined and purified further. The final, partially purified product then was examined for the presence of OP-1 by Western blot analysis using OP-1-specific antisera, and tested for in vivo and in vitro activity.

OP-1 purified from the different milk sources were characterized by Western blotting using antibodies raised against OP-1 and BMP2. Antibodies were prepared using standard immunology protocols well known in the art, and as described generally in Example 14, below, using full-length E. coli-produced OP-1 and BMP2 as the immunogens.

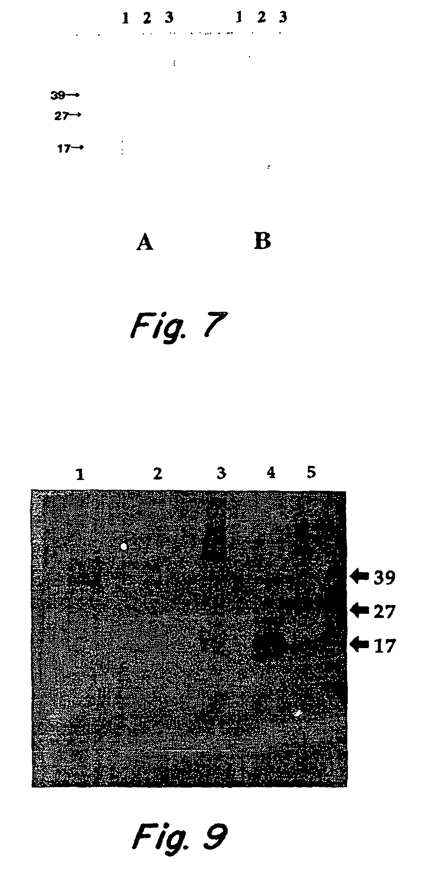

As shown in Fig. 7 OP-1 purified from colostrum reacts with the anti-OP-1 antibody, but not with anti-BMP2 antibody. In Fig. 7 lane 1 contains reduced, purified, recombinantly-produced OP-1; lane 2 contains purified bovine colostrum, and lane 3 contains reduced COP-16, a biosynthetic contruct having morphogenic activity and an amino acid sequence modeled on the proteins described herein, but having highest amino acid sequence homology with BMP2 (see US Pat. No. 5,011,691 for the COP-16 amino acid sequence.) In Fig. 7A the gel was probed with anti-OP-1 antibody; in Fig. 17B, the gel was probed with anti-BMP2 antibody. As can be seen in the figure, anti-OP-1 antibody hybridizes only with protein in lanes 1 and 2, but not 3; while anti-BMP2 antibody hybridizes with lane 3 only.

Column-purified mammary gland extract and 57-day milk also reacts specifically with anti-OP-1 antibodies, including antibody raised against the full length E. coli OP-1, full length mammalian-produced OP-1, and the OP-1 Ser-17-Cys peptide (e.g., the OP-1 N-terminal 17 amino acids).

The morphogenic activity of OP-1 purified from mammary gland extract was evaluated in vivo as follows. A sample was prepared from each OP-1 immunoreactive fraction of the mammary gland extract-derived OP-1 final product by lyophilizing a portion (33%) of the fraction and resuspending the protein in 220µl of 50% acetonitrile/0.1% TFA. After vortexing, 25 mg of collagen matrix was added. The samples were lyophilized overnight, and implanted in Long Evans rats (Charles River Laboratories, Wilmington, MA, 28-35 days old). Each fraction was implanted in duplicate. For details of the collagen matrix implantation procedure, see, for example, U.S. Pat. No. 4,968,590, hereby incorporated by reference. After 12 days, the implants were removed and evaluated for new bone formation by histological observation.

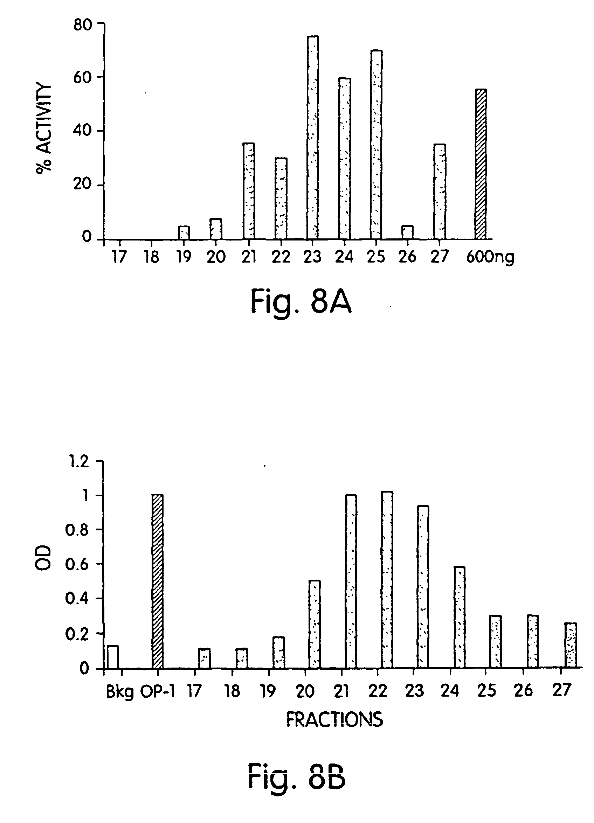

The results are presented in Fig.8A, where "% activity" refers to the percent of bone formation/total area covered by bone in the histology sample. In the figure, solid bars represent implants using mammary extract-derived OP-1, each bar corresponding to an immunoreactive fraction of the purified product, the fraction number being indicated on the x-axis. The hatched bar represents an implant using recombinantly produced OP-1 (600 ng). As can be seen in the figure, all immunoreactive fractions are osteogenically active.

Similarly, the morphogenic activity of OP-1 purified from mammary gland extract was evaluated in vitro by measuring alkaline phosphatase activity in vitro using the following assay. Test samples were prepared as for the in vivo assay, using 15-20% of individual immunoreactive fractions collected from the final product. Alkaline phosphatase activity was tested as described above in Example 4. The results, presented in Fig. 8B, indicate that the immunoreactive fractions can stimulate alkaline phosphatase activity in vitro. Moreover, the activity correlates well with that produced by highly purified, recombinantly produced, OP-1. In Fig. 8B solid bars represent assays performed with mammary gland-purified OP-1, each bar corresponding to an immunoreactive fraction of column-purified OP-1, the fraction numbers being indicated on the x-axis; the hatched bar represents the assay performed with purified, recombinantly-produced OP-1 (100 ng ml); and the cross-hatched bar represents background.

### 10.2 Morphogen Detection in Serum

Morphogen may be detected in serum using morphogen-specific antibodies. The assay may be performed using any standard immunoassay, such as Western blot (immunoblot) and the like. Preferably, the assay is performed using an affinity column to which the morphogen-specific antibody is bound and through which the sample serum then is poured, to selectively extract the morphogen of interest. The morphogen then is eluted. A suitable elution buffer may be determined empirically by determining appropriate binding and elution conditions first with a control (e.g., purified, recombinantly-produced morphogen.) Fractions then are tested for the presence of the morphogen by standard immunoblot, and the results confirmed by N-terminal sequencing. Preferably, the affinity column is prepared using monoclonal antibodies. Morphogen concentrations in serum or other fluid samples then may be determined using standard protein quantification techniques, including by spectrophotometric absorbance or by quantitation of conjugated antibody.

Presented below is a sample protocol for identifying OP-1 in serum. Following this general methodology other morphogens may be detected in body fluids, including serum. The identification of morphogen in serum further indicates that systemic administratrion is a suitable means for providing therapeutic concentrations of a morphogen to an individual, and that morphogens likely behave systemically as endocrine-like factors. Finally, using this protocol, fluctuations in endogenous morphogen levels can be detected, and these altered levels may be used as an indicator of bone tissue dysfunction. Alternatively, fluctuations in morphogen levels may be assessed by monitoring morphogen transcription levels, either by standard northern blot analysis as described in Example 1, or by in situ hybridization, using a labelled probe capable of hybridizing specifically to morphogen RNA, and standard RNA hybridization protocols well described in the art and described generally in Example 1.

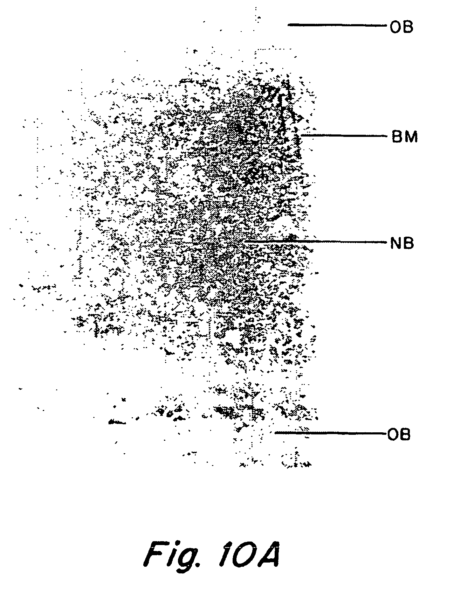

OP-1 was detected in human serum using the following assay. A monoclonal antibody raised against mammalian, recombinantly produced OP-1 using standard immunology techniques well described in the art and described generally in Example 14, was immobilized by passing the antibody over an agarose-activated gel (e.g., Affi-Gel™, from Bio-Rad Laboratories, Richmond, CA, prepared following manufacturer's instructions) and used to purify OP-1 from serum. Human serum then was passed over the column and eluted with 3M K-thiocyanate. K-thiocyanante fractions then were dialyzed in 6M urea, 20mM PO₄, pH 7.0, applied to a C8 HPLC column, and eluted with a 20 minute, 25-50% acetonitrile/0.1% TFA gradient. Mature, recombinantly produced OP-1 homodimers elute between 20-22 minutes. Fractions then were collected and tested for the presence of OP-1 by standard immunoblot using an OP-1 specific antibody as for Example 10.A. Fig. 9 is an immunoblot showing OP-1 in human sera under reducing and oxidized conditions. In the figure, lanes 1 and 4 are OP-1 standards, run under oxidized (lane 1) and reduced (lane 4) conditions. Lane 5 shows molecular weight markers at 17, 27 and 39 kDa. Lanes 2 and 3 are human sera OP-1, run under oxidized (lane 2) and reduced (lane 3) conditions.

Morphogens may be used in diagnostic applications by comparing the quantity of morphogen present in a body fluid sample with a predetermined reference value, with fluctuations in fluid morphogen levels indicating a change in the status of bone tissue. Alternatively, fluctuations in the level of endogenous morphogen antibodies may be detected by this method, most likely in serum, using an antibody or other binding protein capable of interacting specifically with the endogenous morphogen antibody. Detected fluctuations in the levels of the endogenous antibody may be used as indicators of a change in tissue status.

### Example 11. Morphogen-induced Periosteal and Endosteal Bone Formation

Osteoclast-induced bone resorption occurs primarily at the endosteal surface of bone tissue. Accordingly, in bone remodeling disorders the marrow cavity is enlarged unnaturally, weakening the weight bearing capacity of the remaining bone. The following example provides means for evaluating the ability of the morphogens decribed herein to increase endosteal and preiosteal bone mass in a mammal. In this example, both periosteal and endosteal bone formation are induced by direct injection of a morphogen in a biocompatible solution directly to the bone tissue. As demonstrated below, morphogens can induce new bone formation and increase bone mass at both surfaces when provided to the bone by direct injection. Direct injection may be a preferred mode of administration for providing therapeutically effective concentrations to reduce an enlarged marrow cavity, and/or to repair fractures and other damage to bone tissue microstructure.

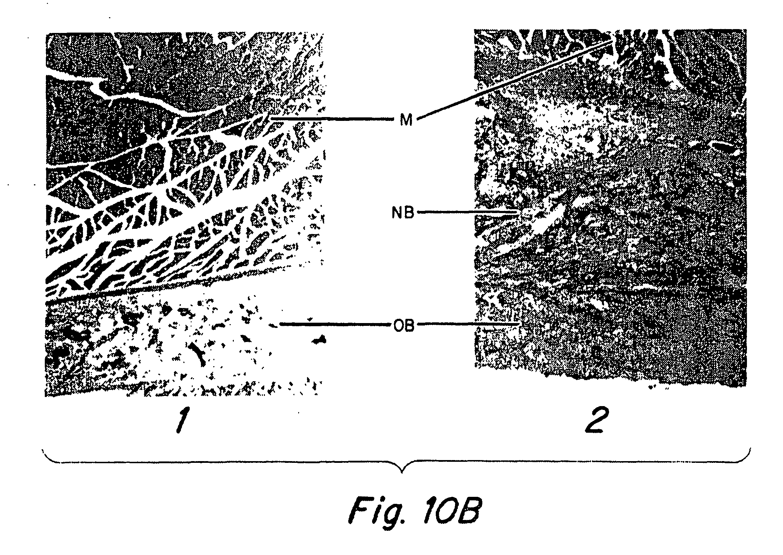

Morphogen was provided to either the periosteum (outer or peripheral bone surface) and endosteum (interior bone surface, e.g., that surface lining the marrow cavity) of a rat femur by a single injection in each case. Specifically, morphogen (e.g., OP-1, 2-20 µg) was provided to the bone tissue as an insoluble colloidal suspension in phosphate-buffered saline. Endosteal injection was performed through a microhole made with a hand-held orthopedic drill. After 7 days, the treated bones were removed and prepared for histological evaluation as described in U.S. Pat. No. 4,968,590. As little as 2 µg morphogen is sufficient to induce new bone formation at the site of injection within 4-7 days. In addition, bone induction is dose-dependent. Photomicrographs of the histology are presented in Fig. 10. In the figure, "ob" means old bone, "bm" means bone marrow, "nb" means new bone, and "m" means muscle. Fig.10A shows new bone formed following injection of morphogen to the endosteal surface. As can be seen in the figure, new bone has formed within the bone marrow cavity, filling in the periphery of the cavity. Fig 10B shows new bone formed following injection of morphogen to the periosteal surface, replacing the muscle normally present.

### Example 12. Effect of Morphogen on Bone Resorption

The effect of morphogen on bone resorption may be evaluated using rat osteoclasts on bovine bone slices, in the presence and absence of morphogen, and the effect of morphogen on pit formation (resorption index) determined. Under standard conditions rat osteoclasts begin resorbing the bone tissue, causing pit formation on the bone slice surface. In this experiment OP-1 was the morphogen tested, at concentrations of 0, 5, 10, 20, 40, 50, and 100 ng/ml.

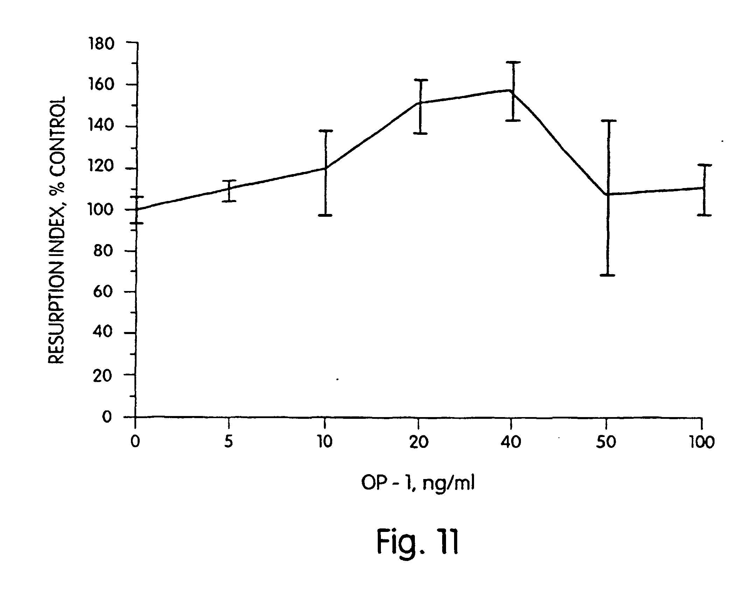

The results are presented in figure 11, where the resorption index is calculated as a percent of the control (e.g., bone resorption in the absence of morphogen), calculated as the number of pits per a given slice surface area. Below 40 ng bone resorption is enhanced; above 40 ng, OP-1 has no apparent effect on bone resorption. The results highlight the integral role the morphogen plays in bone remodeling. OP-1 is stored in bone tissue in vivo. In a normal bone remodeling cycle, the local concentration of OP-1 at the surface likely is low when osteoclasts begin resorbing bone, and the low concentration may enhance and/or stimulate bone resorption. As resorption continues, the local concentration of OP-1 at the surface likely increases, to a concentration that no longer has an effect on osteoclasts, but continues to affect osteoblast growth and activity (see Examples 2-7), stimulating bone growth.

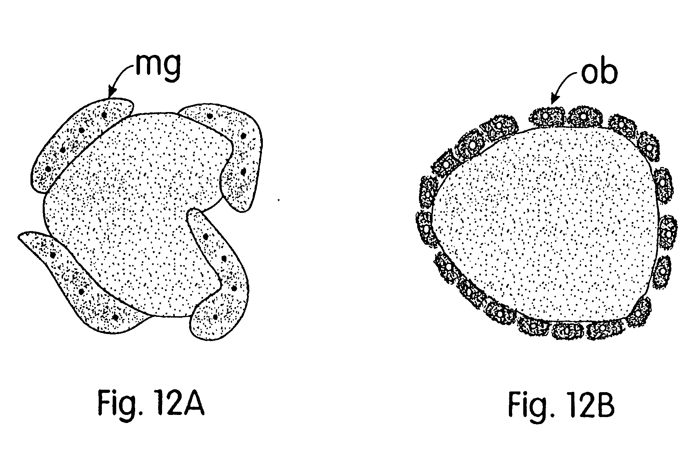

In addition, morphogens can inhibit multinucleation of mononuclear phagocytic cells under conditions where these cells normally would be activated. For example, in the absence of morphogen, an implanted substrate material (e.g., implanted subcutaneously) composed of, for example, mineralized bone, a ceramic such as titanium oxide or any other substrate that provokes multinucleated giant cell formation, rapidly becomes surrounded by multinucleated giant cells, e.g., activated phagocytes stimulated to respond and destroy the foreign object. In the presence of morphogen however, the recruited cells remain in their mononuclear precursor form and the matrix material is undisturbed. Figure 12 illustrates this effect of morphogens, in a schematic representation of histology results of a titanium oxide substrate implanted subcutaneously. In the figure, "mg" means multinucleated giant cells and "ob" means osteoblasts. The substrate represented in Fig. 12B was implanted together with morphogen (OP-1) and newly formed osteoblasts are evident surrounding the substrate. By contrast, the substrate represented in Fig. 12A was implanted without morphogen and extensive multinucleated giant cell formation is evident surrounding the substrate. Accordingly, the morphogens' effect in inhibiting excessive bone mass loss in a mammal also may include inhibiting activation of these cells.

### Example 13. Effect of Morphogen Neutralization on Bone Growth

The effect of the morphogens described herein on bone growth in developing mammals also may be evaluated using neutralizing antibodies specific for particular morphogens and assessing the effect of these antibodies on bone development. Specifically, anti-morphogen monoclonal and/or polyclonal antibodies may be prepared using standard methodologies including, for example, the protocol provided in Example 14, below.

Purified antibodies then are provided regularly to new born mice, e.g., 10-100µg/injection/day for 10-15 days. At 10 or 21 days, the mice are sacrificed and the effect of morphogen on bone development assessed by body veight, gross visual examination and histology. In this example, anti-OP-1 antibodies were used. Morphogen neutralization significantly stunted body growth, including bone growth, as indicated by the reduced body weight and reduced bone length of the treated mammals.

Similarly, morphogen activity may be assessed in fetal development in the mouse model using the following assay. Single lip injections comprising 10-100µg/injection of morphogen-specific antibody are administered to pregnant female mice during each day of the gestation period and bone development in treated and control new mice evaluated by standard histomorphometric analysis at birth. Similarly, single lip injections also may be provided to juvenile and adult mice (e.g., 10-100 µg) over a prolonged time (e.g., 10-15 days) to evaluate the effect on bone growth and bone integrity and to evaluate the onset of osteoporosis. The antibodies are anticipated to inhibit tissue morphogenesis, including bone growth and bone development, in the developing embryos.

### Example 14. Screening Assay for Candidate Compounds which Alter Endogenous Morphogen Levels

Candidate compound(s) which may be administered to affect the level of a given morphogen may be found using the following screening assay, in which the level of morphogen production by a cell type which produces measurable levels of the morphogen is determined with and without incubating the cell in culture with the compound, in order to assess the effects of the compound on the cell. This can be accomplished by detection of the morphogen either at the protein or RNA level.

### 14.1 Growth of Cells in Culture

Cell cultures of kidney, adrenals, urinary bladder, brain, or other organs, may be prepared as described widely in the literature. For example, kidneys may be explanted from neonatal or new born or young or adult rodents (mouse or rat) and used in organ culture as whole or sliced (1-4 mm) tissues. Primary tissue cultures and established cell lines, also derived from kidney, adrenals, urinary, bladder, brain, mammary, or other tissues may be established in multiwell plates (6 well or 24 well) according to conventional cell culture techniques, and are cultured in the absence or presence of serum for a period of time (1-7 days). Cells may be cultured, for example, in Dulbecco's Modified Eagle medium (Gibco, Long Island, NY) containing serum (e.g., fetal calf serum at 1%-10%, Gibco) or in serum-deprived medium, as desired, or in defined medium (e.g., containing insulin, transferrin, glucose, albumin, or other growth factors).

Samples for testing the level of morphogen production includes culture supernatants or cell lysates, collected periodically and evaluated for morphogen production by immunoblot analysis (Sambrook et al., eds., 1989, Molecular Cloning, Cold Spring Harbor Press, Cold Spring Harbor, NY), or a portion of the cell culture itself, collected periodically and used to prepare polyA+ RNA for RNA analysis. To monitor de novo morphogen synthesis, some cultures are labeled according to conventional procedures with an ³⁵S-methionine/³⁵S-cysteine mixture for 6-24 hours and then evaluated for morphogenic protein synthesis by conventional immunoprecipitation methods.

### 14.2 Determination of Level of Morphogenic Protein

In order to quantitate the production of a morphogenic protein by a cell type, an immunoassay may be performed to detect the morphogen using a polyclonal or monoclonal antibody specific for that protein. For example, OP-1 may be detected using a polyclonal antibody specific for OP-1 in an ELISA, as follows.

1 µg/100 µl of affinity-purified polyclonal rabbit IgG specific for OP-1 is added to each well of a 96-well plate and incubated at 37°C for an hour. The wells are washed four times with 0.167M sodium borate buffer with 0.15 M NaCl (BSB), pH 8.2, containing 0.1% Tween 20. To minimize non-specific binding, the wells are blocked by filling completely with 1% bovine serum albumin (BSA) in BSB and incubating for 1 hour at 37°C. The wells are then washed four times with BSB containing 0.1% Tween 20. A 100 µl aliquot of an appropriate dilution of each of the test samples of cell culture supernatant is added to each well in triplicate and incubated at 37°C for 30 min. After incubation, 100 µl biotinylated rabbit anti-OP-1 serum (stock solution is about 1 mg/ml and diluted 1:400 in BSB containing 1% BSA before use) is added to each well and incubated at 37°C for 30 min. The wells are then washed four times with BSB containing 0.1% Tween 20. 100 µl strepavidin-alkaline (Southern Biotechnology Associates, Inc. Birmingham, Alabama, diluted 1:2000 in BSB containing 0.1% Tween 20 before use) is added to each well and incubated at 37°C for 30 min. The plates are washed four times with 0.5M Tris buffered Saline (TBS), pH 7.2. 50 µl substrate (ELISA Amplification System Kit, Life Technologies, Inc., Bethesda, MD) is added to each well incubated at room temperature for 15 min. Then, 50 µl amplifier (from the same amplification system kit) is added and incubated for another 15 min at room temperature. The reaction is stopped by the addition of 50 µl 0.3 M sulphuric acid. The OD at 490 nm of the solution in each well is recorded. To quantitate OP-1 in culture media, a OP-1 standard curve is performed in parallel with the test samples.

Polyclonal antibody may be prepared as follows. Each rabbit is given a primary immunization of 100 ug/500 µl E. coli-produced OP-1 monomer (amino acids 328-431 in SEQ ID NO:5) in 0.1% SDS mixed with 500 µl Complete Freund's Adjuvant. The antigen is injected subcutaneously at multiple sites on the back and flanks of the animal. The rabbit is boosted after a month in the same manner using incomplete Freund's Adjuvant. Test bleeds are taken from the ear vein seven days later. Two additional boosts and test bleeds are performed at monthly intervals until antibody against OP-1 is detected in the serum using an ELISA assay. Then, the rabbit is boosted monthly with 100 µg of antigen and bled (15 ml per bleed) at days seven and ten after boosting.

Monoclonal antibody specific for a given morphogen may be prepared as follows. A mouse is given two injections of E. coli produced OP-1 monomer. The first injection contains 100µg of OP-1 in complete Freund's adjuvant and is given subcutaneously. The second injection contains 50 µg of OP-1 in incomplete adjuvant and is given intraperitoneally. The mouse then receives a total of 230 µg of OP-1 (amino acids 307-431 in SEQ ID NO:5) in four intraperitoneal injections at various times over an eight month period. One week prior to fusion, both mice are boosted intraperitoneally with 100 µg of OP-1 (307-431) and 30 µg of the N-terminal peptide (Ser₂₉₃-Asn₃₀₉-Cys) conjugated through the added cysteine to bovine serum albumin with SMCC crosslinking agent. This boost was repeated five days (IP), four days (IP), three days (IP) and one day (IV) prior to fusion. The mouse spleen cells are then fused to commercially available myeloma cells at a ratio of 1:1 using PEG 1500 (Boeringer Mannheim, Germany), and the cell fusion is plated and screened for OP-1-specific antibodies using OP-1 (307-431) as antigen. The cell fusion and monoclonal screening then are according to standard procedures well described in standard texts widely available in the art.

## Claims

1. Use of a morphogen in the manufacture of a medicament for the treatment of metabolic bone disease by oral administration, wherein said morphogen:
(i) stimulates endochondral bone formation in an in vivo bone assay; and
(ii) comprises an amino acid sequence selected from the group consisting of any of: OPX sequence defined by SEQ ID NO: 29, human OP-1 (SEQ ID NOS: 5, 16, and 17), mouse OP-1 (SEQ ID NOS: 6, 18, and 19), human. OP-2 (SEQ ID NOS: 7, 20, and 21), mouse OP-2 (SEQ ID NOS: 8, 22 and 23), 60A (SEQ ID NO: 24), GDF-1 (SEQ ID NOS: 14, 32, and 33), BMP2A (SEQ ID NO: 9), BMP2B (SEQ ID NO: 10), DPP (SEQ ID NO: 23), Vgl (SEQ ID NO: 12), Vgr-1 (SEQ ID NO: 13), BMP3 (SEQ ID NO: 26), BMP5 (SEQ ID NO: 27), and BMP6 (SEQ ID NO: 28); and
(iii) is suitable for administration in an amount effective for increasing the ratio of cancellous bone volume to total bone volume in said mammal
wherein the medicament does not comprise vitamin D or another bone resorption inhibitor.

2. Use of a morphogen in the manufacture of a medicament for the treatment of metabolic bone disease by parenteral administration, wherein said morphogen:
(i) stimulates endochondral bone formation in an in vivo bone assay; and
(ii) comprises an amino acid sequence selected from the group consisting of any of: human OP-1 (SEQ ID NOS: 5, 16, and 17), mouse OP-1 (SEQ ID NOS: 6, 18, and 19), human OP-2 (SEQ ID NOS: 7, 20, and 21), mouse OP-2 (SEQ ID NOS: 8, 22 and 23), 60A (SEQ ID NO: 24), GDF-1 (SEQ ID NOS: 14, 32, and 33), DPP (SEQ ID NO: 23), Vgl (SEQ ID NO: 12), BMP5 (SEQ ID NO: 27), and BMP6 (SEQ ID NO: 28); and
(iii) is suitable for administration in an amount effective for increasing the ratio of cancellous bone volume to total bone volume in said mammal
wherein the medicament does not comprise vitamin D or another bone resorption inhibitor.

3. The use of claim 1 or 2 wherein said morphogen is in a soluble form.

4. The use of claim 3 wherein said soluble form comprises a morphogen associated with a morphogen pro-domain.

5. The use of any one of the preceding claims wherein said metabolic bone disorder is any of osteoporosis, osteomalacia and renal osteodystrophy.

6. The use of any one of the preceding claims wherein said metabolic bone disorder is caused by a nutritional or hormonal deficiency.

7. The use of claim 2, wherein the morphogen is formulated for injection.

8. The use of claim 7, wherein the morphogen is formulated for intravenous injection.

## Patentansprüche

1. Verwendung eines Morphogens bei der Herstellung eines Medikaments zur Behandlung einer metabolischen Knochenerkrankung durch orale Verabreichung, wobei das Morphogen:
(i) endochondrale Knochenbildung in einem in vivo Knochenassay stimuliert; und
(ii) eine Aminosäuresequenz umfasst, ausgewählt aus der Gruppe bestehend aus irgendeinem von: OPX Sequenz definiert durch SEQ ID NO:29, menschlichem OP-1 (SEQ ID NO: 5, 16 und 17), OP-1 von der Maus (SEQ ID NO: 6, 18 und 19), menschlichem OP-2 (SEQ ID NO: 7, 20 und 21), OP-2 von der Maus (SEQ ID NO: 8, 22 und 23), 60A (SEQ ID NO: 24), GDF-1 (SEQ ID NO: 14, 32 und 33), BMP2A (SEQ ID NO: 9), BMP2B (SEQ ID NO: 10), DPP (SEQ ID NO: 23), Vgl (SEQ ID NO: 12), Vgr-1 (SEQ ID NO: 13), BMP3 (SEQ ID NO: 26), BMP5 (SEQ ID NO: 27) und BMP6 (SEQ ID NO: 28); und
(iii) zur Verabreichung in einer zum Erhöhen des Verhältnisses von Spongiosavolumen zu gesamten Knochenvolumen in dem Säuger effektiven Menge geeignet ist;
wobei das Medikament kein Vitamin D oder einen anderen Knochenresorptionsinhibitor enthält.

2. Verwendung eines Morphogens bei der Herstellung eines Medikaments zur Behandlung einer metabolischen Knochenerkrankung durch parenterale Verabreichung, wobei das Morphogen:
(i) endochondrale Knochenbildung in einem in vivo Knochenassay stimuliert; und
(ii) eine Aminosäuresequenz umfasst, ausgewählt aus der Gruppe bestehend aus irgendeinem von: menschlichem OP-1 (SEQ ID NO: 5, 16 und 17), OP-1 von der Maus (SEQ ID NO: 6, 18 und 19), menschlichem OP-2 (SEQ ID NO: 7, 20 und 21), OP-2 von der Maus (SEQ ID NO: 8, 22 und 23), 60A (SEQ ID NO: 24), GDF-1 (SEQ ID NO: 14, 32 und 33), DPP (SEQ ID NO: 23), Vgl (SEQ ID NO: 12), BMP5 (SEQ ID NO: 27) und BMP6 (SEQ ID NO: 28); und
(iv) zur Verabreichung in einer zum Erhöhen des Verhältnisses von Spongiosavolumen zu gesamten Knochenvolumen in dem Säuger effektiven Menge geeignet ist;
wobei das Medikament kein Vitamin D oder einen anderen Knochenresorptionsinhibitor enthält.

3. Verwendung nach Anspruch 1 oder 2, wobei das Morphogen in einer löslichen Form vorliegt.

4. Verwendung nach Anspruch 3, wobei die lösliche Form ein Morphogen umfasst, das mit einer morphogenen Pro-Domäne zusammenhängt.

5. Verwendung nach einem der vorstehenden Ansprüche, wobei die metabolische Knochenkrankheit eine von Osteoporose, Osteomalazie und Nieren-Osteodystrophie ist.

6. Verwendung nach einem der vorstehenden Ansprüche, wobei die metabolische Knochenkrankheit durch einen Ernährungs- oder Hormonmangel verursacht ist.

7. Verwendung nach Anspruch 2, wobei das Morphogen zur Injektion formuliert ist.

8. Verwendung nach Anspruch 7, wobei das Morphogen zur intravenösen Injektion formuliert ist.

## Revendications

1. Utilisation d'un morphogène dans la préparation d'un médicament pour le traitement de la maladie du métabolisme osseux par administration orale, ledit morphogène :
(i) stimulant la formation d'os endochondral dans un examen des os in vivo ; et
(ii) comprenant une séquence d'acides aminés choisie parmi le groupe constitué par : une séquence OPX définie par SEQ ID NO : 29, la OP-1 humaine (SEQ ID NO : 5, 16 et 17), la OP-1 de souris (SEQ ID NO: 6, 18 et 19), la OP-2 humaine (SEQ ID NO: 7, 20 et 21), la OP-2 de souris (SEQ ID NO: 8, 22 et 23), 60A (SEQ ID NO: 24), GDF-1 (SEQ ID NO: 14, 32 et 33), BMP2A (SEQ ID NO: 9), BMP2B (SEQ ID NO: 10), DPP (SEQ ID NO: 23), Vgl (SEQ ID NO: 12), Vgr-1 (SEQ ID NO: 13), BMP3 (SEQ ID NO: 26), BMP5 (SEQ ID NO: 27), et BMP6 (SEQ ID NO: 28) ; et
(iii) étant approprié pour une administration en une quantité efficace pour augmenter le volume d'os spongieux par rapport au volume osseux total dans ledit mammifère,
le médicament ne comprenant pas de vitamine D ou un autre inhibiteur de la résorption osseuse.

2. Utilisation d'un morphogène dans la préparation d'un médicament pour le traitement de la maladie du métabolisme osseux par administration parentérale, ledit morphogène :
(i) stimulant la formation d'os endochondral dans un examen des os in vivo ; et
(ii) comprenant une séquence d'acides aminés choisie parmi le groupe constitué par : une séquence OPX définie par SEQ ID NO : 29, la OP-1 humaine (SEQ ID NO : 5, 16 et 17), la OP-1 de souris (SEQ ID NO: 6, 18 et 19), la OP-2 humaine (SEQ ID NO: 7, 20 et 21), la OP-2 de souris (SEQ ID NO: 8, 22 et 23), 60A (SEQ ID NO: 24), GDF-1 (SEQ ID NO: 14, 32 et 33), BMP2A (SEQ ID NO: 9), BMP2B (SEQ ID NO: 10), DPP (SEQ ID NO: 23), Vgl (SEQ ID NO: 12), Vgr-1 (SEQ ID NO: 13), BMP3 (SEQ ID NO: 26), BMP5 (SEQ ID NO: 27), et BMP6 (SEQ ID NO: 28) ; et
(iii) étant approprié pour une administration en une quantité efficace pour augmenter le volume d'os spongieux par rapport au volume osseux total dans ledit mammifère,
le médicament ne comprenant pas de vitamine D ou un autre inhibiteur de la résorption osseuse.

3. Utilisation selon la revendication 1 ou 2, dans laquelle ledit morphogène est présent sous une forme soluble.

4. Utilisation selon la revendication 3, dans laquelle ladite forme soluble comprend un morphogène en association avec un prodomaine de morphogène.

5. Utilisation selon l'une quelconque des revendications précédentes, dans laquelle ledit trouble du métabolisme osseux représente un trouble quelconque choisi parmi le groupe comprenant l'ostéoporose, l'ostéomalacie et l'ostéodystrophie rénale.

6. Utilisation selon l'une quelconque des revendications précédentes, dans laquelle ledit trouble du métabolisme osseux est provoqué par une déficience nutritionnelle ou hormonale.

7. Utilisation selon la revendication 2, dans laquelle le morphogène est formulé pour injection.

8. Utilisation selon la revendication 2, dans laquelle le morphogène est formulé pour injection intraveineuse.
